# EUROPEAN PATENT SPECIFICATION

(11) **EP 2 921 048 B1**
(45) Date of publication and mention of the grant of the patent: **19.12.2018**
(21) Application number: 15160302.4
(22) Date of filing: 23.03.2015
(51) Int. Cl.: C12N 15/85, C12N 15/90, A01K 67/027

(54) **SUS SCROFA V2G: A SAFE-HARBOR SITE FOR LONG-TERM EXPRESSION AND HIGH INTEGRATION RATE OF TRANSGENES IN PIG**
WILDSCHWEIN-V2G: SICHERE STELLE ZUR LANGFRISTIGEN EXPRESSION UND HOHER INTEGRATIONSRATE VON TRANSGENEN IN SCHWEINEN
SUS SCROFA V2G : SITE SÛR POUR EXPRESSION À LONG TERME ET GRANDE VITESSE D'INTÉGRATION DE TRANSGÈNES DANS UN PORC

(30) Priority: 21.03.2014 IT MI20140484
(43) Date of publication of application: 23.09.2015
(73) Proprietor: Avantea SRL, 26100 Cremona (IT)
(72) Inventor: PEVERALI, Antonio Fiorenzo, 27100 Pavia (IT); BIAMONTI, Giuseppe, 27100 Pavia (IT); BONCOMPAGNI, Eleonora, 27100 Pavia (IT); CUBELLS, Matthieu, 31500 Toulouse (FR); GALLI, Cesare, 26100 Cremona (IT); LAZZARI, Giovanna, 26100 Cremona (IT); PEROTA, Andrea, 21017 Samarate (IT); LAGUTINA, Irina, 26100 Cremona (IT)
(74) Representative: Zaccaro, Elisabetta

(56) References cited:
- WO-A-2013/188358
- BRUNETTI DARIO ET AL: "Transgene Expression of Green Fluorescent Protein and Germ Line Transmission in Cloned Pigs Derived from In Vitro Transfected Adult Fibroblasts", CLONING AND STEM CELLS, vol. 10, no. 4, December 2008 (2008-12), pages 409-419, XP055139558, ISSN: 1536-2302
- DATABASE EMBL [Online] 14 December 2008 (2008-12-14), "Pig DNA sequence *** SEQUENCING IN PROGRESS *** from clone CH242-358A3", XP002729623, retrieved from EBI accession no. EM_HTG:FP102391 Database accession no. FP102391
- JANNIK EJNAR JAKOBSEN ET AL: "Generation of minipigs with targeted transgene insertion by recombinase-mediated cassette exchange (RMCE) and somatic cell nuclear transfer (SCNT)", TRANSGENIC RESEARCH, vol. 22, no. 4, 31 October 2012 (2012-10-31), pages 709-723, XP055105241, ISSN: 0962-8819, DOI: 10.1007/s11248-012-9671-6
- YIN Z ET AL: "Position effect variegation and epigenetic modification of a transgene in a pig model", GENETICS AND MOLECULAR RESEARCH, vol. 11, no. 1, 2012, pages 355-369, XP002729624,
- GALLI C ET AL: "Somatic Cell Nuclear Transfer and Transgenesis in Large Animals: Current and Future Insights", REPRODUCTION IN DOMESTIC ANIMALS, vol. 47, no. Suppl. 3, Sp. Iss. SI, June 2012 (2012-06), pages 2-11, XP002729625,

## Description

### FIELD OF THE INVENTION

The present invention concerns the field of gene integration and durable gene expression in specific genomic sites of transgenic animals suitable for the generation of tools for biomedical research and biotechnological applications.

### STATE OF THE ART

Disease models in large animals play an increasing role in biomedical research and have important implications for the development of diagnostic tools and clinical treatments of human diseases. Pigs are used as models i.e. for cardiovascular disease, atherosclerosis, wound repair, cancer, diabetes, cystic fibrosis, intestinal, ophthalmological and neurodegenerative diseases (Alzheimer's disease). Pigs share anatomical and physiological similarities with humans. Moreover, a longer life-span may allow studies of chronic diseases, and a human-size body may enable studies of surgical intervention. Thus, genetically engineered swine disease models may be expected to reflect human pathophysiology much better than small animal models and could provide an alternative source of organs for xenotransplantation. In addition, pigs have advantages over other animal models in that societal concern presumably is lower for utilization of a food animal as a research model in comparison with companion animals.

Transgenic technology has been used for years to study gene function, synthetize proteins of biomedical relevance and generate models for the study of human diseases. However, the efficiency of producing transgenic animal lines that retain normal function and express the transgene is influenced by several genetic and epigenetic factors. Moreover, transgenes may present a variegated pattern of expression among tissues or even within the same tissue. Transgene expression may also undergo to spatial and/or temporal changes during the development or the life-span of the animal.

Achieving desired levels of transgene expression in an animal is not only a function of the transgene construct, but also of the location at which it integrates. Position effect variegation of transgene expression is well-documented [1]. Genetic transformation of animals generally results in a large and random variation in transgene expression and this variability is attributed to different integration sites of the transgene. Integration of foreign DNA plays a pivotal role in both genetic manipulation of cell lines and technologies related to therapeutic gene transfer. Current integrations strategies based upon plasmidic, retroviral, lentiviral or DNA transposon-based vector systems allow efficient gene insertion, and however gene insertion is mostly random and cannot be directed to predetermined positions of the host genome. Epigenetic modifications of the chromatin are responsible for position-effect variegation of gene expression and spreading of heterochromatin from flanking genomic regions on promoters of transgene may lead to a partial or complete transcriptional silencing. As such, transgenic animal models often suffer from reduced gene expression, or lack of gene activity in tissues and cells in which transcription is required to develop a desired phenotype. Thus, the site of integration is of great importance for gene expression profile of the inserted gene.

Over 130 knock-in cell lines have been generated based on the Gt(ROSA)26Sor (gtrosa26, R26R3, R26^{tg}, ROSA26, Rosa26lacZ, ROSA26-lacZ, TgR(ROSA26)26Sor) locus (http://www.informatics.jax.org/), which is a well-characterized mouse locus supporting long-term gene expression. In pig, a ROSA26-homologous site was mapped on chromosome 13 (Patent Number: CN101886075B), however stable, efficient and long-term expression of exogenous genes have not been exhaustively investigated, so far. By homologous recombination (HR), a recombinant DNA of interest may be targeted into specific genomic regions. Nevertheless, high efficient integration of the transgene by HR has been described almost exclusively in embryonic stem cells (ESC). More recently, endonuclease-mediated homologous recombination opened the possibility to efficiently knock-in transgene of interest in somatic cells. These engineered cells may be used i.e. for somatic cell nuclear transfer (SCNT) to generate transgenic animals, in particular transgenic pigs [2].

The need and importance are increasingly felt for the identification of a site of integration in the pig genome, which allows for stable and long term gene expression and also high rate of integration for the generation of transgenic animals useful for biomedical and biotechnological purposes. A candidate locus is carried by the transgenic pig, Verro2GFP, which showed high and stable expression of EGFP signal in all tissue analyzed (liver, pancreas, lung, cardiac muscle cells, striated muscle cells and central nervous system cells) [3]. These suggested that this transgenic line may carry the plasmid vector integrated into a suitable chromosomal locus allowing a long-term and ubiquitous expression of the transgene, transmitted to the progeny as a Mendelian character.

It is therefore object of the present invention the identification and development of a genomic site with high rate of integration and suitable for stable and long-term expression of transgenes.

### SUMMARY OF THE INVENTION

The present invention concerns a pig genome specific integration site (or Sus Scrofa or S.scrofa genome specific integration site), which has the advantage of allowing a stable, long-term, efficient and ubiquitous expression of the exogenous transgene which is not subjected to cellular variegations. The exogenous transgene expression can be tissue- and time-specific, as a consequence of the promoter which is chosen.

As will be further described in the detailed description of the invention, the specific integration site of the pig (swine, Sus Scrofa or S.scrofa) genome of the present invention has the sequence defined in SEQ ID NO:49 and has the advantages of allowing a high efficient site specific targeting of a gene of interest by engineered-endonucleases mediated homologous recombination and/or RMCE, namely recombinase-mediated cassette exchange, once the lox sites have been inserted. The specific integration site can have sequence identity of at least 95% or at least 97% to the sequence defined in SEQ ID NO:49.

The specific integration site of the invention regards a site of the swine genome, namely S.scrofa V2G, mapped between the NC_010451.3 gi:347618785 region: bp 22911474- 22912619 GPC_000000591 serine protease 23 isoform 3 gene at the 5' side and NC_010451.3 gi:347618785 region: bp 23002834-23006559 frizzled 4 gene at the 3' side on the Sus scrofa breed mixed chromosome 9, Sscrofa10.2 Sequence ID: ref|NC_010451.3| gi:347618785 GPC_000000591.

More in detail, the cloned flanking sequences of the S.scrofa V2G integration site were mapped on the Sus scrofa breed mixed chromosome 9, Sscrofa10.2 Sequence ID: ref|NC_010451.3| gi:347618785 (bp 22916605 - 22918906) as outlined by sequence homology of natural variants isolated from different swine breeds including SEQ ID NO:24, SEQ ID NO:25 and SEQ ID NO:26.

One aspect of the invention relates to the natural variants isolated from different swine breeds having the sequences of SEQ ID NO:24, SEQ ID NO:25 and SEQ ID NO:26.

In a further aspect, the invention regards a targeting vector of plasmidic origin integrated into the Sus scrofa genome specific integration site according to the present invention.

In a still further aspect the invention relates to the use of the vector for the long-term expression and the stable integration into the host genome of a recombinant gene of interest.

In a still further aspect, the invention relates to a site-specific endonuclease target site of the Sus scrofa specific integration site of SEQ ID NO:49, SEQ ID NO:24, SEQ ID NO:25 and SEQ ID NO:26.

### BRIEF DESCRIPTION OF THE DRAWINGS

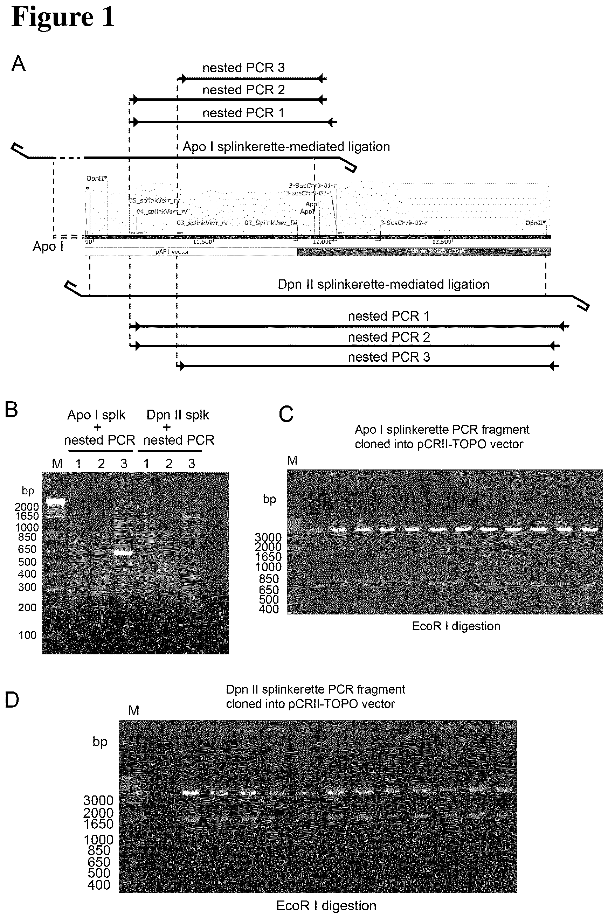

The characteristics and advantages of the present invention will be apparent from the detailed description reported below, from the Examples given for illustrative and non-limiting purposes, and from the annexed FIGUREs 1-11, wherein:
Figure 1:
   Cloning of the pAP1-integration site of the Verro2GFP transgenic swine genome by splinkerette assay. (A) Schematic representation of the Verro2GFP genomic sequence joined to the (SEQ ID NO:1) Kpnl-linearized pAP1 vector. Verro2GFP genomic DNA was digested with the Apol or Dpnll restriction enzymes and ligated with Apol- or Dpnll-splinkerette linkers, respectively (see examples 1-3). Using the ligations as templates, three consecutive nested PCR reactions were set up with the indicated primer sets to amplify the genomic sequence joined to the pAP1 vector. (B) The splinkerette PCR products were resolved on 1.5% agarose gel electrophoresis, purified from the gels, cloned into the pCRII-TOPO and sequenced. (C and D) EcoRI digestion of Apol (650bp) and DpnII (1700 bp) - splinkerette derivative clones. M is the 1kb plus ladder molecular size marker (Life-Technology).

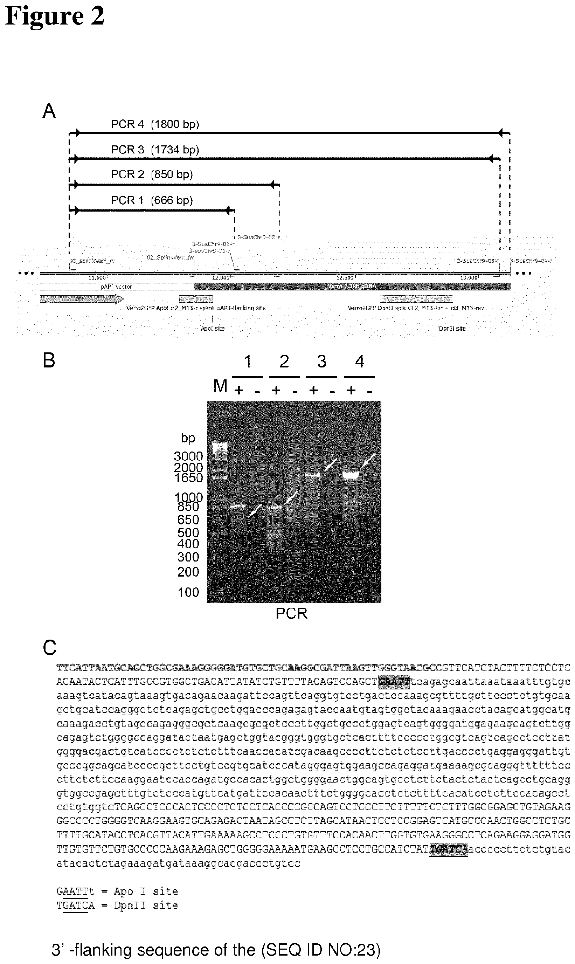

Figure 2: Validation of the Verro2GFP 3'-end flanking sequence of the pAP1-integration site. (A) Schematic representation of the pAP1 vector joined to the Verro2GFP genomic sequence. The positions relative to the Verro2GFP genome of the (SEQ ID NO:12) and (SEQ ID NO:13) splinkerette DNA fragments are indicated by yellow boxes. The outward (SEQ ID NO:6) 03_SplinkVerr_rv primer targeting the (SEQ ID NO:1) pAP1 vector was challenged with (SEQ ID NO: 14) *3-SusChr9-01-r,* (SEQ ID NO: 15) *3-SusChr9-02-r*, (SEQ ID NO:16) *3-SusChr9-03-r* and (SEQ ID NO: 17) *3-SusChr9-04-r* primers targeting swine genomic sequences external to the 3'-end of each DNA sequence identified by the splinkerette assays. (B) Agarose gel electrophoresis of the PCR products described in A are indicated by white arrows. (C) The 3' -flanking sequence of the (SEQ ID NO:23) Verro2GFP-integration site is shown. The (SEQ ID NO:12) and (SEQ ID NO:13) DNA fragments isolated by splinkerette assays are indicated in upper case of which the pAP1 plasmidic sequence corresponds to the first 60 bases. The Apol (italic and green highlight) and Dpnll sites (italic and light blu highlight) are also shown.

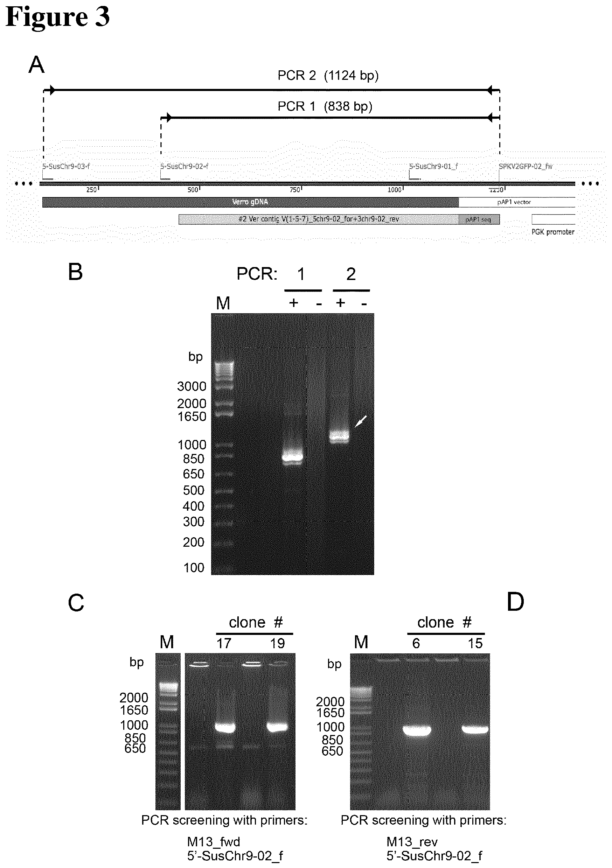

Figure 3: Cloning of the Verro2GFP 5'-end flanking sequence of the pAP1-integration site. (A) Schematic representation: The outward (SEQ ID NO:21) SPKV2GFP-02_fw primer targeting the 3'-end of the (SEQ ID NO:1) pAP1 was challenged with (SEQ ID NO: 19) *5-SusChr9-02-f* and (SEQ ID NO: 20) *5-SusChr9-03-f* targeting the Verro2GFP swine genome at the 5'end of the pAP1 integration site mapped by splinkerette assay. (B) The PCR1 and PCR2 products were resolved on agarose gel electrophoresis before cloning into the pCRII-TOPO vector. The positive clones carrying the longer (1.1kb) amplified product, PCR2 (white arrow), were identified by PCR screening the recombinant plasmids with (SEQ ID NO:21) SPKV2GFP-02_fw and the universal primers, (SEQ ID NO:10) *M13for* (C) or (SEQ ID NO:11) *M13rev* (D). Positive clones were sequenced by standard procedure. The sequenced region of swine DNA joined to pAP1 vector is indicated by a yellow box.

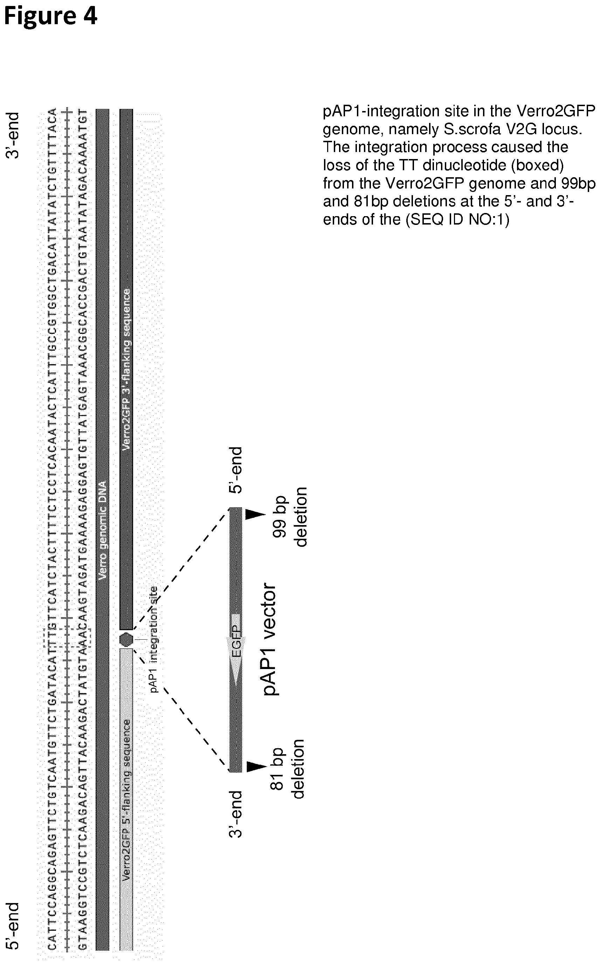

Figure 4: Schematic representation of the pAP1-integration site in the Verro2GFP genome, namely S.scrofa V2G locus. The integration process caused the loss of the TT dinucleotide (boxed) from the Verro2GFP genome and 99bp and 81bp deletions at the 5'- and 3'-ends of the (SEQ ID NO:1) Kpnl-linearized pAP1 vector, respectively. The 5'-3' orientation of the EGFP coding sequence (indicated as an arrow) was used to establish the 5'-3' orientation of the pAP1 vector. Upon integration into the host Verro2GFP genome, the pAP1 vector is in the reverse orientation compared to the Sscrofa10.2: Sequence ID: ref|NC_010451.3|, GI:347618785 that maps on chromosome 9.

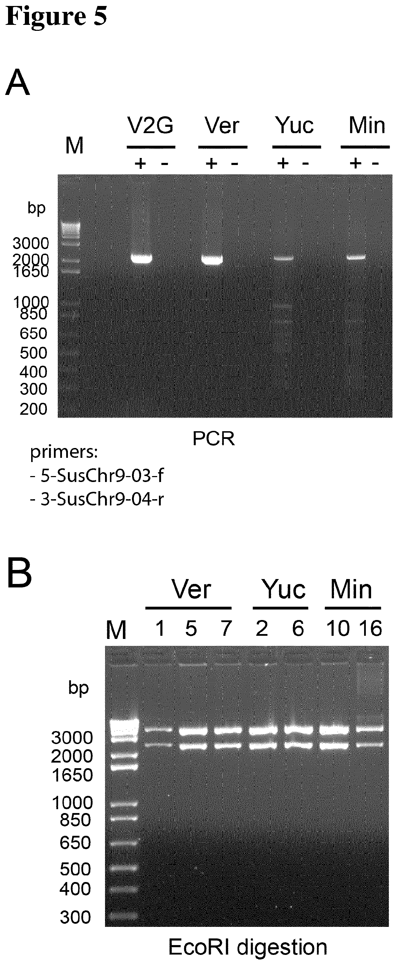

Figure 5: Cloning of the S.scrofa V2G homologous sequences from the genome of (SEQ ID NO:25) Yucatan, (SEQ ID NO:26) Minnesota and (SEQ ID NO:24) Verro2 swine breeds. (A) The 2.3kb genomic DNA fragments of Verro2, Yucatan and Minnesota breeds were PCR-amplified with the (SEQ ID NO: 20) *5-SusChr9-03-f* and (SEQ ID NO:17) *3-SusChr9-04-r* primers. As a positive control, the Verro2GFP genomic DNA carrying one copy of the wild type allele, was amplified. The 2.3kb amplified bands were cloned into pCRII-TOPO vector. (B) To identify positive clones, recombinant plasmids were digested with EcoRI restriction enzyme, resolved on 1% agarose-gel electrophoresis and sequenced by standard procedure. M is the 1kb plus DNA ladder molecular size marker (Life technology).

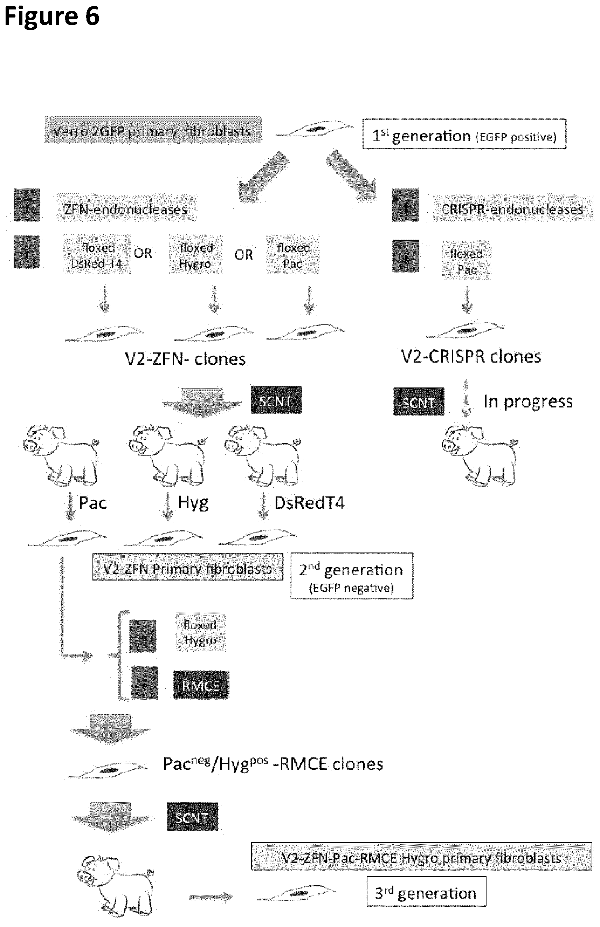

Figure 6: S.scrofa V2G is a safe-harbor locus with high integration rate and durable expression of transgenes. Flow chart of the experimental design:
   The pAP1 vector integrated into the S.scrofa V2G locus was used as a platform for the ZFN- (left side) or CRISPR (right side) - genome editing of the recipient Verro2GFP primary fibroblasts. Homologous recombination promoter-less targeting vectors, (SEQ-ID NO:27) pB5'3'Puro-PL, (SEQ-ID NO:28) pB5'3'Hygro-PL and (SEQ-ID NO:29) pB5'3'RedT4-PL carrying the floxed Pac, Hygro and DsRedT4 genetic markers, respectively, were cotransfected together with EGFP standard ZFN- or (SEQ ID NO:41) CRISPR-endonuclease expressing vectors. Recombinant positive fibroblasts were isolated and used as a source for somatic cell nuclear transfer (SCNT) to generate transgenic pigs. To validate the Recombinase Mediated Cassette Exchange (RMCE) procedure, second generation fibroblasts isolated from these transgenic animals were transfected with floxed-vector confering a different phenotype (change of antibiotic resistance, i.e. Pac to Hygro, or gain/loss of fluorescence, i.e. green to red fluorescence,) together with a standard Cre-recombinase expression vector. Nuclei of RMCE-positive fibroblasts were used to generate transgenic pigs by SCNT. Lastly, third generation of primary fibroblasts were then isolated from the latter transgenic pigs.

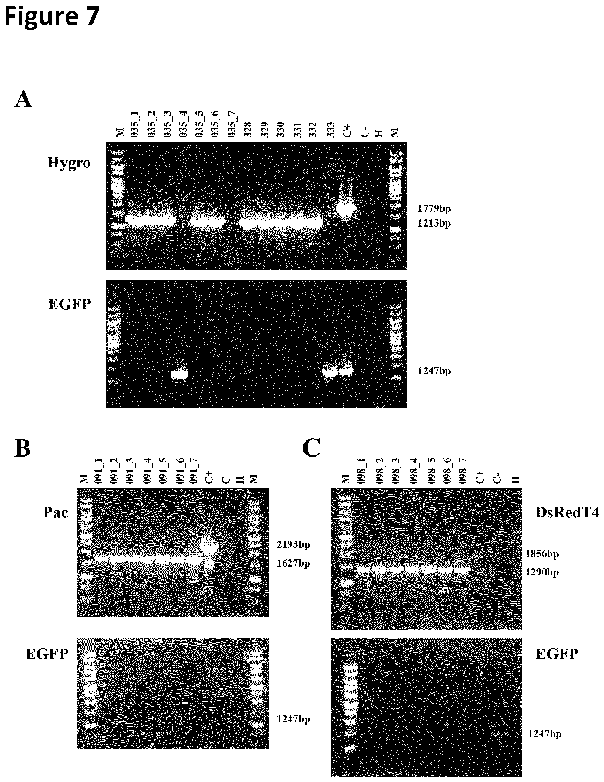

Figure 7: PCR identification of cloned V2ZFN-Hygro animals and cloned V2ZFN-Puro and V2ZFN-DsRedT4 fetuses.
   A. The delivered cloned stillborn (035_1 to 035_7) and live (328 to 333) piglets were PCR analysed to verify the Hygromycin B cassette integration. (Upper panel) Ten out of 13 cloned piglets showed the expected 1213 bp band of the Hygromycin insertion. The 1779 bp band of the positive control (C+) is amplified by the (SEQ-ID NO:33) C+ZFN-Hygro control vector. The negative control (C-) is the Verro2GFP genomic DNA template. (Lower panel) The hygromycin negative, 035_4, 035_7 and 333, piglets showed the 1247 bp EGFP band of the parental Verro2GFP genome. Positive control (C+) is the 1247 bp band of the Verro2GFP genomic DNA. Negative control (C-) is the genomic DNA of Verro2 line. H is the mock control without template. M is the 1kb DNA ladder molecular size marker.
   B. Analysis of the Puromycin resistance (Pac) cassette integration into the S.scrofa V2G locus. The genomic DNA of the seven analysed fetuses, (091_1 to 091_7), showed the 1627 bp band of the Pac cassette (Upper panel). No one amplified the parental genomic 1247 bp band of the EGFP gene (Lower panel). C+ is the template of the (SEQ-ID NO:32) pC+ZFN/Puro3 control vector; C- is the EGFP positive control from the parental Verro2GFP genomic DNA. H is the mock control without template. M is the 1kb DNA ladder molecular size marker. C. PCR analysis of the DsRedT4 cassette integration into the S.scrofa V2G locus. The seven analysed fetuses (098_1 to 098_7) were positive for the detection of DsRedT4 (1856 bp) (Upper panel) and negative for EGFP(1247 bp) bands (Lower panel). C+ is the template of the (SEQ-ID NO:34) C+ZFN-RedT4 control vector. C- is the Verro2GFP line DNA template. M is the 1kb DNA ladder molecular size marker. H is the mock control without template.

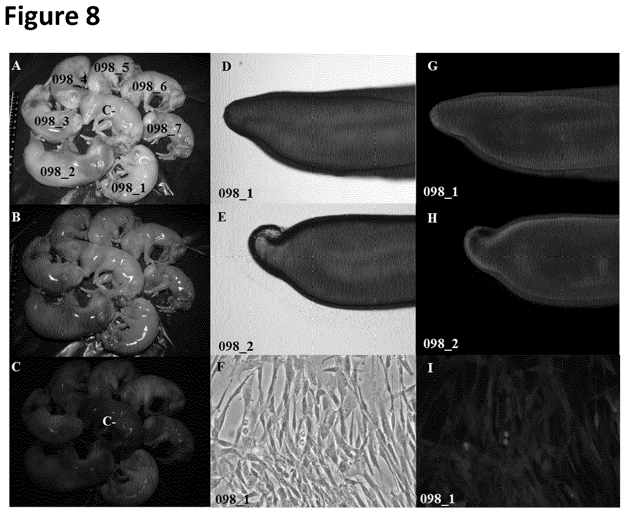

Figure 8: Phenotype analysis of the cloned V2ZFN-DsRedT4 fetuses. The seven DsRedT4 PCR-positive fetuses (098_1 to 098_7) and a negative control (C-) are photographed under a standard white light (Photogram A), or epi-illuminated with the blue (λ=480nm) LED light without any filter (Photogram B), or with EtBr (EDAS120 590DF100/49 600) filter (Photogram C). Note that the spread of high red fluorescence is visible in all the cloned fetuses, whereas no signal is detected on the C- fetus (Photogram C).
   DsRedT4 fluorescent signal is detected on the tails isolated from 098_1 (Photogram G) and 098_2 (Photogram H) fetuses and primary fibroblasts of the 098_1 fetus (Photogram I) by epifluorescent light mounted on Nikon TE-DH100W UV-light inverted microscopy with a G2A long-pass (λ >590nm) filter and a digital imaging system (Nikon DIGITAL SIGHT DS-L1). The same fields are shown in bright field (Photograms D, E and F).

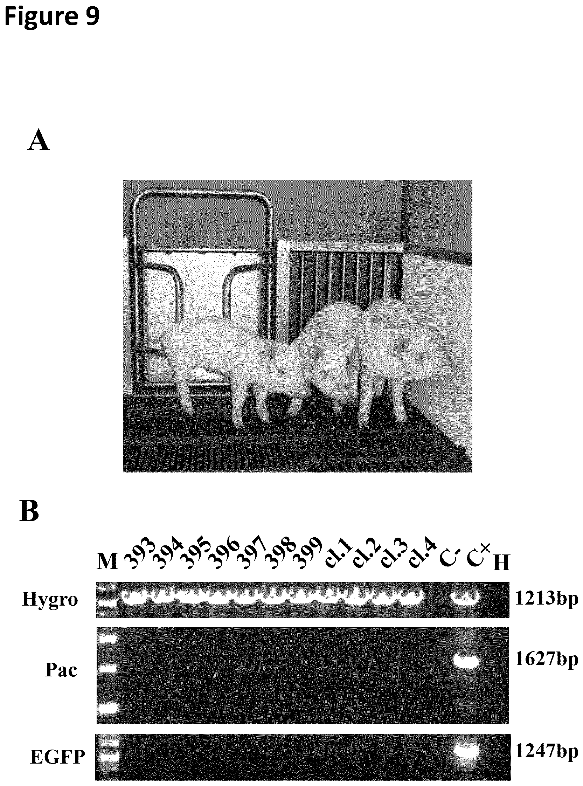

Figure 9: RMCE into the S.scrofa V2G locus. (A) V2Cre-Hygro (# 394, 395 and 399) transgenic cloned piglets are shown. RMCE assay was carried out on V2ZFN-Pac fibroblasts to replace the (SEQ-ID NO:27) pB5'3'Puro-PL floxed Pac cassette integrated into the S.scrofa V2G locus by ZFN-mediated HR with the (SEQ-ID NO:28) pB5'3'Hygro-PL floxed Hygro cassette. Nuclei of positive cells were then used for somatic cell nuclear transfer to generate the transgenic cloned animals shown in A. (B) By PCR screening (see example 14), the Hygromycin positive piglet templates generated the 1213bp amplicon (upper panel). Loss of Pac (middle panel) and EGFP (lower panel) markers was also verified by PCR. C+ is the positive control for each primer set. C- is template from Verro2 line. H is the mock without any template. M is the 1kb DNA ladder molecular size marker.

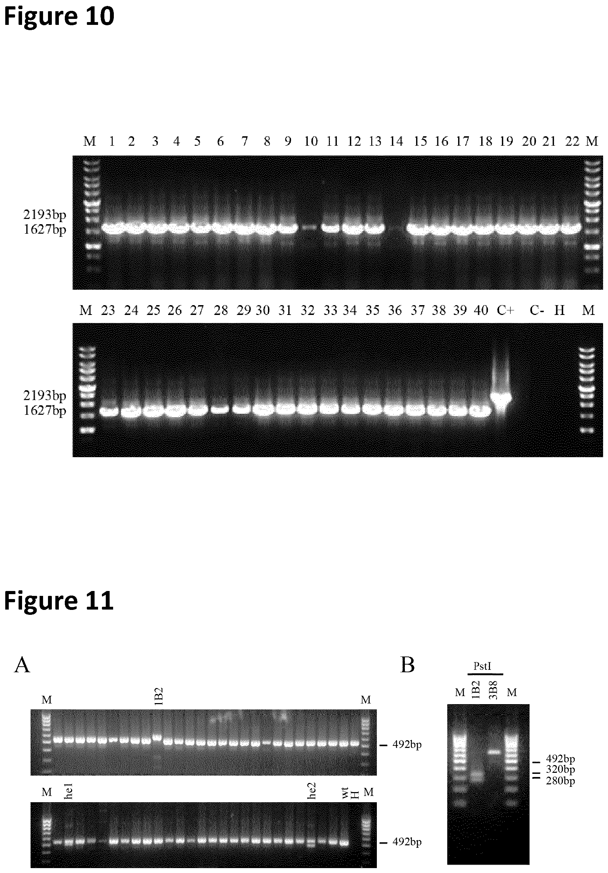

Figure 10: Genome editing of Verro2GFP fibroblasts with CRISPR-endonuclease targeting the EGFP gene. PCR identification of V2Crisprs-Puro colonies.
   The (SEQ ID NO:41) PX330-GFPcr, a CRISPR-endonuclease expression vector targeting the EGFP gene, and the (SEQ-ID NO:27) pB5'3'Puro-PL floxed pac vector were co-transfected into V2GFP fibroblasts and the recombinant clones were selected in puromycin medium. Genomic DNA was isolated from 40 puromycin resistant clones and PCR-screened for the amplification of the expected 1627bp PCR band (see example 15). The 2193bp amplified band is the (SEQ-ID NO:32) pC+ZFN/Puro3 control vector (C+). C- is Verro2GFP template. H is the mock without any template. M is the 1kb DNA ladder molecular size marker.
Figure 11: Genome editing of Minnesota primary fibroblasts with CRISPR-endonuclease targeting the wild type S.scrofa V2G locus. Minnesota primary fibroblasts were cotransfected with the (SEQ ID NO:45) PX330-V2cr3 CRISPR-expression vector together with the (SEQ-ID NO:46) V2cr3-RMCE 120bp single strand DNA oligonucleotide. (Panel A) PCR screening with primers to identify electrophoretic mobility shifted amplicons. The wild type allele generates the 429bp amplicon. Conversely, clone *1B2* (CRISPR-targeted on both alleles) showed an up-shift, whereas clone *he1* and *he2* showed two bands, the wild type and the down-shifted bands, indicating that the shorter (lower band) allele resulted by the endonuclease-assisted homologous recombination. (Panel B) Pstl-RFLP analysis Targeted clones generated the 320bp and 280bp upon Pstl-digestion of the 1B2 amplicon, whereas the up-shifted 3B8 amplicon (>492bp) was not digested. M is the 1kb ladder size marker.

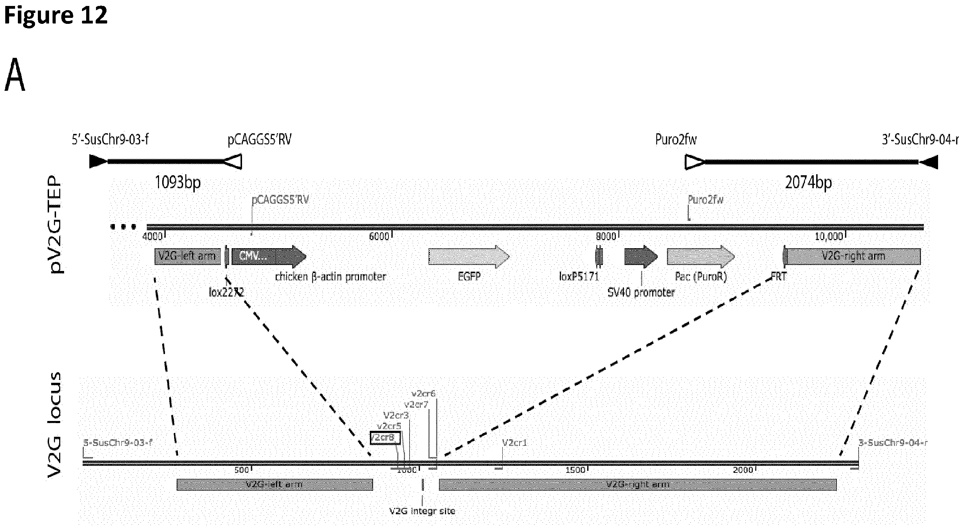

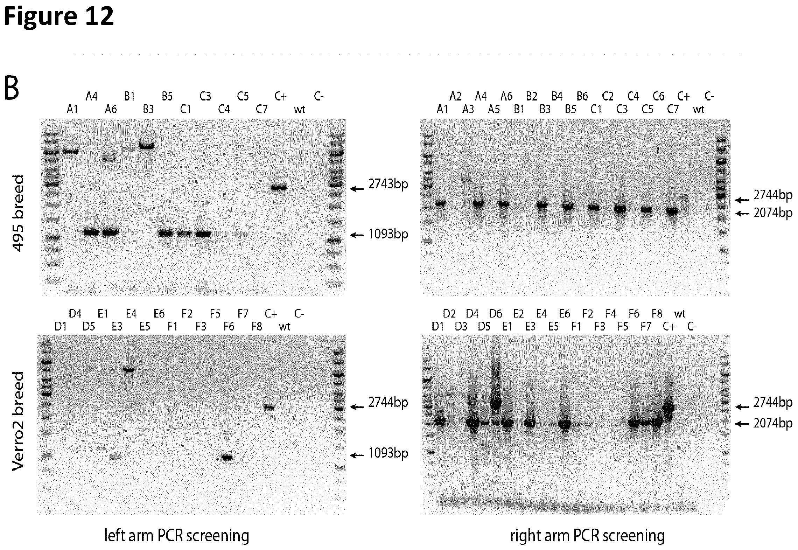

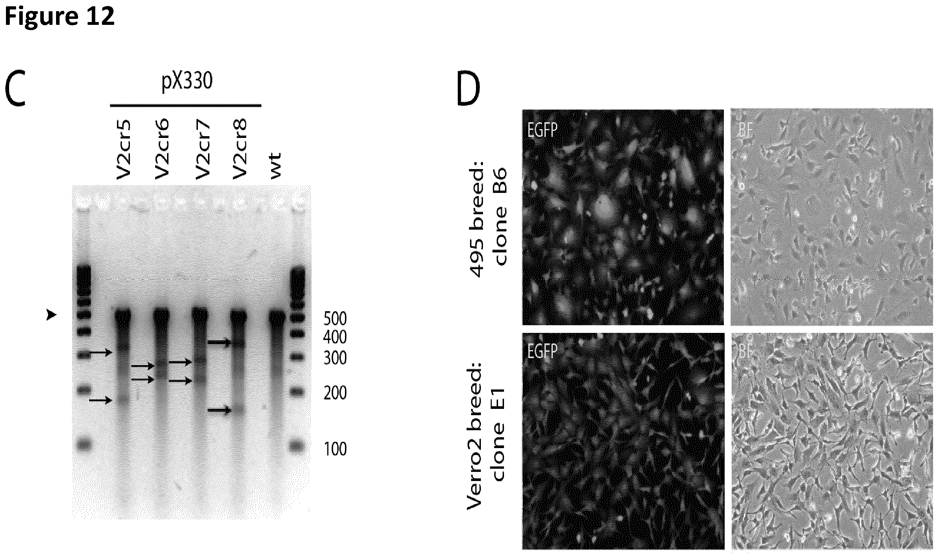

Figure 12: CRISPR/Cas9 gene targeting of the wild type S.scrofa V2G locus in primary fibroblasts of Verro2 and commercial 495 breeds. (A) Schematic representations of the V2G locus and the (SEQ-ID NO:58) pV2G-TEP targeting vector. The left and right V2G homologous arms cloned into the targeting vector are shown between broken lines. The positions of the CRISPR targeting sequences are shown on the V2G locus. Homologous integration of the (SEQ-ID NO:58) pV2G-TEP targeting vector into the V2G locus were assessed by PCR with left and right primer sets. Since each primer set targets the vector (empty arrow) and the genomic V2G DNA (full arrows), only homologous recombination of the targeting vector generates the 1093bp left and 2074bp right amplicons, respectively. (B) Genomic DNA was extracted from transfected 495 and Verro2 clones and amplified by PCR. The indicated 2074bp and 1093bp bands were generated only if homologous recombination events occurred. Amplification of positive controls generated the expected 2744bp and 1093bp bands. (C) DNA electrophoresis of the Surveyor assay to identify efficient CRISPR-espressing pX330 vectors targeting the V2G locus. The arrowhead indicates the wild type 500bp amplicon of the V2G locus, whereas the arrows show the shorter bands of the Surveyor assay upon positive targeting of the V2G locus by the CRISPR-guided endonuclease. The pX330-V2Gcr8 CRISPR expression vector was used for the experiments described in B. (D) Representative Verro2 clone E1 and 495 clone B1 showing the homogeneous Enhanced Green Fluorescence Protein (EGFP) expression in the positively CRISPR-targeted cells. The same fields were photographed in bright field (BF).

### DETAILED DESCRIPTION OF THE INVENTION

The present invention therefore concerns a Sus Scrofa genome specific integration site, defined as "Verro2GFP Chr 9 locus" or "S.scrofa V2G" having the sequence defined in SEQ ID NO:49.

The specific integration site can have sequence identity of at least 95% or at least 97% to the sequence defined in SEQ ID NO:49 and has the advantages of allowing a high efficient site specific targeting of a gene of interest by engineered-endonucleases mediated homologous recombination and/or RMCE, namely recombinase-mediated cassette exchange.

The specific integration site of the invention regards a site of the swine genome, namely S.scrofa V2G, mapped between the serine protease 23 isoform 3 gene (NC_010451.3; gi:347618785) 3986 bp at the 5' side and frizzled 4 gene (NC_010451.3; gi:347618785) 83928bp at the 3' side on the Sus scrofa breed mixed chromosome 9, S.scrofa 10.2. More in detail, the cloned flanking sequences of the S.scrofa V2G integration site were mapped on the Sus scrofa breed mixed chromosome 9, Sscrofa10.2 Sequence ID: ref|NC_010451.3| gi:347618785 region: bp 22916605 - 22918906 as outlined by sequence homology of natural variants isolated from different swine breeds including SEQ ID NO:24 (parental Verro2 breed), SEQ ID NO:25 (Yucatan breed) and SEQ ID NO:26 (Minnesota breed).

More in detail the vector SEQ ID NO:1 was mapped into the Verro2GFP transgenic pig genome at the integration site corresponding to Sus scrofa breed mixed chromosome 9, Sscrofa10.2 Sequence ID: ref|NC_010451.3| gi:347618785 region: bp 22917612-22917613, as indicated in SEQ ID NO:23.

As anticipated above, the S.scrofa V2G has the advantage of allowing (1) a stable and efficient integration of a DNA sequence; (2) long-term and ubiquitous expression of the exogenous transgene. Therefore the transgene is not subjected to cellular variegations (positional effect variegation) and its expression can be tissue- and time-specific, as a consequence of the promoter which is chosen.

One aspect of the invention relates to the natural variants isolated from different swine breeds having the sequences of SEQ ID NO:24, SEQ ID NO:25 and SEQ ID NO:26.

In a further aspect, the invention regards a targeting vector of plasmidic origin integrated into the Sus scrofa genome specific integration site according to the present invention.

The targeting vector can be a site specific targeting vector, and in a preferred aspect the vector has the sequence defined in SEQ ID NO:23, SEQ ID NO:27, SEQ ID NO:28, SEQ ID NO:29 and SEQ ID NO:58, and is integrated into one of the swine specific sites chosen from the group consisting of SEQ ID NO:49, SEQ ID NO:24, SEQ ID NO:25 and SEQ ID NO:26.

The expression vector can be a "floxed" expression vector.

In genetics the term "floxed" is used to describe the sandwiching of a DNA sequence between two "LoxP" sites, and is a contraction of the phrase "flanked by LoxP". The term "floxed" is also extended to all lox variants.

In a still further aspect, the invention relates to the use of the expression vector of plasmidic origin, having the sequences defined in SEQ ID NO:23, SEQ ID NO:27, SEQ ID NO:28 and SEQ ID NO:29 and derivatives, for the expression and/or the stable integration into the host genome of a recombinant gene of interest.

Such a gene can code for example for a polypeptide, an antibody, an enzyme, a DNA vaccine, an antisense or silencer nucleic acid molecule, a microRNA (miRNA), a regulatory RNA molecule, a noncoding RNA, a biosensor, a recombinase, receptor, signaling molecule, transcription factor, pharmaceutically active protein or peptide, a drug target candidate, a disease causing gene product, a toxin, a protein regulating epigenetic functions, a CIS-targeting sequence and/or chromatin organization.

The gene of interest can further be chosen from the group consisting of PAC, Hygro, DsRedT4, EGFP and V2crRMCE (SEQ ID NO:44 and SEQ ID NO:45)

In a still further aspect the invention relates to the use of the vector for the long-term expression and the stable integration into the host genome of a recombinant gene of interest.

Transgenic pigs are very often used in "pig to non-human primates" xenotransplantation experiments to prolong the graft survival into the host by the expression of bioactive human proteins (complement repressors, anti-coagulant and anti-inflammatory factors), as they share many anatomical and physiological characteristics with humans.

In a still further aspect, the invention relates to a site-specific endonuclease target site of the Sus scrofa specific integration site of SEQ ID NO:49, SEQ ID NO:24, SEQ ID NO:25 and SEQ ID NO:26.

The genome editing may be carried out with engineered endonucleases, such as ZFN, TALEN, CRISPR, to site specific targeting a vector of plasmidic origin, carrying a gene of interest, into the Sus scrofa breed mixed chromosome 9, Sscrofa10.2 Sequence ID: ref|NC_010451.3| gi:347618785 region: bp 22916605 - 22918906 or into its natural variants isolated from different swine breeds, as defined in SEQ ID NO:24, SEQ ID NO:25 and SEQ ID NO:26.

Preferably the site-specific endonuclease target site has the sequence defined in SEQ ID NO:44 and SEQ ID NO:48, SEQ ID NO:50, SEQ ID NO:51, SEQ ID NO:52 and SEQ ID NO:53.

By site-specific endo-nuclease mediated HR into the (SEQ ID NO: 23) S.scrofa V2G locus, these vectors allow the integration of a gene of interest flanked by lox sites, moreover once the SEQ ID NO: 27, SEQ ID NO: 28, SEQ ID NO: 29 and derivatives are integrated into S.scrofa V2G locus as above, they can be used as a recipient vector for RMCE site-specific integration of any gene of interest.

### EXAMPLES

- Examples 1-7: Molecular cloning of the S.scrofa V2G locus of Verro2GFP transgenic animal.
- Example 8: Molecular cloning of the S.scrofa V2G locus on the wild type genomes of available swine breeds.
- Examples 9-15: Site-specific targeting of vectors with engineered-endonucleases or RMCE into the S.scrofa V2G locus of Verro2GFP transgenic animals.
- Example 16-17: Site-specific targeting of recombinant DNA sequences into the wild type genome of the S.scrofa V2G locus of any swine breed of interest.

### EXAMPLE 1: Preparation of the Splinkerette linkers

To identify the genomic flanking region of the pAP1 vector integrated into the genome of the Verro2GFP transgenic pig [3], the splinkerette ligation-mediated PCR method was applied whose adaptors minimize off-target amplifications by forming intrastrand hairpin loops [4].

Since the pAP1 plasmid was linearized with Kpnl restriction enzyme and transfected into Verro2 cells, a bioinformatics analysis of the vector allowed to choose the 4-bp restriction enzyme cutters, Apol and Dpnll that digest the vector nearby the 5'- and 3' ends of the (SEQ ID NO:1) pAP1 vector. These enzymes released the plasmidic/genomic chimeric sequences of the host genomic integration site that were amplified by PCR with specific primers. Outward and inward primers, distally mapped to the restriction sites, were defined to target the 5'- and 3' end sequences of the (SEQ ID NO:1) pAP1 vector (see EXAMPLE 2).

Moreover, the splinkerette double strand DNA linkers were generated by annealing the bottom strand oligonucleotide: (SEQ ID NO:2) SPLNK-BOT2 -(5'- CGA AGA GTA ACC GTT GCT AGG AGA GAC CGT GGC TGA ATG AGA CTG GTG TCG ACA CTA GTG G -3') either with the ApoI-upper strand 5': (SEQ ID NO:3) SPLNK-AATT-EcoR (5'-AAT TCC ACT AGT GTC GAC ACC AGT CTC TAA TTT TTT TTT TCA AAA AAA-3') oligonucleotide to generate the Apol splinkerette linker or with the Dpnll-upper strand: (SEQ ID NO:4) - (5'-GAT CCC ACT AGT GTC GAC ACC AGT CTC TAA TTT TTT TTT TCA AAA AAA-3') oligonucleotide to generate the Dpnll splinkerette linker, respectively.

The annealing reaction was carried out by mixing the oligonucleotide primers (40µM each) in the annealing buffer: 10 mM Tris-HCl (pH 7.4), 5 mM MgCl₂, heating at 65°C for 10 min and cooling to 4°C at the 1 °C/min rate in a thermalcycler.

### EXAMPLE 2: Validation of primers targeting the 5'- ends of the (SEQ ID NO:1) pAP1 vector integrated into the Verro2GFP genome.

The splinkerette template DNA was purified from Verro2GFP primary fibroblasts. To this aim, high molecular weight DNA was purified with a standard procedure and about 1µg was digested with either Apol or Dpnll restriction enzymes (6 IU/µg) (New England Biolabs) for 6 h at 37 °C.

Upon digestion, DNA was precipitated in 0.3M Na Acetate pH 5.3 / 70% ethanol and resuspended in 35µl 0.1xTE (1mM Tris-HCI ph 7.5, 0.1 mM EDTA pH 8.0).

Aliquots (about 140 ng) of Apol and Dpnll -digested DNAs were assayed as templates to validate the outward primers targeting the integrated (SEQ ID NO:1) pAP1 vector. Therefore, the outward primers (SEQ ID NO:5) *05_splinkVerr_rv* (5'-AGG CCA CCA CTT CAA GAA CTC TG-3') and (SEQ ID NO:6) *03_splinkVerr_rv*: (5'-AAC TGA GAT ACC TAC AGC GTG AGC-3') were challenged with the inward primer (SEQ ID NO:7) *02_SplinkVerr_fw*: (5'-CCT GGC GTT ACC CAA CTT AAT C-3') to generate the expected PCR products of 508 bp (PCR1) and 707 bp (PCR2), respectively.

The PCR reactions were carried out by mixing about 140 ng ApoI or DpnII -digested DNAs, 5x PCR buffer, 0.4µM primers, 200µM dNTPs, 0.02U/ µl Phusion polymerase (BioLabs) and incubated in Eppendorf thermal cycler for 1minute at 98°C, followed by 25 cycles of amplification rounds each of which was 10 seconds at 98°C, 30 seconds at 61 °C, 45 seconds at 72°C, and by 1 cycle for 5 minutes at 72°C. The PCR products were resolved on 1.5% Agarose TBE gel electrophoresis.

### EXAMPLE 3: Splinkerette assay on the pAP1 genomic integration site of the Verro2GFP transgenic pig.

ApoI- and DpnII -digested DNA fragments (about 140 ng) were ligated with the Apol- and DpnII-splinkerette linkers, respectively, in a 50-µl ligation reaction containing 1× ligase buffer, 0.5 µM splinkerette linkers and 1 unit T4 DNA ligase (New England Biolabs) at 16° C for 16 h. Then, splinkerette-ligated DNA (about 14 ng) templates were amplified with nested primers to isolate the genomic flanking region of the integrated vector. The three consecutive PCR reactions were carried out as follows:

### PCR 1:

### Primers:

(SEQ ID NO:5) *05_splinkVerr_rv*: (5'-AGG CCA CCA CTT CAA GAA CTC TG-3')
(SEQ ID NO:8) *SPLNK#1_S1*: (5'- CGA AGA GTA ACC GTT GCT AGG AGA GAC C-3')

### Template:

14 ng Apol- or Dpnll- splinkerette ligated DNA

The PCR protocol was:
1 cycle at 94°C for 75 seconds;
2 cycles of 94°C 20 seconds, 64°C 15 seconds;
35 cycles of 94°C 20 seconds, 62.4°C 15 sec, 72°C 150 seconds;
1 cycle at 72°C for 7 minutes.

### PCR 2:

### Primers:

(SEQ ID NO:5) *05_splinkVerr_rv*: (5'-AGG CCA CCA CTT CAA GAA CTC TG-3')
(SEQ ID NO:9) *SPLNK#2_S2*: (5'-GTG GCT GAA TGA GAC TGG TGT CGA C-3')

### Template:

1µl of 1:5 diluted PCR 1 mix

### Amplification conditions:

1 cycle at 94°C for 120 seconds;
35 cycles: each cycle was 94°C for 20 seconds, 62.4°C for 15 sec, 72°C for 120 seconds;
1 cycle at 72°C for 7 minutes.

### PCR 3:

### Primers:

(SEQ ID NO:6) *03_splinkVerr_rv*: (5'-AAC TGA GAT ACC TAC AGC GTG AGC-3')
(SEQ ID NO:9) *SPLNK#2_S2*: (5'-GTG GCT GAA TGA GAC TGG TGT CGA C-3')

### Template:

1µl of 1:5 diluted PCR 2 mix

### Amplification conditions:

1 cycle at 94°C for 120 seconds;
35 cycles: each cycle was 94°C for 20 seconds, 62.4°C for 15 sec, 72°C for 120 seconds;
1 cycle at 72°C for 7 minutes.

All the PCR reactions were set up by mixing the indicated templates and primers as described above with 5x PCR buffer, 0.6 µM primers, 500µM dNTPs, 3,5 IU Long Range Polymerase (Roche).

The PCR products were resolved on 1.5% Agarose-TBE gel electrophoresis, purified by Qiaquick Gel extraction kit (Qiagen) procedure and cloned into pCRII-TOPO vector (Life-Technology) (FIGURE 1). E.coli Mach 1 competent cells (Life-Technology) were transformed with the recombinant plasmids.

### EXAMPLE 4: Cloning the swine genomic (SEQ ID NO:12) Apo I - and (SEQ ID NO:13) Dpn II - splinkerette fragments.

Cloned plasmids were purified from bacterial cells by quick mini prep extraction procedure. Briefly, colonies grown on LB (Luria Broth) medium containing 1% agar and 100µg/ml Ampicillin (Sigma) were inoculated in about 1ml LB containing ampicillin (100µg/ml) and growth over night at 37°C in a shaker. Each bacterial pellet was dissolved in 100µl Qiagen buffer P1 (Qiagen), alkaline lysed 5 minutes by adding 100µl Qiagen buffer P2 and the mix was neutralized 5 minutes on ice by adding 100µl Qiagen buffer P3. Lysates were clarified by eppendorf centrifugation 10 minutes at 13krpm, the supernatants were 70% ethanol precipitated and plasmid DNA pellets were resuspended in about 30µl 0.1xTE. Positive recombinant plasmids were identified by digestion with EcoRI restriction enzyme and oriented by PCR with the (SEQ ID NO:10) *M13for* (5'-TTG TAA AAC GAC GGC CAG T-3') or (SEQ ID NO:11) *M13rev* (5'- CAG GAA ACA GCT ATG AC-3') universal primers targeting the pCRII-TOPO vector and the (SEQ ID NO:6) *03_splinkVerr_rv* (5'-AAC TGA GAT ACC TAC AGC GTG AGC-3') targeting the 5'-end of the (SEQ ID NO:1) pAP1 vector (FIGURE 1, C and D). The PCR mix was 1µl purified plasmid template, 5x PCR buffer, 0.4µM primers, 200µM dNTPs, 0.02U/ µl Phusion polymerase (BioLabs), PCR grade water. The following thermal cycler protocol was applied: 98°C 60 seconds, 25 cycles of 98°C 10 seconds, 55°C 60 seconds, 72°C 90 seconds followed by 72°C 7 minutes.

At least two DNA inserts of both (SEQ ID NO:12) Apol- and (SEQ ID NO:13) Dpnll splinkerette positive clones were sequenced by standard commercial procedures (http://www.eurofinsgenomics.eu) with universal standard primers, (SEQ ID NO:10) *M13for and* (SEQ ID NO:11) *M13rev.* BLAST alignment of the splinkerette cloned sequences allowed to identify the DNA sequences that share homology with the swine *Sus scrofa v10.2* genome and with the 5'-end of the (SEQ ID NO:1) pAP1 vector.

### EXAMPLE 5: Validation of the identified flanking sequence by PCR analysis on the Verro2GFP genomic DNA.

To validate the identified pAP1 flanking genomic sequence on the Verro2GFP genomic DNA, 4 PCR reactions were set up with the following nested primers, namely (SEQ ID NO:14) *3-SusChr9-01-r (5'-*AAC GCT TTG GAG TCA GGA CAC CT-3'), (SEQ ID NO:15) *3-SusChr9-02-r* (5'-CTC TGC CAA GAC TGC TTC TCC ATC C-3'), (SEQ ID NO:16) *3-SusChr9-03-r* (5'- CTT CCT CTG TGC TTT CAT CAT GCC TTG-3'), (SEQ ID NO:17) *3-SusChr9-04-r* (5'- GCC TGG CAC AAA GCC TTC TTC TC-3') targeting the genomic swine DNA nearby the integration site of the (SEQ ID NO:1) pAP1 vector and the outward pAP1 primer (SEQ ID NO:6) *03_splinkVerr_rv* (5'-AAC TGA GAT ACC TAC AGC GTG AGC-3') to generate the expected 666 bp PCR1, 850 bp PCR2, 1734bp PCR3 and 1800 bp PCR4 products, respectively (FIGURE 2).

The PCR reaction mix was made with about 200 ng genomic DNA purified from Verro2GFP fibroblasts, 5x PCR buffer, 0.4µM primers, 200µM dNTPs, 0.02U/ µl of Phusion DNA polymerase (BioLabs), PCR grade water.

The PCR protocols of PCR1 ((SEQ ID NO:14) *3-SusChr9-01-r* / (SEQ ID NO:6) *03_splinkVerr_rv)* and PCR2 (SEQ ID NO:15) *3-SusChr9-02-r* / (SEQ ID NO:6) *03_splinkVerr_rv)* were: 98° C 60 seconds; 34 cycles of 98°C 10 seconds, 62,4° C 30 seconds; 72°C 50 seconds; followed by 72°C 5 minutes.

The PCR protocols of PCR3 (SEQ ID NO:16) *3-SusChr9-03-r* / (SEQ ID NO:6) *03_splinkVerr_rv)* and PCR4 ((SEQ ID NO:17) *3-SusChr9-04-r* / (SEQ ID NO:6) *03_splinkVerr_rv)* were: 98°C 60 seconds; 34 cycles of 98°C 10 seconds, 62,4°C 30 seconds; 72°C 110 seconds; followed by 72°C 5 minutes.

### EXAMPLE 6: Cloning of the swine 5' flanking sequence of the (SEQ ID NO:1) pAP1 vector integrated into Verro2GFP genome.

To isolate the swine 5'-flanking sequence of the (SEQ ID NO:1) pAP1 vector integrated into Verro2GFP genome, the following (SEQ ID NO:18) *5-SusChr9-01_f* (5'- CTC TCT GAT GCA GCA GGA ATC TCT AGT C-3'), (SEQ ID NO:19) *5-SusChr9-02-f* (5'- CTA CCG AAG ACG AAC TCT GCT CCA AAC-3'), (SEQ ID NO:20) *5-SusChr9-03-f* (5'- CTC CTT TGA CAA GGT CTA GTG GAT GG-3') nested primers targeting the swine genome at the putative 3'-end of the (SEQ ID NO:1) pAP1 integration site were assayed with the (SEQ ID NO:14) *3-SusChr9-01-r* (5'-AAC GCT TTG GAG TCA GGA CAC CT -3') targeting the 3'end flanking the (SEQ ID NO:23) integration site of Verro2GFP. To this aim, the wild type allele of the Verro2GFP genome in which the pAP1 was integrated in single copy, was used as template to assay the PCR conditions.

The PCR reaction was set up by mixing about 200 ng template of genomic DNA isolated from Verro2 wild type fibroblasts, 5x PCR buffer, 0.4µM primers, 200µM dNTPs, 0.02U/ µl Phusion Polymerase, PCR grade water to generate the expected 287 bp PCR1 and 730 bp PCR2 amplified products, respectively.

The PCR protocols was 98°C 60 seconds, 34 cycles of 98°C 10 seconds, 62,4°C 30 seconds, 72°C 25-50 seconds, followed by 72°C 5 minutes.

The above primers generated the expected amplified products on the wild type genomic sequence. To isolate the 5'-flanking sequence of the pAP1 integrated vector, the (SEQ ID NO:19) *5-SusChr9-02-f* and the (SEQ ID NO:20) *5-SusChr9-03-f* primers were challenged with the outward primer (SEQ ID NO:21) *SPKV2GFP-02_fw* (5'- GCT TTT TGC TTC CTC TTG CAA AAC CAC -3') to target the (SEQ ID NO:1) pAP1 vector on the Verro2GFP genomic DNA. Each PCR reaction was set up with about 200 ng template of Verro2GFP genomic DNA, 5x PCR buffer, 0.4µM primers, 200µM dNTPs, 0.02U/ µl Phusion Polymerase, PCR grade water. The PCR protocols was:
- *PCR1:*
   Primers: (SEQ ID NO:19) *5-SusChr9-02_f* and (SEQ ID NO:21) *SPKV2GFP-02_fw*
      98°C 60 seconds, 34 cycles of 98°C 10 seconds, 63°C 30 seconds; 72°C 100 seconds followed by 72°C 5 minutes.
- *PCR2:*
   Primers: (SEQ ID NO:20) *5-SusChr9-03_f* and (SEQ ID NO:21) *SPKV2GFP-02_fw*
      98°C 60 seconds, 34 cycles of 98°C 10 seconds, 63°C 30 seconds; 72°C 100 seconds followed by 72°C 5 minutes.

The PCR reactions generated two PCR products of 838 bp (PCR1) and the (SEQ ID NO:22) 1124 bp (PCR2) length, respectively (FIGURE 3).

The (SEQ ID NO:22) PCR2 (1124 bp) product was fractionated on 1.5% agarose gel electrophoresis, purified from the gel, and cloned into pCRII-TOPO vector as described in EXAMPLE 3. The recombinant plasmids were transformed in E.coli Mach1 cells and alkaline purified, as described in EXAMPLE 3. By PCR screening with (SEQ ID NO:10) *M13 for* or (SEQ ID NO:11) *M13 rev* primer targeting the pCRII-TOPO vector together with the (SEQ ID NO:19) *5'-susChr9_02-f* primer targeting an internal region of the (SEQ ID NO:22) PCR2 cloned sequence, positive clones were identified and sequenced.

The mix (25µl) was made with 1µl template of each recombinant plasmid, 5x PCR buffer, 0.4µM primers, 200µM dNTPs, 0.02U/ µl Phusion Polymerase, PCR grade water. The following PCR protocol was applied: 98°C 120 seconds, 25 cycles of 98°C 10 seconds, 56°C 60 seconds; 72°C 70 seconds, followed by 72°C 7 minutes.

The (SEQ ID NO:22) PCR2 DNA inserts of the clones were sequenced with universal standard primers (SEQ ID NO:10) *M13for* and (SEQ ID NO:11) *M13rev* by standard commercial procedure. These sequences were aligned both against the swine genome (*S.scrofa v10.2*) and the (SEQ ID NO:1) pAP1 plasmid sequences by BLAST (http://blast.ncbi.nlm.nih.gov/Blast.cgi). Bioinformatics analysis confirmed that the (SEQ ID NO:22) cloned DNA fragment spans over the joining sequences between swine gDNA and the 3'-end sequence of the (SEQ ID NO:1) pAP1 vector.

### EXAMPLE 7: Description of the S.scrofa V2G locus.

BLAST analysis of the 5'- and 3'- flanking sequences (SEQ ID NO:23) of the pAP1 integration site of the Verro2GFP revealed 97% and 99% identity, respectively, with the Sequence ID: ref|NC_010451.3| GI:347618785: 22916605-22918906, Sus scrofa breed mixed chromosome 9, Sscrofa10.2.

More in detail, the integration of the pAP1 vector led to the loss of the TT dinucleotide mapped at bp 22917612-22917613 of this genomic sequence, whereas a C to T change (SNP) was observed at bp 22917611 between the (*Sus scrofa v10.2*) Swine Reference sequence and the Verro2 genomic sequence. The KpnI-linearized (SEQ ID NO:1) pAP1 vector was integrated into the *S.scrofa* V2G locus in the reverse orientation compare to the 5'-3' orientation of the ref|NC_010451.3|, GI:347618785 genomic sequence. The pAP1 genomic integration caused the 99 bp and 81bp deletions at the 5'-and 3'-ends of the KpnI-linearized (compare SEQ ID NO:1 with SED ID NO:23) pAP1sequence, respectively (FIGURE 4).

### EXAMPLE 8: Cloning the S. scrofa V2G- homology region of Verro2, Minnesota and Yucatan swine breeds.

The wild type 2.3kb genomic region spanning over the *S.scrofa* V2G integration site was cloned from the parental Verro2 genome by PCR with the (SEQ ID NO:17) *3-SusChr9-04-r* (5'- GCC TGG CAC AAA GCC TTC TTC TC-3') and (SEQ ID NO:20) *5-SusChr9-03-f* (5'- CTC CTT TGA CAA GGT CTA GTG GAT GG-3') primers (FIGURE 5).

BLAST analysis revealed that the (SEQ ID NO:24) 2.3kb Verro2 genomic sequence shares 98% identity with Sus scrofa breed mixed chromosome 9, Sscrofa10.2 Sequence ID: ref|NC_010451.3| gi:347618785: 22916605-22918906. This sequence maps between serine protease 23 isoform 3 gene (NC_010451.3; gi:347618785) 3986 bp at the 5' side and frizzled 4 gene (NC_010451.3; gi:347618785) 83928 bp at the 3' side. As expected, the BLAST alignment showed 100% identity between the (SEQ ID NO:23) S.scrofa V2G genomic flanking sequence of Verro2GFP and the (SEQ ID NO:24) S.scrofa V2G locus of the parental Verro2 wild type genome. The cloned 2.3kb S.scrofa V2G sequence shares two blocks of homology with the Homo sapiens genomic DNA, chromosome 11q, Sequence ID: ref|NC_000011.10|: Range 1: 86867239 to 86867588 (78% identity) and Range 2: 86869109 to 86869198 (82% identity). As shown for the S. scrofa genomic sequence, this homologous human sequence maps between the serine protease 23 precursor (NC_010451.3; gi:347618785) 58427bp at 5' side and the frizzled-4 precursor gene (NC_010451.3; gi:347618785) 83409bp at the 3' side, revealing a certain degree of synteny between the two genomes. The genomic homologous regions of the (SEQ ID NO:25) Yucatan and the (SEQ ID NO:26) Minnesota swine breeds were also cloned (FIGURE 5). Their sequences shared 98% and 99% identity, respectively, with the Sus scrofa breed mixed chromosome 9, Sscrofa10.2 Sequence ID: ref|NC_010451.3| gi:347618785 (bp 22916605 - 22918906).

The PCR reactions were made with about 100ng gDNA templates purified from Verro2, Yucatan or Minnesota wild type fibroblasts, 5x PCR buffer, 0.4µM primers, 200µM dNTPs, 0.02U/ µl Phusion Polymerase, PCR grade water. The PCR protocol was 98°C 60 seconds, 34 cycles of 98°C 10 seconds, 64,2°C 30 seconds, 72°C 150 seconds, followed by 72°5 minutes. The electrophoretic bands of about 2.3kb corresponding to the amplified PCR products from each genome (FIGURE 5 A) were cloned into pCRII-TOPO vector and the E.coli Mach 1 competent cells were transformed with the recombinant plasmids as described in EXAMPLE 3. Positive recombinant plasmids were identified by standard EcoRI restriction analysis that released the expected 2.3 kb DNA fragments (FIGURE 5B). At least two independent recombinant clones containing the genomic sequences of each swine breeds were sequenced by standard procedures to cover the entire 2.3kb genomic sequences.

### EXAMPLE 9: S.scrofa V2G is a safe-harbor locus with high integration rate and long-term expression of transgenes - Experimental design.

In this section, the experimental design (FIGURE 6) is briefly outlined to demonstrate that S.scrofa V2G is a safe-harbor locus with high integration rate and durable expression of transgenes.

Verro2GFP primary fibroblasts (*1^{st} generation of primary cells*) were subjected to sequential double recombination strategy aimed at integrating several different genetic markers. Endonuclease-enhanced Homologous Recombination (HR) was employed to knock-in floxed markers into the S.scrofa V2G locus (*2^{nd} generation of primary cells*)*.* Floxed genes were then assayed by exchanging RMCE cassettes (3^{rd} *generation of primary cells)* (FIGURE 6).

### Endonuclease-assisted Homologous Recombination to knock in genetic marker into the S.scrofa V2G-locus.

ZFN-endonucleases were employed to knock in promoter-less *pac*, *hygro* or *DsRedT4* gene into the pAP1 vector that is stably integrated into the S.scrofa V2G- locus (EXAMPLE 10). These markers were flanked by the Cre-heterologous target sites, Iox2272 and lox5171, to allow site-specific recombination events leading to cassette exchange. CRISPR-endonucleases (EXAMPLE 15) were also assayed to knock in the floxed *pac* marker into the same locus. The replacement of the EGFP gene by endonuclease mediated homologous recombination generated clones that were negative to green fluorescent light emission and able to grow in hygromycin (*hygromycin* gene) (See EXAMPLE 11) or in puromycin (*pac* gene) (See EXAMPLE 12 and 15) selective medium, or emitting a red fluorescent light *(DsRedT4* gene) (See EXAMPLE 13), respectively. Nuclei were then isolated from these recombinant clones and the corresponding transgenic piglets were generated by SCNT. Second generation (*2^{nd} gen of fibroblasts*) fibroblasts carrying one of the above markers were then isolated from the transgenic piglets.

### Site-specific recombination by RMCE into the S.scrofa V2G locus.

Second generation fibroblasts, namely the V2-ZFN-Pac, were then challenged by Cre-RMCE (Cre-Recombinase Mediated Cassette Exchange) to exchange the floxed-pac marker with the floxed Hygro RMCE cassette into the S.scrofa V2G- locus. Nuclei of positive RMCE cells were then used to generate transgenic pigs by SCNT and the relative primary fibroblasts (*3^{rd} generation of primary cells)* were isolated from the newborn piglets (EXAMPLE 14).

### CRISPR-endonuclease mediated knock-in of a genetic marker into the S.scrofa V2G genomic site of wild type swine cells.

To extend the use of the S.scrofa V2G safe harbor locus to other swine breeds, the Minnesota swine homologous sequence was targeted by CRISPR-endonucleases and the floxed pac marker was knocked in. Recombinant positive clones were then isolated and their relative nuclei may be used to generate transgenic pigs by SCNT as above (EXAMPLE 16).

### EXAMPLE 10: Construction of the floxed promoterless targeting/exchanging vectors used in the endonuclease-mediated homologous recombination and recombinase-mediated cassette exchange experiments.

Recent reports demonstrated that EGFP-specific endo-nucleases could dramatically improve the efficiency of homologous recombination (HR) process [5, 6].Therefore, site specific nucleases (ZFNs and CRISPRs) targeting the EGFP locus of the S.scrofa V2G integrated pAP1 vector were applied to knock in promoterless floxed genetic markers. Three targeting vectors, namely (SEQ ID NO:27) pB5'3'Puro-PL, (SEQ ID NO: 28) pB5'3'Hygro-PL and (SEQ ID NO: 29) pB5'3'RedT4-PL were constructed by cloning into the pBSKSII+ plasmid (Stratagene) the *pac,* the *hygromycin* selectable markers or the gene coding for the red fluorescent protein, DsRedT4, respectively. These genetic markers were flanked by the lox2272 and by the lox5171 Cre-targeting sites at the 5' and 3' ends, respectively. Moreover, the *Apa*I*-Eco*RI 860 bp DNA fragment of the pAP1 parental vector pCXEGFP [7]was cloned at the 5' end of the lox2272 sequence, while the *BglII-HindIII 464* bp-long DNA fragment was cloned at the 3' end of the lox5171 sequence to generate the 5' and 3'-arms of the targeting vectors, respectively.

To set up the experimental conditions for the genetic screening of endonuclease-mediated HR clones, three different control sequences containing the used genetic markers (Pac, Hygromycin and DsRedT4) were also constructed. More in detail, the (SEQ ID NO: 30) 566 bp long EcoRI DNA spacer was isolated from the human EPCR cDNA ref|NM_006404.4| region: bp 169-761 and cloned into the EcoRI site of the (SEQ ID NO:27) pB5'3'Puro-PL vector to generate the pC+5'Puro-PL intermediate vector. Next, the (SEQ ID NO:31) *Sal*I-*Apa*I fragment carrying the CAG hybrid promoter of the pCXEGFP vector was cloned into the pC+5'Puro-PL vector to generate the (SEQ ID NO: 32) C+ZFN/Puro3 vector. By *in vitro* Cre-mediated RMCE, the floxed control *pac* gene of (SEQ ID NO: 32) C+ZFN/Puro3 was replaced with the floxed hygromycin resistance gene of the (SEQ ID NO:28) pB5'3'Hygro-PL or with the floxed DsRedT4 of the (SEQ ID NO:29) pB5'3'RedT4-PL constructs to generate the (SEQ ID NO:33) C+ZFN/Hygro and (SEQ ID NO:34) C+ZFN/RedT4 control vectors, respectively. Next, these control constructs were used as templates to validate the primer sets, in particular the (SEQ ID NO:35) *RTtest5'UTRfw* (5'-TCTGACTGACCGCGTTACTCC-3') primer was employed together with the following reverse primers: (SEQ ID NO:36) *EGFP-rv* (*5'-*TGCTTCATGTGGTCGGGG-3'), (SEQ ID NO:37) *HR5Hygro-rv* (5'-ATCGGCGCAGCTATTTACCC-3'), (SEQ ID NO:38) *Puro2-rv* (5'-CTTGCGGGTCATGCACCAGG-3') and (SEQ ID NO:39) *HR5Red-rv* (*5'-*TTCTTGTAGTCGGGGATGTCGG-3'). PCR conditions were established as described in the following examples.

### EXAMPLE 11: Generation of cloned V2ZFN-Hygro animals.

Verro2GFP primary (10^6) fibroblasts were co-transfected (Nucleofector, program V-024, Amaxa) with 2µg each plasmid (each) expressing the ZFN-endonucleases specifically targeting the EGFP sequence (CompoZR®-Sigma; [5, 8, 9] and 1µg promoterless Hygromycin targeting vector, (SEQ ID NO:28) pB5'3'Hygro-PL. The resulting hygromycin resistant colonies were PCR screened with the (SEQ ID NO:35) *RTtest5'UTR-fw* (5'-TCTGACTGACCGCGTTACTCC-3') and (SEQ ID NO:37) *HR5Hygro-rv* (5'-ATCGGCGCAGCTATTTACCC-3') primer set. Positive clones were used in 2 zona-free SCNT experiments [10] . After nuclear transfer, all the recipient sows became pregnant. One saw went to term by delivering 13 piglets (6 live and 7 stillborn). Biopsies were taken and primary cell lines established from each piglets. Next, genomic DNA was purified from these cells and PCR screenings were carried out with the (SEQ ID NO:35) *RTtest5'UTR-fw* (5'-TCTGACTGACCGCGTTACTCC-3') and (SEQ ID NO:37) *HR5Hygro-rv* (5'-ATCGGCGCAGCTATTTACCC-3') primer set to detect the integrations of the Hygromycin cassette into the S.scrofa V2G locus, whereas the (SEQ ID NO:35) *RTtest5'UTR-fw* (5'-TCTGACTGACCGCGTTACTCC-3') and (SEQ ID NO:36) *EGFP-rv* (*5'-*TGCTTCATGTGGTCGGGG-3') primer set was also employed to verify the replacement of the recipient EGFP cassette. Each PCR reaction was set up by mixing about 200 ng template of genomic DNA isolated from fibroblasts colonies or pig tissues, 1x PCR GCI buffer, 0.8µM primers, 400µM dNTPs, betaine 1M, 0.05U/µl LA-Taq polymerase, PCR grade water to generate the expected PCR amplified product. The PCR protocol for each target was:
94°C 2 minutes, 10 cycles of 94°C 30 seconds, 65°C (-1 °C/cycle) 30 seconds, 72°C 1 minute and 45 seconds, 30 cycles of 94°C 30 seconds, 55°C 30 seconds, 72°C 1 minute and 45 seconds, followed by 72°C 7 minutes.
- *Hygro detection:*
   Primers: (SEQ ID NO:35) *RTtest5'UTR-fw* (5'-TCTGACTGACCGCGTTACTCC-3') and (SEQ ID NO:37) *HR5Hygro-rv* (5'-ATCGGCGCAGCTATTTACCC-3').
   Amplicon = 1213 bp for positive samples and 1779 bp for positive control.
- *EGFP detection:*
   Primers: (SEQ ID NO:35) *RTtest5'UTR-fw* (5'-TCTGACTGACCGCGTTACTCC-3') and (SEQ ID NO:36) *EGFP-rv* (5'-TGCTTCATGTGGTCGGGG-3').
   Amplicon = 1247 bp.

Ten (5 live+5 stillborn) out of 13 delivered piglets were positive for Hygromycin detection and negative for EGFP upon the PCR assay (FIGURE 7 A). The amplified DNA products were sequenced with the (SEQ ID NO:40) *INTRrev* (5'-GGAGCGCACAAAGCCCCG-3') primer to verify the presence of the intron sequence of pCXEGFP promoter upstream of the *Apa*I integration site which was involved in the HR events. Sequence alignment of the PCR products showed 100% identity in 9 out of 10 piglet gDNAs, whereas a G to A SNP was detected into the stillborn animal 035_5 genomic sequence.

### EXAMPLE 12: Generation of cloned V2ZFN-Puro fetuses.

Verro2GFP primary fibroblasts were co-transfected with ZFN-endonucleases targeting the EGFP sequence and the promoterless Puromycin targeting vector, (SEQ ID NO:27) pB5'3'Puro-PL. Positive clones were processed as above, (EXAMPLE 11). Upon SCNT, 7 cloned V2ZFN-Puro fetuses were obtained from 1 sow. PCR analyses with the (SEQ ID NO:35) *RTtest5'UTRfw* (*5'-*TCTGACTGACCGCGTTACTCC-3') and (SEQ ID NO:38) *Puro2-rv* (5'-CTTGCGGGTCATGCACCAGG-3') primer set revealed that all piglets were Puro-positive and EGFP negative (for EGFP see PCR conditions described in EXAMPLE 11) (FIGURE 7 B). To detect the *pac* genetic marker, the following primer set was employed:
Primers: (SEQ ID NO:35) *RTtest5'UTR-fw* (5'-TCTGACTGACCGCGTTACTCC-3') and (SEQ ID NO:38) *Puro2-rv* (5'-CTTGCGGGTCATGCACCAGG-3') following this PCR protocol:
   94°C 2 minutes, 10 cycles of 94°C 30 seconds, 65°C (-1°C/cycle) 30 seconds, 72°C 2 minutes and 30 seconds, 30 cycles of 94°C 30 seconds, 55°C 30 seconds, 72°C 2 minutes and 30 seconds, followed by 72°C 7 minutes.
   Amplicon = 1627 bp for positive samples and 2193 bp for positive control.

### EXAMPLE 13: Generation of cloned V2ZFN-DsRedT4 fetuses.

Following the procedure described in the EXAMPLE 11, ZFN-endonucleases targeting the EGFP sequence were co-transfected into Verro2GFP primary fibroblasts together with the promoterless DsRedT4 targeting vector, (SEQ ID NO:29) pB5'3'RedT4-PL. Seven cloned V2ZFN-DsRedT4 fetuses were obtained from one of the two pregnant sows. PCR analyses with (SEQ ID NO:35) *RTtest5'UTRfw* (5'-TCTGACTGACCGCGTTACTCC-3') and (SEQ ID NO:39) *HR5Red-rv* (*5'-*TTCTTGTAGTCGGGGATGTCGG-3') primer set revealed that all fetuses were DsRedT4 positive and EGFP negative (for EGFP detection see PCR conditions described in EXAMPLE 11) (FIGURE 7 C).-To detect the *DsRedT4* genetic marker, the (SEQ ID NO:35) *RTtest5'UTR-fw* (*5'-*TCTGACTGACCGCGTTACTCC-3') and (SEQ ID NO:39) *HR5Red-rv* (5'-TTCTTGTAGTCGGGGATGTCGG-3') primers were employed to generate the 1290 bp amplicon of the positive samples and a 1856 bp PCR products of the positive control.

### PCR protocol:

94°C 2 minutes, 10 cycles of 94°C 30 seconds, 65°C (-1 °C/cycle) 30 seconds, 72°C 1 minute and 45 seconds, 30 cycles of 94°C 30 seconds, 55°C 30 seconds, 72°C 1 minute and 45 seconds, followed by 72°C 7 minutes.

Moreover, bright and ubiquitous red light emission was detected upon UV/blue-light-irradiation in 7 out of 7 fetuses, in the corresponding tails and in the primary derived cells (FIGURE 8).

### EXAMPLE 14: Establishment of V2Cre-Hygro colonies by RMCE.

ZFN-endonuclease homologous recombination was applied to knock-in a floxed cassette into the S.scrofa V2G locus resulting in a loss of the EGFP fluorescent phenotype. These floxed ZFN-nuclease cassettes were then employed to exchange the genetic markers by RMCE (see EXAMPLES 10-13) conferring novel phenotypes to the recipient cells. Upon nucleofection of V2ZFN-Puro fibroblasts with the Hygro cassette (SEQ ID NO:28) pB5'3'Hygro-PL and the Cre-expression vector (Addgene plasmid 13776), hygromycin resistant/puromycin sensitive colonies were isolated and subjected to PCR screening with the (SEQ ID NO:35) *RTtest5'UTR-fw* (5'-TCTGACTGACCGCGTTACTCC-3') and (SEQ ID NO:37) *HR5Hygro-rv* (5'-TTCTTGTAGTCGGGGATGTCGG-3') primer set. Nuclei of RMCE positive colonies were then employed to generate transgenic pigs by SCNT. Next, 9 live and 4 stillborn piglets were generated. Tissue samples of these piglets were isolated and subjected to PCR assay with the (SEQ ID NO:35) *RTtest5'UTR-fw* (5'-TCTGACTGACCGCGTTACTCC-3') and (SEQ ID NO:37) *HR5Hygro-rv* (5'-TTCTTGTAGTCGGGGATGTCGG-3') primers to assess the presence of the hygromycin genetic marker; with the (SEQ ID NO:35) *RTtest5'UTRfw* (5'-TCTGACTGACCGCGTTACTCC-3') and (SEQ ID NO:38) *Puro2-rv* (5'-CTTGCGGGTCATGCACCAGG-3') primers to demonstrate the loss of the pac marker and with (SEQ ID NO:35) *RTtest5'UTR-fw* (5'-TCTGACTGACCGCGTTACTCC-3') and (SEQ ID NO:36) *EGFP-rv (5'-*TGCTTCATGTGGTCGGGG-3') primer set to demonstrate the loss of the EGFP genetic marker (FIGURE 9). PCR conditions for these primer sets are described in EXAMPLE 11 and in EXAMPLE 12 Thus, the S.scrofa V2G locus is a safe harbor site for efficient integration of genetic markers by ZFN-endonuclease homologous recombination and by recombination mediated site-specific cassette exchange (RMCE) assay. Moreover, the S.scrofa V2G site also allows efficient expression of several genetic markers even in the absence of selective pressure, as shown for DsRedT4.

### EXAMPLE 15: Establishment of V2CRISPR-Puro colonies.

To assay different endonuclease-mediated knock-in procedure involving the S.scrofa V2G locus of the Verro2GFP trangenic animal, CRISPR-endonuclease mediated HR was assayed. Firstly, CRISPR-targeting EGFP plasmid (SEQ ID NO:41) PX330-GFPcr was generated by cloning the protospacer sequence [6] (into the Bbsl site of the PX330 expression vector (Addgene plasmid 42230). Therefore, the (SEQ ID NO:41) PX330-GFPcr and the floxed promoterless *pac* targeting vector, (SEQ ID NO:27) pB5'3'Puro-PL were co-transfected into Verro2GFP primary fibroblast by nucleofection as described in EXAMPLE 11. Fourty V2CRISPR-Puro resistant colonies were PCR screened with the (SEQ ID NO:35) *RTtest5'UTR-fw* (5'-TCTGACTGACCGCGTTACTCC-3') and (SEQ ID NO:38) *Puro2-rv (5'-*CTTGCGGGTCATGCACCAGG-3') primers and all colonies amplified the 1.6kb positive band of the *pac* gene integrated into the S.scrofa V2G locus (FIGURE 10). PCR conditions are described in EXAMPLE 12. In summary, CRISPR-mediated HR targets the genetic marker of interest at high efficiency into the S.scrofa V2G locus.

### EXAMPLE 16: CRISPR-endonuclease mediated knock-in of a genetic marker into the S.scrofa V2G- wild type sequence of Minnesota swine cells.

CRISPR- endonuclease-mediated knock in was applied to the S.scrofa V2G safe harbor locus of swine breeds that do not carry the pAP1 vector integrated into the S.scrofa V2G locus. Therefore, bioinformatics analysis of the (SEQ ID NO:26) Minnesota swine S.scrofa V2G sequence was carried out to identify putative RNA-guided CRISPR-endonuclease targets. Several protospacer sequences were cloned into the Bbsl sites of the CRISPR/Cas9-endonuclease expression vector PX330 (Addgene plasmid 42230). These vectors (0.4µg) were transfected into Minnesota primary (5*10^5) fibroblasts by Nucleofector-Amaxa, program V-024. About 72h after transfection, cells were lysed and pooled genomic DNA was PCR amplified with (SEQ ID NO:42) *PCRV2cr-fw* (5'-TGCTCCTAATGCAGTGTTGC-3') and (SEQ ID NO:43) *PCRV2cr-rv* (5'-CATGCTGTAGGTTCTTTGTAGC-3') primers and subjected to the Surveyor assay (Transgenomics). This procedure allowed to choose the protospacer (SEQ ID NO:44) V2cr3 (5'-GGAGGCATTCAGTAAATATCAGG-3') and (SEQ ID NO:48) V2cr1 sequences for targeting the swine wild type S.scrofa V2G locus by CRISPR-mediated genome editing assay. To validate the CRISPR-mediated knock-in procedure, the (SEQ ID NO:45) CRISPR-endonuclease expression vector PX330-V2cr3 containing the selected protospacer (SEQ ID NO:44) V2cr3 was cotransfected as above with 0.2 nmol of a single strand 120bp long DNA oligo (SEQ ID NO:46) V2cr3-RMCE into Minnesota primary (5*10^5) fibroblasts. The (SEQ ID NO:46) V2cr3-RMCE carries the Cre-Iox targeting sites, lox2272 and lox5171 separated by the restriction enzyme Pstl site and flanked at the 5' and 3' ends by two 25 bp-long homology arms of the (SEQ ID NO:26) Minnesota S.scrofa V2G locus nearby the (SEQ ID NO:44) V2cr3 protospacer target sequence. After transfection, cells were single cell plated to isolate well growing colonies (Ø ≥ 5mm) and cultured for 11 days in standard medium. To identify the positive clones, genomic DNA was purified from single colonies, amplified and PstI digested as above.

PCR protocol was as follows: about 200 ng template of genomic DNA, 2x PCR GCI buffer, 0.8µM (SEQ ID NO:42) *PCRV2cr-fw* and (SEQ ID NO:43) *PCRV2cr-rv* primers, 400µM dNTPs, 0.05U/ µl LA-Taq polymerase, PCR grade water. The PCR cycle was 94°C 2 minutes, 40 cycles of 94°C 30 seconds, 60°C 30 seconds, 72°C 30 seconds, followed by 72°C incubation for 7 minutes. Upon PCR-amplification (FIGURE 11 A) the wild type template generates a 492 bp amplicon that is Pstl negative, on the contrary the amplification of the targeted allele is expected to generate DNA fragments of variable size up to 566bp that should be digested by Pstl restriction enzyme. Since the exogenous (SEQ ID NO:46) V2cr3-RMCE sequence carries short homology arms (25bp) for the homology recombination events, integration of the exogenous 120bp (SEQ ID NO:46) V2cr3-RMCE sequence into the host S.scrofa V2G sequence may occur, although partial integration of the 120bp sequence is also expected as well as short rearrangement of the host genome surrounding the CRISPR-target site by DNA repair mechanisms. Fourteen out of 410 (3.5%) screened colonies revealed electrophoretic profiles different (higher or lower) from the wild type allele. Amplicons were PstI digested and 3 of which were sequenced. In summary, the (SEQ ID NO:46) V2cr3-RMCE 120bp oligonucleotide was partially integrated with at least one lox site. Biallelic targeting of the S.scrofa V2G locus was observed in the (SEQ ID NO:47) clone 1B2 (FIGURE 11 B). These data indicated that the CRISPR-mediated targeting of the S.scrofa V2G locus occurred on the wild type sequence of Minnesota genome.

### EXAMPLE 17: CRISPRS-endonuclease mediated knock-in of a genetic marker into the S.scrofa V2G- wild type sequence of Verro2 and the commercial 495 breeds.

The example 16 described the (SEQ ID NO:46) V2cr3-RMCE 120bp oligonucleotide knock-in mediated by the (SEQ ID NO:45) CRISPR-endonuclease expression vector PX330-V2cr3 containing the protospacer (SEQ ID NO:44) V2cr3 into Minnesota primary fibroblasts.

To further validate this procedure on primary fibroblasts isolated from Verro2 and the "495" commercial breeds, other CRISPR targeting sequences were chosen, namely (SEQ ID NO:50) V2cr5, (SEQ ID NO:51) V2cr6, (SEQ ID NO:52) V2cr7 and (SEQ ID NO:53) V2cr8, (Figure 12C) and cloned into the pX330-expression vector, as described in example 16. Upon the Surveyor assay (Transgenomics), all these protospacer sequences guided efficiently the CRISPR-endonuclease on the corresponding targeting sequences of the V2G locus. Next, the pX330-vector expressing the (SEQ ID NO:53) V2cr8 was chosen for the homologous recombination targeting assay mediated by CRISPR-endonuclease. To this aim, the (SEQ ID NO:58) pV2G-TEP vector was constructed by flanking the vector cassette containing the EGFP and Pac (PuroR) transcription units with the (SEQ ID NO:56) S.scrofa V2G left arm and (SEQ ID NO:57) S.scrofa right arm, respectively (Figure 12). Because the left and right homology arms of the (SEQ ID NO:58) pV2G-TEP vector were derived from the (SEQ ID NO:24) V2G locus, homologous recombination events between the vector and the V2G locus may occur at high frequency upon CRISPR-targeting of the V2G locus. Thus, puromycin positive clones were selected upon transfection of primary fibroblasts and the relative genomic DNA was purified for the molecular characterization of the HR targeting of the vector. By PCR with (SEQ ID NO:54) pCAGGS5'RV and (SEQ ID NO:20) 5'SusChr9-03-f primer set the HR targeting event was verified on the left arm of the vector, while the (SEQ ID NO:55) Puro2-fw and (SEQ ID NO:17) 3'-SusChr9-04-r primer set was employed to check the HR targeting into V2G locus on the right side of the vector. Importantly, each primer set is made by one primer targeting a specific sequence on the vector, (SEQ ID NO:54) pCAGGS5'RV and (SEQ ID NO:55) Puro2-fw, and by the other targeting the V2G locus outside of the chosen homology sequences, therefore only HR targeting of the vector into the host V2G locus can generate the expected left side 1093bp and right side 2074bp amplicons on the Verro2 and "495" genomes. Lastly, fluorescent analysis of the HR positive clones showed that all fibroblasts were EGFP positive, indicating that V2G locus is indeed a novel swine safe harbor.

PCR reactions were as described in example 16. The PCR program was: an hot start at 94°C for 2 min followed by 6 cycles of denaturation at 94 C for 30 seconds, a touch down at the annealing temperature of 68°C for 30 second and lowered by 1 °C each cycle, the extension step was at 72°C for 80 seconds, then 35 cycles at 94°C 30 seconds, annealing at 63°C 30 seconds and extension at 72°C for 80 seconds. Lastly, a final step of 72 °C for 5 minutes.

In summary, (1) it is possible to knock-in any vector and/or expression vector of interest into the S.scrofa V2G by endonuclease-mediated Homologous Recombination, and (2) the wild type S.scrofa V2G locus is permissive to site specific genome editing in different swine breeds.

From the above description and the above-noted examples, the advantage obtained by the product described and obtained according to the present invention are apparent.

### REFERENCES:

1. Voigt K, Izsvák Z, Ivics Z: Targeted gene insertion for molecular medicine. J MolMed 2008, 86:1205-1219.
2. Galli C, Perota A, Brunetti D, Lagutina I, Lazzari G, Lucchini F: Genetic engineering including superseding microinjection: new ways to make GM pigs. Xenotransplantation 2010, 17:397-410.
3. Brunetti D, Perota A, Lagutina I, Colleoni S, Duchi R, Calabrese F, Seveso M, Cozzi E, Lazzari G, Lucchini F, Galli C: Transgene expression of green fluorescent protein and germ line transmission in cloned pigs derived from in vitro transfected adult fibroblasts. Cloning Stem Cells 2008, 10:409-419.
4. Potter CJ, Luo L: Splinkerette PCR for Mapping Transposable Elements in Drosophila. PLoS ONE 2010, 5:e10168.
5. Watanabe M, Umeyama K, Matsunari H, Takayanagi S, Haruyama E, Nakano K, Fujiwara T, Ikezawa Y, Nakauchi H, Nagashima H: Knockout of exogenous EGFP gene in porcine somatic cells using zinc-finger nucleases. Biochem Biophys Res Commun 2010, 402:14-18.
6. Mali P, Yang L, Esvelt KM, Aach J, Guell M, DiCarlo JE, Norville JE, Church GM: RNA-Guided Human Genome Engineering via Cas9. Science 2013, 339:823-826.
7. Okabe M, Ikawa M, Kominami K, Nakanishi T, Nishimune Y: "Green mice" as a source of ubiquitous green cells. FEBS letters 1997, 407:313-319.
8. Geurts AM, Moreno C: Zinc-finger nucleases: new strategies to target the rat genome. Clin Sci 2010, 119:303-311.
9. Whyte JJ, Zhao J, Wells KD, Samuel MS, Whitworth KM, Walters EM, Laughlin MH, Prather RS: Gene targeting with zinc finger nucleases to produce cloned eGFP knockout pigs. Mol Reprod Dev 2011, 78:2.
10. Lagutina I, Lazzari G, Galli C: Birth of cloned pigs from zona-free nuclear transfer blastocysts developed in vitro before transfer. Cloning Stem Cells 2006, 8:283-293.

## Claims

1. A Sus Scrofa genome specific integration site consisting of a sequence of at least 95% identity to the sequence defined in SEQ ID NO:49.

2. The Sus Scrofa genome specific integration site according to claim 1 having a sequence identity of at least 97% to the sequence defined in SEQ ID NO:49.

3. The Sus Scrofa genome specific integration site according to anyone of claims 1 or 2, having the sequences defined in SEQ ID NO:24, SEQ ID NO:25 and SEQ ID NO:26.

4. The Sus scrofa genome specific integration site according to anyone of claims 1 - 3, comprising a vector of plasmidic origin having the sequence defined in SEQ ID NO:23, SEQ ID NO:27, SEQ ID NO:28, SEQ ID NO:29 and SEQ ID NO:58 integrated inside said integration site.

5. The Sus scrofa genome specific integration site according to claim 4, further comprising a recombinant gene of interest.

6. The Sus scrofa genome specific integration site according to claim 5, wherein the recombinant gene of interest is a gene encoding a polypeptide, an antisense or silencer nucleic acid molecule, an antibody, a DNA vaccine, a regulatory RNA molecule, a non-coding RNA, miRNA, CIS-targeting sequence or a biosensor.

7. Use of the Sus scrofa genome specific integration site according to anyone of claims 4-6, for stable integration into the host chromosome of a recombinant gene of interest.

8. A site-specific endonuclease target site of the Sus scrofa according to anyone of claims 1 to 3, consisting of the sequence defined in SEQ ID NO:44 , SEQ ID NO:48, SEQ ID NO:50, SEQ ID NO:51, SEQ ID NO:52 or SEQ ID NO:53.

## Patentansprüche

1. Sus Scrofa-Genom-spezifische Integrationsstelle, bestehend aus einer Sequenz mit zumindest 95%-iger Übereinstimmung mit der Sequenz, die in der SEQ ID NO:49 definiert ist.

2. Sus-Scrofa-Genom-spezifische Integrationsstelle gemäß Anspruch 1 mit einer Sequenz-Übereinstimmung von zumindest 97% mit der Sequenz, die in der SEQ ID NO:49 definiert ist.

3. Sus-Scrofa-Genom-spezifische Integrationsstelle gemäß einem der Ansprüche 1 oder 2 mit den Sequenzen, die in SEQ ID NO:24, SEQ ID NO:25 und SEQ ID NO:26 definiert sind.

4. Sus-Scrofa-Genom-spezifische Integrationsstelle gemäß einem der Ansprüche 1 bis 3, aufweisend einen Vektor von plasmidischem Ursprung mit den Sequenzen, die in SEQ ID NO:23, SEQ ID NO:27, SEQ ID NO:28, SEQ ID NO:29 und SEQ ID NO:58 definiert sind.

5. Sus-Scrofa-Genom-spezifische Integrationsstelle gemäß Anspruch 4, weiter aufweisend ein rekombinantes Gen von Interesse.

6. Sus-Scrofa-Genom-spezifische Integrationsstelle gemäß Anspruch 5, wobei das rekombinante Gen von Interesse ein Gen ist, das ein Polypeptid, ein Antisens- oder Dämpfungs-Nukleidsäuremolekül, einen Antikörper, ein DNA-Vakzin, ein regulatorisches RNS-Molekül, eine nicht-kodierende RNS-, MikroRNS-, CIS-gerichtete Sequenz oder einen Biosensor kodiert.

7. Verwendung der Sus-Scrofa-Genom-spezifischen Integrationsstelle gemäß einem der Ansprüche 4 bis 6 zur stabilen Integration im Wirtschromosom eines rekombinanten Gens von Interesse.

8. Stellenspezifischer Endonuklease-Wirkort des sus scrofa gemäß einem der Ansprüche 1 bis 3, bestehend aus den Sequenzen, die in SEQ ID NO:44, SEQ ID NO:48, SEQ ID NO:50, SEQ ID NO:51, SEQ ID NO:52 und SEQ ID NO:53 definiert sind.

## Revendications

1. Site d'intégration spécifique au génome de Sus Scrofa consistant en une séquence ayant une identité d'au moins 95 % avec la séquence définie dans SEQ ID NO : 49.

2. Site d'intégration spécifique au génome de Sus Scrofa selon la revendication 1, ayant une identité de séquence d'au moins 97 % avec la séquence définie dans SEQ ID NO : 49.

3. Site d'intégration spécifique au génome de Sus Scrofa selon l'une quelconque des revendications 1 ou 2, ayant les séquences définies dans SEQ ID NO : 24, SEQ ID NO : 25 et SEQ ID NO : 26.

4. Site d'intégration spécifique au génome de Sus Scrofa selon l'une quelconque des revendications 1 à 3, comprenant un vecteur d'origine plasmidique ayant la séquence définie dans SEQ ID NO : 23, SEQ ID NO : 27, SEQ ID NO : 28, SEQ ID NO : 29 et SEQ ID NO : 58 intégrée à l'intérieur dudit site d'intégration.

5. Site d'intégration spécifique au génome de Sus Scrofa selon la revendication 4, comprenant en outre un gène recombinant d'intérêt.

6. Site d'intégration spécifique au génome de Sus Scrofa selon la revendication 5, dans lequel le gène recombinant d'intérêt est un gène codant un polypeptide, une molécule d'acide nucléique antisens ou silenceur, un anticorps, un vaccin à ADN, une molécule d'ARN régulatrice, un ARN non codant, un microARN, une séquence cible CIS ou un biocapteur.

7. Utilisation du site d'intégration spécifique au génome de Sus Scrofa selon l'une quelconque des revendications 4 à 6, pour une intégration stable dans le chromosome hôte d'un gène recombinant d'intérêt.

8. Site cible d'endonucléase spécifique au site du Sus Scrofa selon l'une quelconque des revendications 1 à 3, consistant en la séquence définie dans SEQ ID NO : 44, SEQ ID NO : 48, SEQ ID NO : 50, SEQ ID NO : 51, SEQ ID NO : 52 ou SEQ ID NO : 53.


### SEQUENCE LISTING

<110> AVANTEA S.R.L.
<120> Sus scrofa V2G: a safe-harbor site for long-term expression and high
   integration rate of transgenes in pig
<130> 12799PTEP
<150> ITMI2014A000484
   <151> 2014-03-21
<160> 58
<170> BiSSAP 1.3
<210> 1
   <211> 10908
   <212> DNA
   <213> Unknown
<220>
   <223> Plasmid vector cloning vector pAP1
<400> 1
   ccaattcgcc ctatagtgag tcgtattacg cgcgctcact ggccgtcgtt ttacaacgtc     60
   gtgactggga aaaccctggc gttacccaac ttaatcgcct tgcagcacat ccccctttcg    120
   ccagctgcat taatgaatcg gccaacgcgc ggggagaggc ggtttgcgta ttgggcgctc    180
   ttccgcttcc tcgctcactg actcgctgcg ctcggtcgtt cggctgcggc gagcggtatc    240
   agctcactca aaggcggtaa tacggttatc cacagaatca ggggataacg caggaaagaa    300
   catgtgagca aaaggccagc aaaaggccag gaaccgtaaa aaggccgcgt tgctggcgtt    360
   tttccatagg ctccgccccc ctgacgagca tcacaaaaat cgacgctcaa gtcagaggtg    420
   gcgaaacccg acaggactat aaagatacca ggcgtttccc cctggaagct ccctcgtgcg    480
   ctctcctgtt ccgaccctgc cgcttaccgg atacctgtcc gcctttctcc cttcgggaag    540
   cgtggcgctt tctcatagct cacgctgtag gtatctcagt tcggtgtagg tcgttcgctc    600
   caagctgggc tgtgtgcacg aaccccccgt tcagcccgac cgctgcgcct tatccggtaa    660
   ctatcgtctt gagtccaacc cggtaagaca cgacttatcg ccactggcag cagccactgg    720
   taacaggatt agcagagcga ggtatgtagg cggtgctaca gagttcttga agtggtggcc    780
   taactacggc tacactagaa ggacagtatt tggtatctgc gctctgctga agccagttac    840
   cttcggaaaa agagttggta gctcttgatc cggcaaacaa accaccgctg gtagcggtgg    900
   tttttttgtt tgcaagcagc agattacgcg cagaaaaaaa ggatctcaag aagatccttt    960
   gatcttttct acggggtctg acgctcagtg gaacgaaaac tcacgttaag ggattttggt   1020
   catgagatta tcaaaaagga tcttcaccta gatcctttta aattaaaaat gaagttttaa   1080
   atcaatctaa agtatatatg agtaaacttg gtctgacagt taccaatgct taatcagtga   1140
   ggcacctatc tcagcgatct gtctatttcg ttcatccata gttgcctgac tccccgtcgt 1200
   gtagataact acgatacggg agggcttacc atctggcccc agtgctgcaa tgataccgcg 1260
   agacccacgc tcaccggctc cagatttatc agcaataaac cagccagccg gaagggccga 1320
   gcgcagaagt ggtcctgcaa ctttatccgc ctccatccag tctattaatt gttgccggga 1380
   agctagagta agtagttcgc cagttaatag tttgcgcaac gttgttgcca ttgctacagg 1440
   catcgtggtg tcacgctcgt cgtttggtat ggcttcattc agctccggtt cccaacgatc 1500
   aaggcgagtt acatgatccc ccatgttgtg caaaaaagcg gttagctcct tcggtcctcc 1560
   gatcgttgtc agaagtaagt tggccgcagt gttatcactc atggttatgg cagcactgca 1620
   taattctctt actgtcatgc catccgtaag atgcttttct gtgactggtg agtactcaac 1680
   caagtcattc tgagaatagt gtatgcggcg accgagttgc tcttgcccgg cgtcaatacg 1740
   ggataatacc gcgccacata gcagaacttt aaaagtgctc atcattggaa aacgttcttc 1800
   ggggcgaaaa ctctcaagga tcttaccgct gttgagatcc agttcgatgt aacccactcg 1860
   tgcacccaac tgatcttcag catcttttac tttcaccagc gtttctgggt gagcaaaaac 1920
   aggaaggcaa aatgccgcaa aaaagggaat aagggcgaca cggaaatgtt gaatactcat 1980
   actcttcctt tttcaatatt attgaagcat ttatcagggt tattgtctca tgagcggata 2040
   catatttgaa tgtatttaga aaaataaaca aataggggtt ccgcgcacat ttccccgaaa 2100
   agtgccacct gacgtctaag aaaccattat tatcatgaca ttaacctata aaaataggcg 2160
   tatcacgagg ccctttcgtc tcgcgcgttt cggtgatgac ggtgaaaacc tctgacacat 2220
   gcagctcccg gagacggtca cagcttgtct gtaagcggat gccgggagca gacaagcccg 2280
   tcagggcgcg tcagcgggtg ttggcgggtg tcggggctgg cttaactatg cggcatcaga 2340
   gcagattgta ctgagagtgc accattcgac gctctccctt atgcgactcc tgcattagga 2400
   agcagcccag tagtaggttg aggccgttga gcaccgccgc cgcaaggaat ggtgcatgca 2460
   aggagatggc gcccaacagt cccccggcca cggggcctgc caccataccc acgccgaaac 2520
   aagcgctcat gagcccgaag tggcgagccc gatcttcccc atcggtgatg tcggcgatat 2580
   aggcgccagc aaccgcacct gtggcgccgg tgatgccggc cacgatgcgt ccggcgtaga 2640
   ggatctggct agcgatgacc ctgctgattg gttcgctgac catttccggg tgcgggacgg 2700
   cgttaccaga aactcagaag gttcgtccaa ccaaaccgac tctgacggca gtttacgaga 2760
   gagatgatag ggtctgcttc agtaagccag atgctacaca attaggcttg tacatattgt 2820
   cgttagaacg cggctacaat taatacataa ccttatgtat catacacata cgatttaggt 2880
   gacactatag aatacaagct tctcgaggtc gacattgatt attgactagt tattaatagt 2940
   aatcaattac ggggtcatta gttcatagcc catatatgga gttccgcgtt acataactta 3000
   cggtaaatgg cccgcctggc tgaccgccca acgacccccg cccattgacg tcaataatga 3060
   cgtatgttcc catagtaacg ccaataggga ctttccattg acgtcaatgg gtggactatt 3120
   tacggtaaac tgcccacttg gcagtacatc aagtgtatca tatgccaagt acgcccccta 3180
   ttgacgtcaa tgacggtaaa tggcccgcct ggcattatgc ccagtacatg accttatggg 3240
   actttcctac ttggcagtac atctacgtat tagtcatcgc tattaccatg ggtcgaggtg 3300
   agccccacgt tctgcttcac tctccccatc tcccccccct ccccaccccc aattttgtat 3360
   ttatttattt tttaattatt ttgtgcagcg atgggggcgg gggggggggg ggcgcgcgcc 3420
   aggcggggcg gggcggggcg aggggcgggg cggggcgagg cggagaggtg cggcggcagc 3480
   caatcagagc ggcgcgctcc gaaagtttcc ttttatggcg aggcggcggc ggcggcggcc 3540
   ctataaaaag cgaagcgcgc ggcgggcggg agtcgctgcg ttgccttcgc cccgtgcccc 3600
   gctccgcgcc gcctcgcgcc gcccgccccg gctctgactg accgcgttac tcccacaggt 3660
   gagcgggcgg gacggccctt ctcctccggg ctgtaattag cgcttggttt aatgacggct 3720
   cgtttctttt ctgtggctgc gtgaaagcct taaagggctc cgggagggcc ctttgtgcgg 3780
   gggggagcgg ctcggggggt gcgtgcgtgt gtgtgtgcgt ggggagcgcc gcgtgcggcc 3840
   cgcgctgccc ggcggctgtg agcgctgcgg gcgcggcgcg gggctttgtg cgctccgcgt 3900
   gtgcgcgagg ggagcgcggc cgggggcggt gccccgcggt gcgggggggc tgcgagggga 3960
   acaaaggctg cgtgcggggt gtgtgcgtgg gggggtgagc agggggtgtg ggcgcggcgg 4020
   tcgggctgta acccccccct gcacccccct ccccgagttg ctgagcacgg cccggcttcg 4080
   ggtgcggggc tccgtgcggg gcgtggcgcg gggctcgccg tgccgggcgg ggggtggcgg 4140
   caggtggggg tgccgggcgg ggcggggccg cctcgggccg gggagggctc gggggagggg 4200
   cgcggcggcc ccggagcgcc ggcggctgtc gaggcgcggc gagccgcagc cattgccttt 4260
   tatggtaatc gtgcgagagg gcgcagggac ttcctttgtc ccaaatctgg cggagccgaa 4320
   atctgggagg cgccgccgca ccccctctag cgggcgcggg cgaagcggtg cggcgccggc 4380
   aggaaggaaa tgggcgggga gggccttcgt gcgtcgccgc gccgccgtcc ccttctccat 4440
   ctccagcctc ggggctgccg cagggggacg gctgccttcg ggggggacgg ggcagggcgg 4500
   ggttcggctt ctggcgtgtg accggcggct ctagagcctc tgctaaccat gttcatgcct 4560
   tcttcttttt cctacagctc ctgggcaacg tgctggttgt tgtgctgtct catcattttg 4620
   gcaaagaatt catggtgagc aagggcgagg agctgttcac cggggtggtg cccatcctgg 4680
   tcgagctgga cggcgacgta aacggccaca agttcagcgt gtccggcgag ggcgagggcg 4740
   atgccaccta cggcaagctg accctgaagt tcatctgcac caccggcaag ctgcccgtgc 4800
   cctggcccac cctcgtgacc accctgacct acggcgtgca gtgcttcagc cgctaccccg 4860
   accacatgaa gcagcacgac ttcttcaagt ccgccatgcc cgaaggctac gtccaggagc 4920
   gcaccatctt cttcaaggac gacggcaact acaagacccg cgccgaggtg aagttcgagg 4980
   gcgacaccct ggtgaaccgc atcgagctga agggcatcga cttcaaggag gacggcaaca 5040
   tcctggggca caagctggag tacaactaca acagccacaa cgtctatatc atggccgaca 5100
   agcagaagaa cggcatcaag gtgaacttca agatccgcca caacatcgag gacggcagcg 5160
   tgcagctcgc cgaccactac cagcagaaca cccccatcgg cgacggcccc gtgctgctgc 5220
   ccgacaacca ctacctgagc acccagtccg ccctgagcaa agaccccaac gagaagcgcg 5280
   atcacatggt cctgctggag ttcgtgaccg ccgccgggat cactctcggc atggacgagc 5340
   tgtacaagta agaattcaat tcactcctca ggtgcaggct gcctatcaga aggtggtggc 5400
   tggtgtggcc aatgccctgg ctcacaaata ccactgagat ctttttccct ctgccaaaaa 5460
   ttatggggac atcatgaagc cccttgagca tctgacttct ggctaataaa ggaaatttat 5520
   tttcattgca atagtgtgtt ggaatttttt gtgtctctca ctcggaagga catatgggag 5580
   ggcaaatcat ttaaaacatc agaatgagta tttggtttag agtttggcaa catatgccca 5640
   tatgctggct gccatgaaca aaggttggct ataaagaggt catcagtata tgaaacagcc 5700
   ccctgctgtc cattccttat tccatagaaa agccttgact tgaggttaga ttttttttat 5760
   attttgtttt gtgttatttt tttctttaac atccctaaaa ttttccttac atgttttact 5820
   agccagattt ttcctcctct cctgactact cccagtcata gctgtccctc ttctcttatg 5880
   gagatccctc gacctgcagc ccgggggatc cgtaatacaa ttgtaccagg ttttggttta 5940
   ttacatgtga ctgacggctt cctgtgcgtg ctcaggaaac ggcagttggg cactgcactg 6000
   cccggtgatg gtgccacggt ggctcctgcc gccttctttg atattcactc tgttgtattt 6060
   catctcttct tgccgatgaa aggatataac agtctgtgag gaaatacttg gtatttcttc 6120
   tgatcagcgt ttttataagt aatgttgaat attggataag gctgtgtgtc ctttgtcttg 6180
   ggagacaaag cccacagcag gtggtggttg gggtggtggc agctcagtga caggagaggt 6240
   ttttttgcct gttttttttg ttgttttttt tttttaagta aggtgttctt ttttcttagt 6300
   aaaatttcta ctggactgta tgttttgaca ggtcagaaac atttcttcaa aagaagaacc 6360
   ttttggaaac tgtacagccc ttttctttca ttcccttttt gctttctgtg ccaatgcctt 6420
   tggttctgat tgcattatgg aaaacgttga tcggaacttg aggtttttat ttatagtgtg 6480
   gcttgaaagc ttggatagct gttgttacat gagatacctt attaagttta ggccagcttg 6540
   atgctttatt ttttttcctt tgaagtagtg agcgttctct ggtttttttc ctttgaaact 6600
   ggcgaggctt agatttttct aatgggattt tttacctgat gatctagttg catacccaaa 6660
   tgcttgtaaa tgttttccta gttaacatgt tgataacttc ggatttacat gttgtatata 6720
   cttgtcatct gtgtttctag taaaaatata tggcatttat agaaatacgt aattcctgat 6780
   ttcctttttt ttttatctct atgctctgtg tgtacaggtc aaacagactt cactcctatt 6840
   tttatttata gaattttata tgcagtctgt cgttggttct tgtgttgtaa ggatacagcc 6900
   ttaaatttcc tagagcgatg ctcagtaagg cgggttgtca catgggttca aatgtaaaac 6960
   gggcacgttt gctgctgcct tcccagatcc aggacactaa actgcttctg caactgaggt 7020
   ataaatcgct tcagatccca ggaagtgtag atccacgtgc atattcttaa agaagaatga 7080
   atactttcta aaatatgttg gcataggaag caagctgcat ggatttattt gggacttaaa 7140
   ttattttggt aacggagtgc ataggtttta aacacagttg cagcatgcta acgagtcaca 7200
   gcatttatgc agaagtgatg cctgttgcag ctgtttacgg cactgccttg cagtgagcat 7260
   tgcagatagg ggtggggtgc tttgtgtcgt gttgggacac gctgccacac agccacctcc 7320
   cgaacatatc tcacctgctg ggtacttttc aaaccatctt agcagtagta gatgagttac 7380
   tatgaaacag agaagttcct cagttggata ttctcatggg atgtcttttt tcccatgttg 7440
   ggcaaagtat gataaagcat ctctatttgt aaattatgca cttgttagtt cctgaatcct 7500
   ttctatagca ccacttattg cagcaggtgt aggctctggt gtggcctgtg tctgtgcttc 7560
   aatcttttaa gcttctttgg aaatacaccg acttgattga agtctcttga agatagtaaa 7620
   cagtacttac ctttgatccc aatgaaatcg agcatttcag ttgtaaaaga attccgccta 7680
   ttcataccat gtaatgtaat tttacacccc cagtgctgac actttggaat atattcaagt 7740
   aatagacttt ggcctcaccc tcttgtgtac tgtattttgt aatagaaaat attttaaact 7800
   gtgcatatga ttattacatt atgaaagaga cattctgctg atcttcaaat gtaagaaaat 7860
   gaggagtgcg tgtgctttta taaatacaag tgattgcaaa ttagtgcagg tgtccttaaa 7920
   aaaaaaaaaa agtaatataa aaaggaccag gtgttttaca agtgaaatac attcctattt 7980
   ggaaaacagt tacattttta tgaagattac cagcgcttgc tgactttcta aacataaggc 8040
   tgtattgtct tcctgtacca ttgcatttcc tcattcccaa tttgcacaag gatgtctggg 8100
   taaactattc aagaaatggc tttgaaatac agcatgggag cttgtctgag ttggaatgca 8160
   gagttgcact gcaaaatgtc aggaaatgga tgtctctcag aatgcccaac tccaaaggat 8220
   ttatatgtgt atatagtaag cagtttcctg attccagcag gccaaagagt ctgctgaatg 8280
   ttgcgttgcc ggagacctgt atttctcaac aaggtaagat ggtatcctag caactgcgga 8340
   ttttaataca ttttcagcag aagtacttag ttaatctcta cctttaggga tcgtttcatc 8400
   atttttagat gttatacttg aaatactgca taacttttag ctttcatggg ttcctttttt 8460
   tcagccttta ggagactgtt aagcaatttg ctgtccaact tttgtgttgg tcttaaactg 8520
   caatagtagt ttaccttgta ttgaagaaat aaagaccatt tttatattaa aaaatacttt 8580
   tgtctgtctt cattttgact tgtctgatat ccttgcagtg ctcattatgt cagttctgtc 8640
   agatattcac acatcaaaac ttaacgtgag ctcagtggag ttacagctgc ggttttgatg 8700
   ctgttattat ttctgaaact agaaatgatg ttgtcttcat ctgctcatca aacacttcat 8760
   gcagcagttt aaggctagtg agaaatgcat acatttattg atactttttt aaagtcaact 8820
   ttttatcaga tttttttttc atttggaaat atattgtttt ctagagcggc cgcgggccca 8880
   tcgataagct tgggcggtcg agggacctaa taacttcgta tagcatacat tatacgaagt 8940
   tatattaagg gttccggatc cccgggcgag ctccaccgcg gtggcggccg gccgctctag 9000
   cctcgatcga gctcgagccc cagctggttc tttccgcctc agaagccata gagcccaccg 9060
   catccccagc atgcctgcta ttgtcttccc aatcctcccc cttgctgtcc tgccccaccc 9120
   caccccccag aatagaatga cacctactca gacaatgcga tgcaatttcc tcattttatt 9180
   aggaaaggac agtgggagtg gcaccttcca gggtcaagga aggcacgggg gaggggcaaa 9240
   caacagatgg ctggcaacta gaaggcacag tcgaggctga tcagcgagct ctagagaatt 9300
   gatcccctca gaagaactcg tcaagaaggc gatagaaggc gatgcgctgc gaatcgggag 9360
   cggcgatacc gtaaagcacg aggaagcggt cagcccattc gccgccaagc tcttcagcaa 9420
   tatcacgggt agccaacgct atgtcctgat agcggtccgc cacacccagc cggccacagt 9480
   cgatgaatcc agaaaagcgg ccattttcca ccatgatatt cggcaagcag gcatcgccat 9540
   gggtcacgac gagatcatcg ccgtcgggca tgcgcgcctt gagcctggcg aacagttcgg 9600
   ctggcgcgag cccctgatgc tcttcgtcca gatcatcctg atcgacaaga ccggcttcca 9660
   tccgagtacg tgctcgctcg atgcgatgtt tcgcttggtg gtcgaatggg caggtagccg 9720
   gatcaagcgt atgcagccgc cgcattgcat cagccatgat ggatactttc tcggcaggag 9780
   caaggtgaga tgacaggaga tcctgccccg gcacttcgcc caatagcagc cagtcccttc 9840
   ccgcttcagt gacaacgtcg agcacagctg cgcaaggaac gcccgtcgtg gccagccacg 9900
   atagccgcgc tgcctcgtcc tgcagttcat tcagggcacc ggacaggtcg gtcttgacaa 9960
   aaagaaccgg gcgcccctgc gctgacagcc ggaacacggc ggcatcagag cagccgattg 10020
   tctgttgtgc ccagtcatag ccgaatagcc tctccaccca agcggccgga gaacctgcgt 10080
   gcaatccatc ttgttcaatg gccgatccca tattggctgc aggtcgaaag gcccggagat 10140
   gaggaagagg agaacagcgc ggcagacgtg cgcttttgaa gcgtgcagaa tgccgggcct 10200
   ccggaggacc ttcgggcgcc cgccccgccc ctgagcccgc ccctgagccc gcccccggac 10260
   ccaccccttc ccagcctctg agcccagaaa gcgaaggagc aaagctgcta ttggccgctg 10320
   ccccaaaggc ctacccgctt tcatttgctc agcggtgctg tccatctgca cgagactagt 10380
   gagacgtgct actttccatt tgtcacgtcc tgcacgacgc gagctgcggg gcggggggga 10440
   acttcctgac taggggagga gtagaaggtg gcgcgaaggg gccaccaaag aacggagccg 10500
   gttggcgcct accggtggat gtggaatgtg tgcgaggcca gaggccactt gtgtagcgcc 10560
   aagtgcccag cggggctgct aaagcgcatg ctccagactg ccttgggaaa agcgcctccc 10620
   ctacccggta gaattcgcca atgacaagac gctgggcggg gtttgctcga cattgggtgg 10680
   aaacattcca ggcctgggtg gagaggcttt ttgcttcctc ttgcaaaacc acactgctcg 10740
   acattgggtg gaaacattcc aggcctgggt ggagaggctt tttgcttcct cttgaaaacc 10800
   acactgctcg acctgcaggt cgagggacct aataacttcg tatagcatac attatacgaa 10860
   gttatattaa gggttccgga tcgataccgt cgaggggggg cccggtac 10908
<210> 2
   <211> 61
   <212> DNA
   <213> Unknown
<220>
   <223> Primer
<400> 2
   cgaagagtaa ccgttgctag gagagaccgt ggctgaatga gactggtgtc gacactagtg 60
   g 61
<210> 3
   <211> 48
   <212> DNA
   <213> Unknown
<220>
   <223> Primer
<400> 3
   aattccacta gtgtcgacac cagtctctaa tttttttttt caaaaaaa 48
<210> 4
   <211> 48
   <212> DNA
   <213> Unknown
<220>
   <223> Primer
<400> 4
   gatcccacta gtgtcgacac cagtctctaa tttttttttt caaaaaaa 48
<210> 5
   <211> 23
   <212> DNA
   <213> Unknown
<220>
   <223> Primer
<400> 5
   aggccaccac ttcaagaact ctg 23
<210> 6
   <211> 24
   <212> DNA
   <213> Unknown
<220>
   <223> Primer
<400> 6
   aactgagata cctacagcgt gagc 24
<210> 7
   <211> 22
   <212> DNA
   <213> Unknown
<220>
   <223> Primer
<400> 7
   cctggcgtta cccaacttaa tc 22
<210> 8
   <211> 28
   <212> DNA
   <213> Unknown
<220>
   <223> Primer
<400> 8
   cgaagagtaa ccgttgctag gagagacc 28
<210> 9
   <211> 25
   <212> DNA
   <213> Unknown
<220>
   <223> Primer
<400> 9
   gtggctgaat gagactggtg tcgac 25
<210> 10
   <211> 19
   <212> DNA
   <213> Unknown
<220>
   <223> Primer
<400> 10
   ttgtaaaacg acggccagt 19
<210> 11
   <211> 17
   <212> DNA
   <213> Unknown
<220>
   <223> Primer
<400> 11
   caggaaacag ctatgac 17
<210> 12
   <211> 135
   <212> DNA
   <213> Sus scrofa
<220>
   <223> S. scrofa bp 1-75, pAP1 vector bp 76-135
<400> 12
   aattcagctg gactgtaaaa cagatataat gtcagccacg gcaaatgagt attgtgagga 60
   gaaaagtaga tgaacggcgt tacccaactt aatcgccttg cagcacatcc ccctttcgcc 120
   agctgcatta atgaa 135
<210> 13
   <211> 294
   <212> DNA
   <213> Sus scrofa
<400> 13
   gatcaataga tggcaggagg cttcattttt cccccagctc tttcttgggg gcacagaaca 60
   caaccatcct ccttctgagg cccttcacac caagttgtgg aaacacaggg aggctttttc 120
   aatgtaacgt gaggtatgca aaagcagagg ccagttgggc atgactccgg aggagttatg 180
   ctaagaggct attagtctct gcacttcctt gaccccaggg gcccttctac agctccgcca 240
   aagagaaaaa gaagggagga ctggcggggt gaggagaggg gagtgggagg ctga 294
<210> 14
   <211> 23
   <212> DNA
   <213> Sus scrofa
<220>
   <223> Primer
<400> 14
   aacgctttgg agtcaggaca cct 23
<210> 15
   <211> 25
   <212> DNA
   <213> Sus scrofa
<220>
   <223> Primer
<400> 15
   ctctgccaag actgcttctc catcc 25
<210> 16
   <211> 27
   <212> DNA
   <213> Sus scrofa
<220>
   <223> Primer
<400> 16
   cttcctctgt gctttcatca tgccttg 27
<210> 17
   <211> 23
   <212> DNA
   <213> Sus scrofa
<220>
   <223> Primer
<400> 17
   gcctggcaca aagccttctt ctc 23
<210> 18
   <211> 28
   <212> DNA
   <213> Sus scrofa
<220>
   <223> Primer
<400> 18
   ctctctgatg cagcaggaat ctctagtc 28
<210> 19
   <211> 27
   <212> DNA
   <213> Sus scrofa
<220>
   <223> Primer
<400> 19
   ctaccgaaga cgaactctgc tccaaac 27
<210> 20
   <211> 26
   <212> DNA
   <213> Sus scrofa
<220>
   <223> Primer
<400> 20
   ctcctttgac aaggtctagt ggatgg 26
<210> 21
   <211> 27
   <212> DNA
   <213> Unknown
<220>
   <223> Primer
<400> 21
   gctttttgct tcctcttgca aaaccac 27
<210> 22
   <211> 1124
   <212> DNA
   <213> Sus scrofa
<220>
   <223> Sus scrofa 1..1024, pAP1 cloning vector 1025..1124
<400> 22
   ctcctttgac aaggtctagt ggatggaaaa gaaggactct gggcctaggc agggtcattt      60
   atcttttggt cactgatttt tttttccttc actcagtgtt ggaggggacg gtccagggcc     120
   agaatcagag gacttggatt ctgtctctgt cctgctgccg tctaggacag agacagaagt     180
   cagttgacct taaacaagtc agttgacctc tgtgagcact gttgacctta aacaagtcag     240
   ttgacctctg tgagcactgt tccctcccta attcagcatt cagtatctac tgaagacgaa     300
   ctctgctcca aactctgagt taggctctgg gggagtagaa acatacaagg gatatggctc     360
   ttgtctgcac atggcccttc gtccgcagga ggaaacaggg ctggtttcac gtggtcagtc     420
   agggaggggc caagcaaacc tctgggaggc tcctgggccc cagtccttga gatgacttca     480
   gttcccggtg ggtggtgagc cctgagggtg tggccggggt gaggcagtcc tccactcaga     540
   gggactgcag aaattagccc agctgtgtga tgttggacga gtcactgagc ttctctgagc     600
   ctctttcctc ctcccacttc cccgaatagt caagccctgt cttgtggtgc ctttgtgggc     660
   aagccgactc cttcatctgg cactcactct ggtgggtttc agtggttgtc tgcctctttc     720
   cctgggctct cagcagcgtg aaggctgggc tatgtcaatc ttattcacag gggtctcccc     780
   tgctcctaat gcggtgttgc atttagtagg tgctcagtta atgtctgatg aaggattgag     840
   tatatagatg agctgtggag cagggaaggg ctttgctgga ctccatgtac tacagctgct     900
   gctctctgat gcagcaggaa tctctggtcc catcacggcg cctgcatcct cttcacggta     960
   atacctgata tttactgaat gcctccaaca ttccaggcag agttctgtca atgttctgat    1020
   acatagtgtg gttttcaaga ggaagcaaaa agcctctcca cccaggcctg gaatgtttcc    1080
   acccaatgtc gagcagtgtg gttttgcaag aggaagcaaa aagc                     1124
<210> 23
   <211> 13045
   <212> DNA
   <213> Sus scrofa
<220>
   <223> Sus scrofa 1..1024; pAP1 cloning vector 1025..11752; Sus scrofa 11753..13045
<400> 23
   ctcctttgac aaggtctagt ggatggaaaa gaaggactct gggcctaggc agggtcattt 60
   atcttttggt cactgatttt tttttccttc actcagtgtt ggaggggacg gtccagggcc 120
   agaatcagag gacttggatt ctgtctctgt cctgctgccg tctaggacag agacagaagt 180
   cagttgacct taaacaagtc agttgacctc tgtgagcact gttgacctta aacaagtcag 240
   ttgacctctg tgagcactgt tccctcccta attcagcatt cagtatctac tgaagacgaa 300
   ctctgctcca aactctgagt taggctctgg gggagtagaa acatacaagg gatatggctc 360
   ttgtctgcac atggcccttc gtccgcagga ggaaacaggg ctggtttcac gtggtcagtc 420
   agggaggggc caagcaaacc tctgggaggc tcctgggccc cagtccttga gatgacttca 480
   gttcccggtg ggtggtgagc cctgagggtg tggccggggt gaggcagtcc tccactcaga 540
   gggactgcag aaattagccc agctgtgtga tgttggacga gtcactgagc ttctctgagc 600
   ctctttcctc ctcccacttc cccgaatagt caagccctgt cttgtggtgc ctttgtgggc 660
   aagccgactc cttcatctgg cactcactct ggtgggtttc agtggttgtc tgcctctttc 720
   cctgggctct cagcagcgtg aaggctgggc tatgtcaatc ttattcacag gggtctcccc 780
   tgctcctaat gcggtgttgc atttagtagg tgctcagtta atgtctgatg aaggattgag 840
   tatatagatg agctgtggag cagggaaggg ctttgctgga ctccatgtac tacagctgct 900
   gctctctgat gcagcaggaa tctctggtcc catcacggcg cctgcatcct cttcacggta 960
   atacctgata tttactgaat gcctccaaca ttccaggcag agttctgtca atgttctgat 1020
   acatagtgtg gttttcaaga ggaagcaaaa agcctctcca cccaggcctg gaatgtttcc 1080
   acccaatgtc gagcagtgtg gttttgcaag aggaagcaaa aagcctctcc acccaggcct 1140
   ggaatgtttc cacccaatgt cgagcaaacc ccgcccagcg tcttgtcatt ggcgaattct 1200
   accgggtagg ggaggcgctt ttcccaaggc agtctggagc atgcgcttta gcagccccgc 1260
   tgggcacttg gcgctacaca agtggcctct ggcctcgcac acattccaca tccaccggta 1320
   ggcgccaacc ggctccgttc tttggtggcc ccttcgcgcc accttctact cctcccctag 1380
   tcaggaagtt cccccccgcc ccgcagctcg cgtcgtgcag gacgtgacaa atggaaagta 1440
   gcacgtctca ctagtctcgt gcagatggac agcaccgctg agcaaatgaa agcgggtagg 1500
   cctttggggc agcggccaat agcagctttg ctccttcgct ttctgggctc agaggctggg 1560
   aaggggtggg tccgggggcg ggctcagggg cgggctcagg ggcggggcgg gcgcccgaag 1620
   gtcctccgga ggcccggcat tctgcacgct tcaaaagcgc acgtctgccg cgctgttctc 1680
   ctcttcctca tctccgggcc tttcgacctg cagccaatat gggatcggcc attgaacaag 1740
   atggattgca cgcaggttct ccggccgctt gggtggagag gctattcggc tatgactggg 1800
   cacaacagac aatcggctgc tctgatgccg ccgtgttccg gctgtcagcg caggggcgcc 1860
   cggttctttt tgtcaagacc gacctgtccg gtgccctgaa tgaactgcag gacgaggcag 1920
   cgcggctatc gtggctggcc acgacgggcg ttccttgcgc agctgtgctc gacgttgtca 1980
   ctgaagcggg aagggactgg ctgctattgg gcgaagtgcc ggggcaggat ctcctgtcat 2040
   ctcaccttgc tcctgccgag aaagtatcca tcatggctga tgcaatgcgg cggctgcata 2100
   cgcttgatcc ggctacctgc ccattcgacc accaagcgaa acatcgcatc gagcgagcac 2160
   gtactcggat ggaagccggt cttgtcgatc aggatgatct ggacgaagag catcaggggc 2220
   tcgcgccagc cgaactgttc gccaggctca aggcgcgcat gcccgacggc gatgatctcg 2280
   tcgtgaccca tggcgatgcc tgcttgccga atatcatggt ggaaaatggc cgcttttctg 2340
   gattcatcga ctgtggccgg ctgggtgtgg cggaccgcta tcaggacata gcgttggcta 2400
   cccgtgatat tgctgaagag cttggcggcg aatgggctga ccgcttcctc gtgctttacg 2460
   gtatcgccgc tcccgattcg cagcgcatcg ccttctatcg ccttcttgac gagttcttct 2520
   gaggggatca attctctaga gctcgctgat cagcctcgac tgtgccttct agttgccagc 2580
   catctgttgt ttgcccctcc cccgtgcctt ccttgaccct ggaaggtgcc actcccactg 2640
   tcctttccta ataaaatgag gaaattgcat cgcattgtct gagtaggtgt cattctattc 2700
   tggggggtgg ggtggggcag gacagcaagg gggaggattg ggaagacaat agcaggcatg 2760
   ctggggatgc ggtgggctct atggcttctg aggcggaaag aaccagctgg ggctcgagct 2820
   cgatcgaggc tagagcggcc ggccgccacc gcggtggagc tcgcccgggg atccggaacc 2880
   cttaatataa cttcgtataa tgtatgctat acgaagttat taggtccctc gaccgcccaa 2940
   gcttatcgat gggcccgcgg ccgctctaga aaacaatata tttccaaatg aaaaaaaaat 3000
   ctgataaaaa gttgacttta aaaaagtatc aataaatgta tgcatttctc actagcctta 3060
   aactgctgca tgaagtgttt gatgagcaga tgaagacaac atcatttcta gtttcagaaa 3120
   taataacagc atcaaaaccg cagctgtaac tccactgagc tcacgttaag ttttgatgtg 3180
   tgaatatctg acagaactga cataatgagc actgcaagga tatcagacaa gtcaaaatga 3240
   agacagacaa aagtattttt taatataaaa atggtcttta tttcttcaat acaaggtaaa 3300
   ctactattgc agtttaagac caacacaaaa gttggacagc aaattgctta acagtctcct 3360
   aaaggctgaa aaaaaggaac ccatgaaagc taaaagttat gcagtatttc aagtataaca 3420
   tctaaaaatg atgaaacgat ccctaaaggt agagattaac taagtacttc tgctgaaaat 3480
   gtattaaaat ccgcagttgc taggatacca tcttaccttg ttgagaaata caggtctccg 3540
   gcaacgcaac attcagcaga ctctttggcc tgctggaatc aggaaactgc ttactatata 3600
   cacatataaa tcctttggag ttgggcattc tgagagacat ccatttcctg acattttgca 3660
   gtgcaactct gcattccaac tcagacaagc tcccatgctg tatttcaaag ccatttcttg 3720
   aatagtttac ccagacatcc ttgtgcaaat tgggaatgag gaaatgcaat ggtacaggaa 3780
   gacaatacag ccttatgttt agaaagtcag caagcgctgg taatcttcat aaaaatgtaa 3840
   ctgttttcca aataggaatg tatttcactt gtaaaacacc tggtcctttt tatattactt 3900
   tttttttttt ttaaggacac ctgcactaat ttgcaatcac ttgtatttat aaaagcacac 3960
   gcactcctca ttttcttaca tttgaagatc agcagaatgt ctctttcata atgtaataat 4020
   catatgcaca gtttaaaata ttttctatta caaaatacag tacacaagag ggtgaggcca 4080
   aagtctatta cttgaatata ttccaaagtg tcagcactgg gggtgtaaaa ttacattaca 4140
   tggtatgaat aggcggaatt cttttacaac tgaaatgctc gatttcattg ggatcaaagg 4200
   taagtactgt ttactatctt caagagactt caatcaagtc ggtgtatttc caaagaagct 4260
   taaaagattg aagcacagac acaggccaca ccagagccta cacctgctgc aataagtggt 4320
   gctatagaaa ggattcagga actaacaagt gcataattta caaatagaga tgctttatca 4380
   tactttgccc aacatgggaa aaaagacatc ccatgagaat atccaactga ggaacttctc 4440
   tgtttcatag taactcatct actactgcta agatggtttg aaaagtaccc agcaggtgag 4500
   atatgttcgg gaggtggctg tgtggcagcg tgtcccaaca cgacacaaag caccccaccc 4560
   ctatctgcaa tgctcactgc aaggcagtgc cgtaaacagc tgcaacaggc atcacttctg 4620
   cataaatgct gtgactcgtt agcatgctgc aactgtgttt aaaacctatg cactccgtta 4680
   ccaaaataat ttaagtccca aataaatcca tgcagcttgc ttcctatgcc aacatatttt 4740
   agaaagtatt cattcttctt taagaatatg cacgtggatc tacacttcct gggatctgaa 4800
   gcgatttata cctcagttgc agaagcagtt tagtgtcctg gatctgggaa ggcagcagca 4860
   aacgtgcccg ttttacattt gaacccatgt gacaacccgc cttactgagc atcgctctag 4920
   gaaatttaag gctgtatcct tacaacacaa gaaccaacga cagactgcat ataaaattct 4980
   ataaataaaa ataggagtga agtctgtttg acctgtacac acagagcata gagataaaaa 5040
   aaaaaggaaa tcaggaatta cgtatttcta taaatgccat atatttttac tagaaacaca 5100
   gatgacaagt atatacaaca tgtaaatccg aagttatcaa catgttaact aggaaaacat 5160
   ttacaagcat ttgggtatgc aactagatca tcaggtaaaa aatcccatta gaaaaatcta 5220
   agcctcgcca gtttcaaagg aaaaaaacca gagaacgctc actacttcaa aggaaaaaaa 5280
   ataaagcatc aagctggcct aaacttaata aggtatctca tgtaacaaca gctatccaag 5340
   ctttcaagcc acactataaa taaaaacctc aagttccgat caacgttttc cataatgcaa 5400
   tcagaaccaa aggcattggc acagaaagca aaaagggaat gaaagaaaag ggctgtacag 5460
   tttccaaaag gttcttcttt tgaagaaatg tttctgacct gtcaaaacat acagtccagt 5520
   agaaatttta ctaagaaaaa agaacacctt acttaaaaaa aaaaaacaac aaaaaaaaca 5580
   ggcaaaaaaa cctctcctgt cactgagctg ccaccacccc aaccaccacc tgctgtgggc 5640
   tttgtctccc aagacaaagg acacacagcc ttatccaata ttcaacatta cttataaaaa 5700
   cgctgatcag aagaaatacc aagtatttcc tcacagactg ttatatcctt tcatcggcaa 5760
   gaagagatga aatacaacag agtgaatatc aaagaaggcg gcaggagcca ccgtggcacc 5820
   atcaccgggc agtgcagtgc ccaactgccg tttcctgagc acgcacagga agccgtcagt 5880
   cacatgtaat aaaccaaaac ctggtacaat tgtattacgg atcccccggg ctgcaggtcg 5940
   agggatctcc ataagagaag agggacagct atgactggga gtagtcagga gaggaggaaa 6000
   aatctggcta gtaaaacatg taaggaaaat tttagggatg ttaaagaaaa aaataacaca 6060
   aaacaaaata taaaaaaaat ctaacctcaa gtcaaggctt ttctatggaa taaggaatgg 6120
   acagcagggg gctgtttcat atactgatga cctctttata gccaaccttt gttcatggca 6180
   gccagcatat gggcatatgt tgccaaactc taaaccaaat actcattctg atgttttaaa 6240
   tgatttgccc tcccatatgt ccttccgagt gagagacaca aaaaattcca acacactatt 6300
   gcaatgaaaa taaatttcct ttattagcca gaagtcagat gctcaagggg cttcatgatg 6360
   tccccataat ttttggcaga gggaaaaaga tctcagtggt atttgtgagc cagggcattg 6420
   gccacaccag ccaccacctt ctgataggca gcctgcacct gaggagtgaa ttgaattctt 6480
   acttgtacag ctcgtccatg ccgagagtga tcccggcggc ggtcacgaac tccagcagga 6540
   ccatgtgatc gcgcttctcg ttggggtctt tgctcagggc ggactgggtg ctcaggtagt 6600
   ggttgtcggg cagcagcacg gggccgtcgc cgatgggggt gttctgctgg tagtggtcgg 6660
   cgagctgcac gctgccgtcc tcgatgttgt ggcggatctt gaagttcacc ttgatgccgt 6720
   tcttctgctt gtcggccatg atatagacgt tgtggctgtt gtagttgtac tccagcttgt 6780
   gccccaggat gttgccgtcc tccttgaagt cgatgccctt cagctcgatg cggttcacca 6840
   gggtgtcgcc ctcgaacttc acctcggcgc gggtcttgta gttgccgtcg tccttgaaga 6900
   agatggtgcg ctcctggacg tagccttcgg gcatggcgga cttgaagaag tcgtgctgct 6960
   tcatgtggtc ggggtagcgg ctgaagcact gcacgccgta ggtcagggtg gtcacgaggg 7020
   tgggccaggg cacgggcagc ttgccggtgg tgcagatgaa cttcagggtc agcttgccgt 7080
   aggtggcatc gccctcgccc tcgccggaca cgctgaactt gtggccgttt acgtcgccgt 7140
   ccagctcgac caggatgggc accaccccgg tgaacagctc ctcgcccttg ctcaccatga 7200
   attctttgcc aaaatgatga gacagcacaa caaccagcac gttgcccagg agctgtagga 7260
   aaaagaagaa ggcatgaaca tggttagcag aggctctaga gccgccggtc acacgccaga 7320
   agccgaaccc cgccctgccc cgtccccccc gaaggcagcc gtccccctgc ggcagccccg 7380
   aggctggaga tggagaaggg gacggcggcg cggcgacgca cgaaggccct ccccgcccat 7440
   ttccttcctg ccggcgccgc accgcttcgc ccgcgcccgc tagagggggt gcggcggcgc 7500
   ctcccagatt tcggctccgc cagatttggg acaaaggaag tccctgcgcc ctctcgcacg 7560
   attaccataa aaggcaatgg ctgcggctcg ccgcgcctcg acagccgccg gcgctccggg 7620
   gccgccgcgc ccctcccccg agccctcccc ggcccgaggc ggccccgccc cgcccggcac 7680
   ccccacctgc cgccaccccc cgcccggcac ggcgagcccc gcgccacgcc ccgcacggag 7740
   ccccgcaccc gaagccgggc cgtgctcagc aactcgggga ggggggtgca ggggggggtt 7800
   acagcccgac cgccgcgccc acaccccctg ctcacccccc cacgcacaca ccccgcacgc 7860
   agcctttgtt cccctcgcag cccccccgca ccgcggggca ccgcccccgg ccgcgctccc 7920
   ctcgcgcaca cgcggagcgc acaaagcccc gcgccgcgcc cgcagcgctc acagccgccg 7980
   ggcagcgcgg gccgcacgcg gcgctcccca cgcacacaca cacgcacgca ccccccgagc 8040
   cgctcccccc cgcacaaagg gccctcccgg agccctttaa ggctttcacg cagccacaga 8100
   aaagaaacga gccgtcatta aaccaagcgc taattacagc ccggaggaga agggccgtcc 8160
   cgcccgctca cctgtgggag taacgcggtc agtcagagcc ggggcgggcg gcgcgaggcg 8220
   gcgcggagcg gggcacgggg cgaaggcaac gcagcgactc ccgcccgccg cgcgcttcgc 8280
   tttttatagg gccgccgccg ccgccgcctc gccataaaag gaaactttcg gagcgcgccg 8340
   ctctgattgg ctgccgccgc acctctccgc ctcgccccgc cccgcccctc gccccgcccc 8400
   gccccgcctg gcgcgcgccc cccccccccc cgcccccatc gctgcacaaa ataattaaaa 8460
   aataaataaa tacaaaattg ggggtgggga ggggggggag atggggagag tgaagcagaa 8520
   cgtggggctc acctcgaccc atggtaatag cgatgactaa tacgtagatg tactgccaag 8580
   taggaaagtc ccataaggtc atgtactggg cataatgcca ggcgggccat ttaccgtcat 8640
   tgacgtcaat agggggcgta cttggcatat gatacacttg atgtactgcc aagtgggcag 8700
   tttaccgtaa atagtccacc cattgacgtc aatggaaagt ccctattggc gttactatgg 8760
   gaacatacgt cattattgac gtcaatgggc gggggtcgtt gggcggtcag ccaggcgggc 8820
   catttaccgt aagttatgta acgcggaact ccatatatgg gctatgaact aatgaccccg 8880
   taattgatta ctattaataa ctagtcaata atcaatgtcg acctcgagaa gcttgtattc 8940
   tatagtgtca cctaaatcgt atgtgtatga tacataaggt tatgtattaa ttgtagccgc 9000
   gttctaacga caatatgtac aagcctaatt gtgtagcatc tggcttactg aagcagaccc 9060
   tatcatctct ctcgtaaact gccgtcagag tcggtttggt tggacgaacc ttctgagttt 9120
   ctggtaacgc cgtcccgcac ccggaaatgg tcagcgaacc aatcagcagg gtcatcgcta 9180
   gccagatcct ctacgccgga cgcatcgtgg ccggcatcac cggcgccaca ggtgcggttg 9240
   ctggcgccta tatcgccgac atcaccgatg gggaagatcg ggctcgccac ttcgggctca 9300
   tgagcgcttg tttcggcgtg ggtatggtgg caggccccgt ggccggggga ctgttgggcg 9360
   ccatctcctt gcatgcacca ttccttgcgg cggcggtgct caacggcctc aacctactac 9420
   tgggctgctt cctaatgcag gagtcgcata agggagagcg tcgaatggtg cactctcagt 9480
   acaatctgct ctgatgccgc atagttaagc cagccccgac acccgccaac acccgctgac 9540
   gcgccctgac gggcttgtct gctcccggca tccgcttaca gacaagctgt gaccgtctcc 9600
   gggagctgca tgtgtcagag gttttcaccg tcatcaccga aacgcgcgag acgaaagggc 9660
   ctcgtgatac gcctattttt ataggttaat gtcatgataa taatggtttc ttagacgtca 9720
   ggtggcactt ttcggggaaa tgtgcgcgga acccctattt gtttattttt ctaaatacat 9780
   tcaaatatgt atccgctcat gagacaataa ccctgataaa tgcttcaata atattgaaaa 9840
   aggaagagta tgagtattca acatttccgt gtcgccctta ttcccttttt tgcggcattt 9900
   tgccttcctg tttttgctca cccagaaacg ctggtgaaag taaaagatgc tgaagatcag 9960
   ttgggtgcac gagtgggtta catcgaactg gatctcaaca gcggtaagat ccttgagagt 10020
   tttcgccccg aagaacgttt tccaatgatg agcactttta aagttctgct atgtggcgcg 10080
   gtattatccc gtattgacgc cgggcaagag caactcggtc gccgcataca ctattctcag 10140
   aatgacttgg ttgagtactc accagtcaca gaaaagcatc ttacggatgg catgacagta 10200
   agagaattat gcagtgctgc cataaccatg agtgataaca ctgcggccaa cttacttctg 10260
   acaacgatcg gaggaccgaa ggagctaacc gcttttttgc acaacatggg ggatcatgta 10320
   actcgccttg atcgttggga accggagctg aatgaagcca taccaaacga cgagcgtgac 10380
   accacgatgc ctgtagcaat ggcaacaacg ttgcgcaaac tattaactgg cgaactactt 10440
   actctagctt cccggcaaca attaatagac tggatggagg cggataaagt tgcaggacca 10500
   cttctgcgct cggcccttcc ggctggctgg tttattgctg ataaatctgg agccggtgag 10560
   cgtgggtctc gcggtatcat tgcagcactg gggccagatg gtaagccctc ccgtatcgta 10620
   gttatctaca cgacggggag tcaggcaact atggatgaac gaaatagaca gatcgctgag 10680
   ataggtgcct cactgattaa gcattggtaa ctgtcagacc aagtttactc atatatactt 10740
   tagattgatt taaaacttca tttttaattt aaaaggatct aggtgaagat cctttttgat 10800
   aatctcatga ccaaaatccc ttaacgtgag ttttcgttcc actgagcgtc agaccccgta 10860
   gaaaagatca aaggatcttc ttgagatcct ttttttctgc gcgtaatctg ctgcttgcaa 10920
   acaaaaaaac caccgctacc agcggtggtt tgtttgccgg atcaagagct accaactctt 10980
   tttccgaagg taactggctt cagcagagcg cagataccaa atactgtcct tctagtgtag 11040
   ccgtagttag gccaccactt caagaactct gtagcaccgc ctacatacct cgctctgcta 11100
   atcctgttac cagtggctgc tgccagtggc gataagtcgt gtcttaccgg gttggactca 11160
   agacgatagt taccggataa ggcgcagcgg tcgggctgaa cggggggttc gtgcacacag 11220
   cccagcttgg agcgaacgac ctacaccgaa ctgagatacc tacagcgtga gctatgagaa 11280
   agcgccacgc ttcccgaagg gagaaaggcg gacaggtatc cggtaagcgg cagggtcgga 11340
   acaggagagc gcacgaggga gcttccaggg ggaaacgcct ggtatcttta tagtcctgtc 11400
   gggtttcgcc acctctgact tgagcgtcga tttttgtgat gctcgtcagg ggggcggagc 11460
   ctatggaaaa acgccagcaa cgcggccttt ttacggttcc tggccttttg ctggcctttt 11520
   gctcacatgt tctttcctgc gttatcccct gattctgtgg ataaccgtat taccgccttt 11580
   gagtgagctg ataccgctcg ccgcagccga acgaccgagc gcagcgagtc agtgagcgag 11640
   gaagcggaag agcgcccaat acgcaaaccg cctctccccg cgcgttggcc gattcattaa 11700
   tgcagctggc gaaaggggga tgtgctgcaa ggcgattaag ttgggtaacg ccgttcatct 11760
   acttttctcc tcacaatact catttgccgt ggctgacatt atatctgttt tacagtccag 11820
   ctgaatttca gagcaattaa ataaatttgt gcaaagtcat acagtaaagt gacagaacaa 11880
   gattccagtt caggtgtcct gactccaaag cgttttgctt ccctctgtgc aagctgcatc 11940
   cagggctctc agagctgcct ggacccagag agtaccaatg tagtggctac aaagaaccta 12000
   cagcatggca tgcaaagacc tgtagccaga gggcgctcaa gcgcgctccc ttggctgccc 12060
   tggagtcagt ggggatggag aagcagtctt ggcagagtct ggggccagga tactaatgag 12120
   ctggtacggg tgggtgctca cttttccccc tggcgtcagt cagcctcctt atggggacga 12180
   ctgtcatccc ctctctcttt caaccacatc gacaagcccc ttctctctcc ttgacccctg 12240
   aggagggatt gtgcccggca gcatccccgc ttcctgtccg tgcatcccat agggagtgga 12300
   agccagagga tgaaaagcgc agggtttttt cccttctctt ccaaggaatc caccagatgc 12360
   cacactggct ggggaactgg cagtgcctct tctactctac tcagcctgca gggtggccga 12420
   gctttgtctc ccatgttcat gattccacaa ctttctgggg cacctctctt ttcacatcct 12480
   cttccacagc ctcctgtggt ctcagcctcc cactcccctc tcctcacccc gccagtcctc 12540
   ccttcttttt ctctttggcg gagctgtaga agggcccctg gggtcaagga agtgcagaga 12600
   ctaatagcct cttagcataa ctcctccgga gtcatgccca actggcctct gcttttgcat 12660
   acctcacgtt acattgaaaa agcctccctg tgtttccaca acttggtgtg aagggcctca 12720
   gaaggaggat ggttgtgttc tgtgccccca agaaagagct gggggaaaaa tgaagcctcc 12780
   tgccatctat tgatcaaccc ccttctctgt acatacactc tagaaagatg ataaaggcac 12840
   gaccctgtcc ttatgccacg ctccgctcct aagacactag ggacctgatc caggagacag 12900
   ttgtaaaccc cagttcccat ggaagtcagc ctcgagagaa cctgaatttc atatgcaagg 12960
   catgatgaga gcacagagga aggagagaat aattctgaga gcaaatttgg agaggcttta 13020
   aggagaagaa ggctttgtgc caggc 13045
<210> 24
   <211> 2319
   <212> DNA
   <213> Sus scrofa
<400> 24
   ctcctttgac aaggtctagt ggatggaaaa gaaggactct gggcctaggc agggtcattt      60
   atcttttggt cactgatttt tttttccttc actcagtgtt ggaggggacg gtccagggcc     120
   agaatcagag gacttggatt ctgtctctgt cctgctgccg tctaggacag agacagaagt     180
   cagttgacct taaacaagtc agttgacctc tgtgagcact gttgacctta aacaagtcag     240
   ttgacctctg tgagcactgt tccctcccta attcagcatt cagtatctac tgaagacgaa     300
   ctctgctcca aactctgagt taggctctgg gggagtagaa acatacaagg gatatggctc     360
   ttgtctgcac atggcccttc gtccgcagga ggaaacaggg ctggtttcac gtggtcagtc     420
   agggaggggc caagcaaacc tctgggaggc tcctgggccc cagtccttga gatgacttca     480
   gttcccggtg ggtggtgagc cctgagggtg tggccggggt gaggcagtcc tccactcaga     540
   gggactgcag aaattagccc agctgtgtga tgttggacga gtcactgagc ttctctgagc     600
   ctctttcctc ctcccacttc cccgaatagt caagccctgt cttgtggtgc ctttgtgggc     660
   aagccgactc cttcatctgg cactcactct ggtgggtttc agtggttgtc tgcctctttc     720
   cctgggctct cagcagcgtg aaggctgggc tatgtcaatc ttattcacag gggtctcccc     780
   tgctcctaat gcggtgttgc atttagtagg tgctcagtta atgtctgatg aaggattgag     840
   tatatagatg agctgtggag cagggaaggg ctttgctgga ctccatgtac tacagctgct     900
   gctctctgat gcagcaggaa tctctggtcc catcacggcg cctgcatcct cttcacggta     960
   atacctgata tttactgaat gcctccaaca ttccaggcag agttctgtca atgttctgat    1020
   acatttgttc atctactttt ctcctcacaa tactcatttg ccgtggctga cattatatct    1080
   gttttacagt ccagctgaat ttcagagcaa ttaaataaat ttgtgcaaag tcatacagta    1140
   aagtgacaga acaagattcc agttcaggtg tcctgactcc aaagcgtttt gcttccctct    1200
   gtgcaagctg catccagggc tctcagagct gcctggaccc agagagtacc aatgtagtgg    1260
   ctacaaagaa cctacagcat ggcatgcaaa gacctgtagc cagagggcgc tcaagcgcgc    1320
   tcccttggct gccctggagt cagtggggat ggagaagcag tcttggcaga gtctggggcc    1380
   aggatactaa tgagctggta cgggtgggtg ctcacttttc cccctggcgt cagtcagcct    1440
   ccttatgggg acgactgtca tcccctctct ctttcaacca catcgacaag ccccttctct    1500
   ctccttgacc cctgaggagg gattgtgccc ggcagcatcc ccgcttcctg tccgtgcatc    1560
   ccatagggag tggaagccag aggatgaaaa gcgcagggtt ttttcccttc tcttccaagg    1620
   aatccaccag atgccacact ggctggggaa ctggcagtgc ctcttctact ctactcagcc    1680
   tgcagggtgg ccgagctttg tctcccatgt tcatgattcc acaactttct ggggcacctc    1740
   tcttttcaca tcctcttcca cagcctcctg tggtctcagc ctcccactcc cctctcctca    1800
   ccccgccagt cctcccttct ttttctcttt ggcggagctg tagaagggcc cctggggtca    1860
   aggaagtgca gagactaata gcctcttagc ataactcctc cggagtcatg cccaactggc    1920
   ctctgctttt gcatacctca cgttacattg aaaaagcctc cctgtgtttc cacaacttgg    1980
   tgtgaagggc ctcagaagga ggatggttgt gttctgtgcc cccaagaaag agctggggga    2040
   aaaatgaagc ctcctgccat ctattgatca acccccttct ctgtacatac actctagaaa    2100
   gatgataaag gcacgaccct gtccttatgc cacgctccgc tcctaagaca ctagggacct    2160
   gatccaggag acagttgtaa accccagttc ccatggaagt cagcctcgag agaacctgaa    2220
   tttcatatgc aaggcatgat gagagcacag aggaaggaga gaataattct gagagcaaat    2280
   ttggagaggc tttaaggaga agaaggcttt gtgccaggc                           2319
<210> 25
   <211> 2319
   <212> DNA
   <213> Sus scrofa
<400> 25
   ctcctttgac aaggtctagt ggatggaaaa gaaggactct gggcctaggc agggtcattt      60
   atcttttggt cactgatttt tttttccttc actcagtgtt ggaggggaag gtccagggcc     120
   agaatcagag gacttggatt ctgtctctgt cctgctgccg tctaggacag agacagaagt     180
   cagttgacct taaacaagtc agttgacctc tgtgagcact gttgacctta aacaagtcag     240
   ttgacctctg tgagcactgt tccctcccta attcagcatt cagtatctac tgaagacgaa     300
   ctctgctcca aactctgagt taggctctgg gggagtagaa acatacaagg gatatggctc     360
   ttgtctgcac atggcccttc gtccgcagga ggaaacaggg ctggtttcac gtggtcagtc     420
   agggaggggc caagcaaacc tctgggaggc tcctgggccc cagtccttga gatgacttca     480
   gttcccggtg ggtggtgagc cctgagggtg tggccggggt gaggcagtcc tccactcaga     540
   ggggctgcag aaattagccc agctgtgtga tcttggacga gtcactgagc ttctctgagc     600
   ctctttcctc ctcccacttc cccgaatagt caagccctgt cttgtggtgc ctttgtgggc     660
   aagccgactc cttcatctgg cactcactct ggtgggtttc agtggttgtc tgcctctttc     720
   cctgggctct cagcagtgtg aaggctgggc tatgtcaatc ttattcacag gggtctcccc     780
   tgctcctaat gcggtgttgc atttagtagg tgctcagtta atgtctgatg aaggattgag     840
   tatatagatg agctgtggag cagggaaggg ctttgctgga ctccatgtac tacagctgct     900
   gctctctgat gcagcaggaa tctctggtcc catcatggcg cctgcatcct cttcacggta     960
   atacctgata tttactgaat gcctccaaca ttccaggcag agttctgtca atgttctgat    1020
   acatttgttc atctactttt ctcctcacaa tactcatttg ccgtggctga cattatatct    1080
   gttttacagt ccagctgaat ttcagagcaa ttaaataaat ttgtgcaaag tcatacagta    1140
   aagtgacaga acaagattcc agttcaggtg tcctgactcc aaagcgtttt gcttccctct    1200
   gtgcaagctg catccagggc tctcagagct gcctggaccc agagagtacc aatgtagtgg    1260
   ctacaaagaa cctacagcat ggcatgcaaa gacctgtagc cagagggcgc tcaagcgcgc    1320
   tcccttggct gccctggagt cagtggggat ggagaagcag tcttggcaga gtctggggcc    1380
   aggatactaa tgagctggta cgggtgggtg ctcacttttc cccctggcgt cagtcagcct    1440
   ccttatgggg acgactgtca tcccctctct ctttcaacca catcgacaag ccccttctct    1500
   ctccttgacc cctgaggagg gattgtgccc ggcagcatcc ccgcttcctg tccgtgcatc    1560
   ccatagggag tggaagccag aggatgaaaa gcgcagggtt ttttcccttc tcttccaagg    1620
   aatccaccag atgccacact ggctggggaa ccggcagtgc ctcttctacg ctactcagcc    1680
   tgcagggtgg ccgagctttg tctcccatgt tcatgattcc acaactttct ggggcacctc    1740
   tcttttcaca tcctcttcca cagcctcctg tggtctcagc ctcccactcc cctctcctca    1800
   ccccgccagt cctcccttct ttttctcttt ggcggagctg tagaagggcc cctggggtca    1860
   aggaagtgca gagactaata gcctcttagc ataactcctc cggagtcatg cccaactggc    1920
   ctctgctttt gcatacctca cgttacattg aaaaagcctc cctgtgtttc cacaacttgg    1980
   tgtgaagggc ctcagaaaga ggatggttgt gttctgtgcc cccaagaaag agctggggga    2040
   aaaatgaagc ctcctgccat ctattgatca acccccttct ctgtacatac actctagaaa    2100
   gatgataaag gcacgaccct gtccttatgc cacgctccgc tcctaagaca ctagggacct    2160
   gatccaggag acagttgtaa accccagttc ccatggaagt cagcctcgag agaacctgaa    2220
   tttcatatgc aaggcatgat gagagcacag aggaaggaga gaataattct gagagcaaat    2280
   ttggagaggc tttaaggaga agaaggcttt gtgccaggc                          2319
<210> 26
   <211> 2304
   <212> DNA
   <213> Sus scrofa
<400> 26
   ctcctttgac aaggtctagt ggatggaaaa gagggactct gggcctaggc agggtcattt      60
   atcttttggt cactgatttt tttttccttc actcagtgtt ggaggggaag gtccagaatc     120
   agaggacttg gattctgtct ctgtcctgct gccgtctagg acagagacag aagtcagttg     180
   accttaaaca agtcagttga cctctgtgag cactgttgat cttaaataag tcagttgacc     240
   tctgtgagca ctgttccctc cctaattcag cattcagtat ctaccgaaga cgaactctgc     300
   tccaaacgct gagttaggct ctgggggagt agaaacatac aagggatatg gctcttgtct     360
   gcaaatggcc cttcgtccgc aggaggaaac agggctggtt tcacgtggtc agtcagggag     420
   gggccaagca aacctctggg aggctcctgg gccccagtcc ttgagatgac ttcagttccc     480
   ggtgggtggt gagccctgag ggtgtggccg gggtgaggca gtcctccact cagaggggct     540
   gcagaaatta gcccagctgt gtgatcttgg actagtcact gagcttctct gagcctcttt     600
   cctcctccca cttccccgaa tagtcaagcc ctgtcttgtg gtgcctttgt gggcaagccg     660
   actccttcat ctggcactca ctctggtgtg tttcagtggt tgtctgcctc tttccctggg     720
   ctctcagcag cgtgaaggct gggctatgtc aatcttattc acaggggtct cccctgctcc     780
   taatgcggtg ttgcatttag taggtgctca gttaatgtct gatgaaggat tgagtatata     840
   gatgagctgt ggagcaggga agggctttgc tggactccag ctgctgctct ctgatgcagc     900
   aggaatctct agtcccatca cggcgcctgc atcctcttca cggtaatacc tgatatttac     960
   tgaatgcctc caacattcca ggcagagttc tgtcaatgtt ctgatacact tgttcatcta    1020
   cttttctcct cacaatactc atttgccgtg gctgacatta tatctgtttt acagtccagc    1080
   tgaatttcag agcaattaaa taaatctgtg caaagtcata cagtaaagtg acagaacaag    1140
   attccagttc aggtgtcctg actccaaagc gttttgcttc cctctgtgca agctgcatcc    1200
   agggctctca gagctgcctg gacccagaga gtaccaatgt agtggctaca aagaacctac    1260
   agcatggcat gcaaagacct gtagccagag ggcgctcaag cgcgctccct tggctgccct    1320
   ggagtcagtg gggatggaga agcagtcttg gcagagtctg gggccaggat actaatgagc    1380
   tggtgcgcgt gggtgctcac ttttccccct ggcgtcagtc agcctcctta tggggacgac    1440
   tgtcatcccc tctctctttc aaccacatcg acaagcccct tctctctcct tgacccctga    1500
   ggagggattg tgcccggcag catccccgct tcctgtccgt gcatcccata gggagtggaa    1560
   gccagaggat gaaaagcgca gggttttttc ccttctcttc caaggaatcc accagatgcc    1620
   acactggctg gggaaccggc agtgcctctt ctactctact cagcctgcag ggtggccgag    1680
   ctttgtctcc catgttcatg attccacaac tttctggggc acctctcttt tcacatcctc    1740
   tcccacagcc tcctgtggtc tcagcctccc actcccctct cctcaccccg ccagtcctcc    1800
   cttctttttc tctttggcgg agctgtagaa gggcccctgg ggtcaaggaa gtgcagagac    1860
   taatagcctc ttagcataac tcctccggag tcatgcccaa ctggcctctg cttttgcata    1920
   cctcacgtta cattgaaaaa gcctccctgt gtttccacaa cttggtgtga agggcctcag    1980
   aaggaggatg gttgtgttct gtgcccccaa gaaagagctg gggaaaaaat gaagcctcct    2040
   gccatctatt gatcaacccc cttctctgta catacactct agaaagatga taaaggcacg    2100
   accctgtcct tatgacacgc tccgctccta agacactagg gacctgacct aggagacaat    2160
   tgtaaacccc agttcccatg gaagtcagcc tcgagagaac ctgaatttca tatgcaaggc    2220
   atgatgaaag cacagaggaa ggagagaata attctgagag caaatttgga gaggctttaa    2280
   ggagaagaag gctttgtgcc aggc                                           2304
<210> 27
   <211> 4905
   <212> DNA
   <213> Unknown
<220>
   <223> /note="Floxed promoterless pac vector, pB5-3Puro-PL" /note="Vector"
<400> 27
   gttgtaaaac gacggccagt gagcgcgcgt aatacgactc actatagggc gaattggagc      60
   tccaccgcgg tggcggccgc gggccctttg tgcggggggg agcggctcgg ggggtgcgtg     120
   cgtgtgtgtg tgcgtgggga gcgccgcgtg cggcccgcgc tgcccggcgg ctgtgagcgc     180
   tgcgggcgcg gcgcggggct ttgtgcgctc cgcgtgtgcg cgaggggagc gcggccgggg     240
   gcggtgcccc gcggtgcggg ggggctgcga ggggaacaaa ggctgcgtgc ggggtgtgtg     300
   cgtggggggg tgagcagggg gtgtgggcgc ggcggtcggg ctgtaacccc cccctgcacc     360
   cccctccccg agttgctgag cacggcccgg cttcgggtgc ggggctccgt gcggggcgtg     420
   gcgcggggct cgccgtgccg ggcggggggt ggcggcaggt gggggtgccg ggcggggcgg     480
   ggccgcctcg ggccggggag ggctcggggg aggggcgcgg cggccccgga gcgccggcgg     540
   ctgtcgaggc gcggcgagcc gcagccattg ccttttatgg taatcgtgcg agagggcgca     600
   gggacttcct ttgtcccaaa tctggcggag ccgaaatctg ggaggcgccg ccgcaccccc     660
   tctagcgggc gcgggcgaag cggtgcggcg ccggcaggaa ggaaatgggc ggggagggcc     720
   ttcgtgcgtc gccgcgccgc cgtccccttc tccatctcca gcctcggggc tgccgcaggg     780
   ggacggctgc cttcgggggg gacggggcag ggcggggttc ggcttctggc gtgtgaccgg     840
   cggctctaga gcctctgcta accatgttca tgccttcttc tttttcctac agctcctggg     900
   caacgtgctg gttgttgtgc tgtctcatca ttttggcaaa gaattcataa cttcgtataa     960
   agtatcctat acgaagttat accggtatga ccgagtacaa gcccacggtg cgcctcgcca    1020
   cccgcgacga cgtccccggg gccgtacgca ccctcgccgc cgcgttcgcc gactaccctg    1080
   caacacgcca tacagtggac cctgaccgcc acatcgagcg ggtcaccgag ctgcaagaac    1140
   tcttcctcac gcgcgtcggg ctcgacatcg gcaaggtgtg ggtcgcggac gacggcgccg    1200
   cggtggcggt ctggaccacg ccggagagcg tcgaagcggg ggcggtgttc gccgagatcg    1260
   gcccgcgcat ggccgagttg agcggttccc ggctggccgc gcagcaacag atggaaggcc    1320
   tcctggcgcc gcaccggccc aaggagcccg cgtggttcct ggccaccgtc ggcgtctcgc    1380
   ccgaccacca gggcaagggt ctgggcagcg ccgtcgtgct ccccggagtg gaggcggccg    1440
   agcgcgccgg ggtgcccgcc ttcctggaga cctccgcgcc ccgcaacctc cccttctacg 1500
   agcggctcgg cttcaccgtc accgccgacg tcgaggtgcc cgaaggaccg cgcacctggt 1560
   gcatgacccg caagcccggt gcctgaacta gtctcgagat aacttcgtat aatgtgtact 1620
   atacgaagtt atagatcttt ttccctctgc caaaaattat ggggacatca tgaagcccct 1680
   tgagcatctg acttctggct aataaaggaa atttattttc attgcaatag tgtgttggaa 1740
   ttttttgtgt ctctcactcg gaaggacata tgggagggca aatcatttaa aacatcagaa 1800
   tgagtatttg gtttagagtt tggcaacata tgccatatgc tggctgccat gaacaaaggt 1860
   ggctataaag aggtcatcag tatatgaaac agccccctgc tgtccattcc ttattccata 1920
   gaaaagcctt gacttgaggt tagatttttt ttatattttg ttttgtgtta tttttttctt 1980
   taacatccct aaaattttcc ttacatgttt tactagccag atttttcctc ctctcctgac 2040
   tactcccagt catagctgtc cctcttctct tatgaagatc cctcgacctg cagcccaagc 2100
   ttggtaccca gcttttgttc cctttagtga gggttaattg cgcgcttggc gtaatcatgg 2160
   tcatagctgt ttcctgtgtg aaattgttat ccgctcacaa ttccacacaa catacgagcc 2220
   ggaagcataa agtgtaaagc ctggggtgcc taatgagtga gctaactcac attaattgcg 2280
   ttgcgctcac tgcccgcttt ccagtcggga aacctgtcgt gccagctgca ttaatgaatc 2340
   ggccaacgcg cggggagagg cggtttgcgt attgggcgct cttccgcttc ctcgctcact 2400
   gactcgctgc gctcggtcgt tcggctgcgg cgagcggtat cagctcactc aaaggcggta 2460
   atacggttat ccacagaatc aggggataac gcaggaaaga acatgtgagc aaaaggccag 2520
   caaaaggcca ggaaccgtaa aaaggccgcg ttgctggcgt ttttccatag gctccgcccc 2580
   cctgacgagc atcacaaaaa tcgacgctca agtcagaggt ggcgaaaccc gacaggacta 2640
   taaagatacc aggcgtttcc ccctggaagc tccctcgtgc gctctcctgt tccgaccctg 2700
   ccgcttaccg gatacctgtc cgcctttctc ccttcgggaa gcgtggcgct ttctcatagc 2760
   tcacgctgta ggtatctcag ttcggtgtag gtcgttcgct ccaagctggg ctgtgtgcac 2820
   gaaccccccg ttcagcccga ccgctgcgcc ttatccggta actatcgtct tgagtccaac 2880
   ccggtaagac acgacttatc gccactggca gcagccactg gtaacaggat tagcagagcg 2940
   aggtatgtag gcggtgctac agagttcttg aagtggtggc ctaactacgg ctacactaga 3000
   aggacagtat ttggtatctg cgctctgctg aagccagtta ccttcggaaa aagagttggt 3060
   agctcttgat ccggcaaaca aaccaccgct ggtagcggtg gtttttttgt ttgcaagcag 3120
   cagattacgc gcagaaaaaa aggatctcaa gaagatcctt tgatcttttc tacggggtct 3180
   gacgctcagt ggaacgaaaa ctcacgttaa gggattttgg tcatgagatt atcaaaaagg 3240
   atcttcacct agatcctttt aaattaaaaa tgaagtttta aatcaatcta aagtatatat 3300
   gagtaaactt ggtctgacag ttaccaatgc ttaatcagtg aggcacctat ctcagcgatc 3360
   tgtctatttc gttcatccat agttgcctga ctccccgtcg tgtagataac tacgatacgg 3420
   gagggcttac catctggccc cagtgctgca atgataccgc gagacccacg ctcaccggct 3480
   ccagatttat cagcaataaa ccagccagcc ggaagggccg agcgcagaag tggtcctgca 3540
   actttatccg cctccatcca gtctattaat tgttgccggg aagctagagt aagtagttcg 3600
   ccagttaata gtttgcgcaa cgttgttgcc attgctacag gcatcgtggt gtcacgctcg 3660
   tcgtttggta tggcttcatt cagctccggt tcccaacgat caaggcgagt tacatgatcc 3720
   cccatgttgt gcaaaaaagc ggttagctcc ttcggtcctc cgatcgttgt cagaagtaag 3780
   ttggccgcag tgttatcact catggttatg gcagcactgc ataattctct tactgtcatg 3840
   ccatccgtaa gatgcttttc tgtgactggt gagtactcaa ccaagtcatt ctgagaatag 3900
   tgtatgcggc gaccgagttg ctcttgcccg gcgtcaatac gggataatac cgcgccacat 3960
   agcagaactt taaaagtgct catcattgga aaacgttctt cggggcgaaa actctcaagg 4020
   atcttaccgc tgttgagatc cagttcgatg taacccactc gtgcacccaa ctgatcttca 4080
   gcatctttta ctttcaccag cgtttctggg tgagcaaaaa caggaaggca aaatgccgca 4140
   aaaaagggaa taagggcgac acggaaatgt tgaatactca tactcttcct ttttcaatat 4200
   tattgaagca tttatcaggg ttattgtctc atgagcggat acatatttga atgtatttag 4260
   aaaaataaac aaataggggt tccgcgcaca tttccccgaa aagtgccacc taaattgtaa 4320
   gcgttaatat tttgttaaaa ttcgcgttaa atttttgtta aatcagctca ttttttaacc 4380
   aataggccga aatcggcaaa atcccttata aatcaaaaga atagaccgag atagggttga 4440
   gtgttgttcc agtttggaac aagagtccac tattaaagaa cgtggactcc aacgtcaaag 4500
   ggcgaaaaac cgtctatcag ggcgatggcc cactacgtga accatcaccc taatcaagtt 4560
   ttttggggtc gaggtgccgt aaagcactaa atcggaaccc taaagggagc ccccgattta 4620
   gagcttgacg gggaaagccg gcgaacgtgg cgagaaagga agggaagaaa gcgaaaggag 4680
   cgggcgctag ggcgctggca agtgtagcgg tcacgctgcg cgtaaccacc acacccgccg 4740
   cgcttaatgc gccgctacag ggcgcgtccc attcgccatt caggctgcgc aactgttggg 4800
   aagggcgatc ggtgcgggcc tcttcgctat tacgccagct ggcgaaaggg ggatgtgctg 4860
   caaggcgatt aagttgggta acgccagggt tttcccagtc acgac 4905
<210> 28
   <211> 5344
   <212> DNA
   <213> Unknown
<220>
   <223> /note="Vector" /note="Floxed promoterless hygromycin vector, pB5-3Hygro-PL"
<400> 28
   gttgtaaaac gacggccagt gagcgcgcgt aatacgactc actatagggc gaattggagc 60
   tccaccgcgg tggcggccgc gggccctttg tgcgggggga gcggctcggg gggtgcgtgc 120
   gtgtgtgtgt gcgtggggag cgccgcgtgc ggctccgcgc tgcccggcgg ctgtgagcgc 180
   tgcgggcgcg gcgcggggct ttgtgcgctc cgcagtgtgc gcgaggggag cgcggccggg 240
   ggcggtgccc cgcggtgcgg ggggggctgc gaggggaaca aaggctgcgt gcggggtgtg 300
   tgcgtggggg ggggcagggg ggtgtgggcg cgtcggtcgg gctgcaaccc cccctgcacc 360
   cccctccccg agttgctgag cacggcccgg cttcgggtgc ggggctccgt acggggcgtg 420
   gcgcggggct cgccgtgccg ggcggggggt ggcggcaggt gggggtgccg ggcggggcgg 480
   ggccgcctcg ggccggggag ggctcggggg aggggcgcgg cggcccccgg agcgccggcg 540
   gctgtcgagg cgcggcgagc cgcagccatt gccttttatg gtaatcgtgc gagagggcgc 600
   agggacttcc tttgtcccaa atctgtgcgg agccgaaatc tgggaggcgc cgccgcaccc 660
   cctctagcgg gcgcggggcg aagcggtgcg gcgccggcag gaaggaaatg ggcggggagg 720
   gccttcgtgc gtcgccgcgc cgccgtcccc ttctccctct ccagcctcgg ggctgtccgc 780
   ggggggacgg ctgccttcgg gggggacggg gcagggcggg gttcggcttc tggcgtgtga 840
   ccggcggctc tagagcctct gctaaccatg ttcatgcctt cttctttttc ctacagctcc 900
   tgggcaacgt gctggttatt gtgctgtctc atcattttgg caaagaattc ataacttcgt 960
   ataaagtatc ctatacgaag ttataccggt gatatcatgg atagatccgg aaagcctgaa 1020
   ctcaccgcga cgtctgtcga gaagtttctg atcgaaaagt tcgacagcgt ctccgacctg 1080
   atgcagctct cggagggcga agaatctcgt gctttcagct tcgatgtagg agggcgtgga 1140
   tatgtcctgc gggtaaatag ctgcgccgat ggtttctaca aagatcgtta tgtttatcgg 1200
   cactttgcat cggccgcgct cccgattccg gaagtgcttg acattgggga attcagcgag 1260
   agcctgacct attgcatctc ccgccgtgca cagggtgtca cgttgcaaga cctgcctgaa 1320
   accgaactgc ccgctgttct gcagccggtc gcggaggcca tggatgcgat cgctgcggcc 1380
   gatcttagcc agacgagcgg gttcggccca ttcggaccgc aaggaatcgg tcaatacact 1440
   acatggcgtg atttcatatg cgcgattgct gatccccatg tgtatcactg gcaaactgtg 1500
   atggacgaca ccgtcagtgc gtccgtcgcg caggctctcg atgagctgat gctttgggcc 1560
   gaggactgcc ccgaagtccg gcacctcgtg cacgcggatt tcggctccaa caatgtcctg 1620
   acggacaatg gccgcataac agcggtcatt gactggagcg aggcgatgtt cggggattcc 1680
   caatacgagg tcgccaacat cttcttctgg aggccgtggt tggcttgtat ggagcagcag 1740
   acgcgctact tcgagcggag gcatccggag cttgcaggat cgccgcggct ccgggcgtat 1800
   atgctccgca ttggtcttga ccaactctat cagagcttgg ttgacggcaa tttcgatgat 1860
   gcagcttggg cgcagggtcg atgcgacgca atcgtccgat ccggagccgg gactgtcggg 1920
   cgtacacaaa tcgcccgcag aagcgcggcc gtctggaccg atggctgtgt agaagtactc 1980
   gccgatagtg gaaaccgacg ccccagcact cgtccgaggg caaaggaata gctcgagata 2040
   acttcgtata atgtgtacta tacgaagtta tagatctttt tccctctgcc aaaaattatg 2100
   gggacatcat gaagcccctt gagcatctga cttctggcta ataaaggaaa tttattttca 2160
   ttgcaatagt gtgttggaat tttttgtgtc tctcactcgg aaggacatat gggagggcaa 2220
   atcatttaaa acatcagaat gagtatttgg tttagagttt ggcaacatat gccatatgct 2280
   ggctgccatg aacaaaggtg gctataaaga ggtcatcagt atatgaaaca gccccctgct 2340
   gtccattcct tattccatag aaaagccttg acttgaggtt agattttttt tatattttgt 2400
   tttgtgttat ttttttcttt aacatcccta aaattttcct tacatgtttt actagccaga 2460
   tttttcctcc tctcctgact actcccagtc atagctgtcc ctcttctctt atgaagatcc 2520
   ctcgacctgc agcccaagct tggtacccag cttttgttcc ctttagtgag ggttaattgc 2580
   gcgcttggcg taatcatggt catagctgtt tcctgtgtga aattgttatc cgctcacaat 2640
   tccacacaac atacgagccg gaagcataaa gtgtaaagcc tggggtgcct aatgagtgag 2700
   ctaactcaca ttaattgcgt tgcgctcact gcccgctttc cagtcgggaa acctgtcgtg 2760
   ccagctgcat taatgaatcg gccaacgcgc ggggagaggc ggtttgcgta ttgggcgctc 2820
   ttccgcttcc tcgctcactg actcgctgcg ctcggtcgtt cggctgcggc gagcggtatc 2880
   agctcactca aaggcggtaa tacggttatc cacagaatca ggggataacg caggaaagaa 2940
   catgtgagca aaaggccagc aaaaggccag gaaccgtaaa aaggccgcgt tgctggcgtt 3000
   tttccatagg ctccgccccc ctgacgagca tcacaaaaat cgacgctcaa gtcagaggtg 3060
   gcgaaacccg acaggactat aaagatacca ggcgtttccc cctggaagct ccctcgtgcg 3120
   ctctcctgtt ccgaccctgc cgcttaccgg atacctgtcc gcctttctcc cttcgggaag 3180
   cgtggcgctt tctcatagct cacgctgtag gtatctcagt tcggtgtagg tcgttcgctc 3240
   caagctgggc tgtgtgcacg aaccccccgt tcagcccgac cgctgcgcct tatccggtaa 3300
   ctatcgtctt gagtccaacc cggtaagaca cgacttatcg ccactggcag cagccactgg 3360
   taacaggatt agcagagcga ggtatgtagg cggtgctaca gagttcttga agtggtggcc 3420
   taactacggc tacactagaa ggacagtatt tggtatctgc gctctgctga agccagttac 3480
   cttcggaaaa agagttggta gctcttgatc cggcaaacaa accaccgctg gtagcggtgg 3540
   tttttttgtt tgcaagcagc agattacgcg cagaaaaaaa ggatctcaag aagatccttt 3600
   gatcttttct acggggtctg acgctcagtg gaacgaaaac tcacgttaag ggattttggt 3660
   catgagatta tcaaaaagga tcttcaccta gatcctttta aattaaaaat gaagttttaa 3720
   atcaatctaa agtatatatg agtaaacttg gtctgacagt taccaatgct taatcagtga 3780
   ggcacctatc tcagcgatct gtctatttcg ttcatccata gttgcctgac tccccgtcgt 3840
   gtagataact acgatacggg agggcttacc atctggcccc agtgctgcaa tgataccgcg 3900
   agacccacgc tcaccggctc cagatttatc agcaataaac cagccagccg gaagggccga 3960
   gcgcagaagt ggtcctgcaa ctttatccgc ctccatccag tctattaatt gttgccggga 4020
   agctagagta agtagttcgc cagttaatag tttgcgcaac gttgttgcca ttgctacagg 4080
   catcgtggtg tcacgctcgt cgtttggtat ggcttcattc agctccggtt cccaacgatc 4140
   aaggcgagtt acatgatccc ccatgttgtg caaaaaagcg gttagctcct tcggtcctcc 4200
   gatcgttgtc agaagtaagt tggccgcagt gttatcactc atggttatgg cagcactgca 4260
   taattctctt actgtcatgc catccgtaag atgcttttct gtgactggtg agtactcaac 4320
   caagtcattc tgagaatagt gtatgcggcg accgagttgc tcttgcccgg cgtcaatacg 4380
   ggataatacc gcgccacata gcagaacttt aaaagtgctc atcattggaa aacgttcttc 4440
   ggggcgaaaa ctctcaagga tcttaccgct gttgagatcc agttcgatgt aacccactcg 4500
   tgcacccaac tgatcttcag catcttttac tttcaccagc gtttctgggt gagcaaaaac 4560
   aggaaggcaa aatgccgcaa aaaagggaat aagggcgaca cggaaatgtt gaatactcat 4620
   actcttcctt tttcaatatt attgaagcat ttatcagggt tattgtctca tgagcggata 4680
   catatttgaa tgtatttaga aaaataaaca aataggggtt ccgcgcacat ttccccgaaa 4740
   agtgccacct aaattgtaag cgttaatatt ttgttaaaat tcgcgttaaa tttttgttaa 4800
   atcagctcat tttttaacca ataggccgaa atcggcaaaa tcccttataa atcaaaagaa 4860
   tagaccgaga tagggttgag tgttgttcca gtttggaaca agagtccact attaaagaac 4920
   gtggactcca acgtcaaagg gcgaaaaacc gtctatcagg gcgatggccc actacgtgaa 4980
   ccatcaccct aatcaagttt tttggggtcg aggtgccgta aagcactaaa tcggaaccct 5040
   aaagggagcc cccgatttag agcttgacgg ggaaagccgg cgaacgtggc gagaaaggaa 5100
   gggaagaaag cgaaaggagc gggcgctagg gcgctggcaa gtgtagcggt cacgctgcgc 5160
   gtaaccacca cacccgccgc gcttaatgcg ccgctacagg gcgcgtccca ttcgccattc 5220
   aggctgcgca actgttggga agggcgatcg gtgcgggcct cttcgctatt acgccagctg 5280
   gcgaaagggg gatgtgctgc aaggcgatta agttgggtaa cgccagggtt ttcccagtca 5340
   cgac 5344
<210> 29
   <211> 4987
   <212> DNA
   <213> Unknown
<220>
   <223> /note="Floxed promoterless DsRedT4 vector, pB5-3RedT4-PL" /note="Vector"
<400> 29
   gttgtaaaac gacggccagt gagcgcgcgt aatacgactc actatagggc gaattggagc      60
   tccaccgcgg tggcggccgc gggccctttg tgcgggggga gcggctcggg gggtgcgtgc     120
   gtgtgtgtgt gcgtggggag cgccgcgtgc ggctccgcgc tgcccggcgg ctgtgagcgc     180
   tgcgggcgcg gcgcggggct ttgtgcgctc cgcagtgtgc gcgaggggag cgcggccggg     240
   ggcggtgccc cgcggtgcgg ggggggctgc gaggggaaca aaggctgcgt gcggggtgtg     300
   tgcgtggggg ggggcagggg ggtgtgggcg cgtcggtcgg gctgcaaccc cccctgcacc     360
   cccctccccg agttgctgag cacggcccgg cttcgggtgc ggggctccgt acggggcgtg     420
   gcgcggggct cgccgtgccg ggcggggggt ggcggcaggt gggggtgccg ggcggggcgg     480
   ggccgcctcg ggccggggag ggctcggggg aggggcgcgg cggcccccgg agcgccggcg     540
   gctgtcgagg cgcggcgagc cgcagccatt gccttttatg gtaatcgtgc gagagggcgc     600
   agggacttcc tttgtcccaa atctgtgcgg agccgaaatc tgggaggcgc cgccgcaccc     660
   cctctagcgg gcgcggggcg aagcggtgcg gcgccggcag gaaggaaatg ggcggggagg     720
   gccttcgtgc gtcgccgcgc cgccgtcccc ttctccctct ccagcctcgg ggctgtccgc     780
   ggggggacgg ctgccttcgg gggggacggg gcagggcggg gttcggcttc tggcgtgtga     840
   ccggcggctc tagagcctct gctaaccatg ttcatgcctt cttctttttc ctacagctcc     900
   tgggcaacgt gctggttatt gtgctgtctc atcattttgg caaagaattc ataacttcgt     960
   ataaagtatc ctatacgaag ttatagatcc atggcctcct ccgaggacgt catcaaggag    1020
   ttcatgcgct tcaaggtgcg catggagggc tccgtgaacg gccacgagtt cgagatcgag    1080
   ggcgagggcg agggccgccc ctacgagggc acccagaccg ccaagctgaa ggtgaccaag    1140
   ggcggccccc tgcccttcgc ctgggacatc ctgtcccccc agttccagta cggctccaag    1200
   gtgtacgtga agcaccccgc cgacatcccc gactacaaga agctgtcctt ccccgagggc    1260
   ttcaagtggg agcgcgtgat gaacttcgag gacggcggcg tggtgaccgt gacccaggac    1320
   tcctccctgc aggacggctg cttcatctac aaggtgaagt tcatcggcgt gaacttcccc    1380
   tccgacggcc ccgtaatgca gaagaagact atgggctggg aggcctccac cgagcgcctg    1440
   tacccccgcg acggcgtgct gaagggcgag atccacaagg ccctgaagct gaaggacggc    1500
   ggccactacc tggtggagtt caagtccatc tacatggcca agaagcccgt gcagctgccc    1560
   ggctactact acgtggactc caagctggac atcacctccc acaacgagga ctacaccatc    1620
   gtggagcagt acgagcgcgc cgagggccgc caccacctgt tcctgtagag atcccggccg    1680
   ataacttcgt ataatgtgta ctatacgaag ttatagatct ttttccctct gccaaaaatt    1740
   atggggacat catgaagccc cttgagcatc tgacttctgg ctaataaagg aaatttattt    1800
   tcattgcaat agtgtgttgg aattttttgt gtctctcact cggaaggaca tatgggaggg 1860
   caaatcattt aaaacatcag aatgagtatt tggtttagag tttggcaaca tatgccatat 1920
   gctggctgcc atgaacaaag gtggctataa agaggtcatc agtatatgaa acagccccct 1980
   gctgtccatt ccttattcca tagaaaagcc ttgacttgag gttagatttt ttttatattt 2040
   tgttttgtgt tatttttttc tttaacatcc ctaaaatttt ccttacatgt tttactagcc 2100
   agatttttcc tcctctcctg actactccca gtcatagctg tccctcttct cttatgaaga 2160
   tccctcgacc tgcagcccaa gcttggtacc cagcttttgt tccctttagt gagggttaat 2220
   tgcgcgcttg gcgtaatcat ggtcatagct gtttcctgtg tgaaattgtt atccgctcac 2280
   aattccacac aacatacgag ccggaagcat aaagtgtaaa gcctggggtg cctaatgagt 2340
   gagctaactc acattaattg cgttgcgctc actgcccgct ttccagtcgg gaaacctgtc 2400
   gtgccagctg cattaatgaa tcggccaacg cgcggggaga ggcggtttgc gtattgggcg 2460
   ctcttccgct tcctcgctca ctgactcgct gcgctcggtc gttcggctgc ggcgagcggt 2520
   atcagctcac tcaaaggcgg taatacggtt atccacagaa tcaggggata acgcaggaaa 2580
   gaacatgtga gcaaaaggcc agcaaaaggc caggaaccgt aaaaaggccg cgttgctggc 2640
   gtttttccat aggctccgcc cccctgacga gcatcacaaa aatcgacgct caagtcagag 2700
   gtggcgaaac ccgacaggac tataaagata ccaggcgttt ccccctggaa gctccctcgt 2760
   gcgctctcct gttccgaccc tgccgcttac cggatacctg tccgcctttc tcccttcggg 2820
   aagcgtggcg ctttctcata gctcacgctg taggtatctc agttcggtgt aggtcgttcg 2880
   ctccaagctg ggctgtgtgc acgaaccccc cgttcagccc gaccgctgcg ccttatccgg 2940
   taactatcgt cttgagtcca acccggtaag acacgactta tcgccactgg cagcagccac 3000
   tggtaacagg attagcagag cgaggtatgt aggcggtgct acagagttct tgaagtggtg 3060
   gcctaactac ggctacacta gaaggacagt atttggtatc tgcgctctgc tgaagccagt 3120
   taccttcgga aaaagagttg gtagctcttg atccggcaaa caaaccaccg ctggtagcgg 3180
   tggttttttt gtttgcaagc agcagattac gcgcagaaaa aaaggatctc aagaagatcc 3240
   tttgatcttt tctacggggt ctgacgctca gtggaacgaa aactcacgtt aagggatttt 3300
   ggtcatgaga ttatcaaaaa ggatcttcac ctagatcctt ttaaattaaa aatgaagttt 3360
   taaatcaatc taaagtatat atgagtaaac ttggtctgac agttaccaat gcttaatcag 3420
   tgaggcacct atctcagcga tctgtctatt tcgttcatcc atagttgcct gactccccgt 3480
   cgtgtagata actacgatac gggagggctt accatctggc cccagtgctg caatgatacc 3540
   gcgagaccca cgctcaccgg ctccagattt atcagcaata aaccagccag ccggaagggc 3600
   cgagcgcaga agtggtcctg caactttatc cgcctccatc cagtctatta attgttgccg 3660
   ggaagctaga gtaagtagtt cgccagttaa tagtttgcgc aacgttgttg ccattgctac 3720
   aggcatcgtg gtgtcacgct cgtcgtttgg tatggcttca ttcagctccg gttcccaacg 3780
   atcaaggcga gttacatgat cccccatgtt gtgcaaaaaa gcggttagct ccttcggtcc 3840
   tccgatcgtt gtcagaagta agttggccgc agtgttatca ctcatggtta tggcagcact 3900
   gcataattct cttactgtca tgccatccgt aagatgcttt tctgtgactg gtgagtactc 3960
   aaccaagtca ttctgagaat agtgtatgcg gcgaccgagt tgctcttgcc cggcgtcaat 4020
   acgggataat accgcgccac atagcagaac tttaaaagtg ctcatcattg gaaaacgttc 4080
   ttcggggcga aaactctcaa ggatcttacc gctgttgaga tccagttcga tgtaacccac 4140
   tcgtgcaccc aactgatctt cagcatcttt tactttcacc agcgtttctg ggtgagcaaa 4200
   aacaggaagg caaaatgccg caaaaaaggg aataagggcg acacggaaat gttgaatact 4260
   catactcttc ctttttcaat attattgaag catttatcag ggttattgtc tcatgagcgg 4320
   atacatattt gaatgtattt agaaaaataa acaaataggg gttccgcgca catttccccg 4380
   aaaagtgcca cctaaattgt aagcgttaat attttgttaa aattcgcgtt aaatttttgt 4440
   taaatcagct cattttttaa ccaataggcc gaaatcggca aaatccctta taaatcaaaa 4500
   gaatagaccg agatagggtt gagtgttgtt ccagtttgga acaagagtcc actattaaag 4560
   aacgtggact ccaacgtcaa agggcgaaaa accgtctatc agggcgatgg cccactacgt 4620
   gaaccatcac cctaatcaag ttttttgggg tcgaggtgcc gtaaagcact aaatcggaac 4680
   cctaaaggga gcccccgatt tagagcttga cggggaaagc cggcgaacgt ggcgagaaag 4740
   gaagggaaga aagcgaaagg agcgggcgct agggcgctgg caagtgtagc ggtcacgctg 4800
   cgcgtaacca ccacacccgc cgcgcttaat gcgccgctac agggcgcgtc ccattcgcca 4860
   ttcaggctgc gcaactgttg ggaagggcga tcggtgcggg cctcttcgct attacgccag 4920
   ctggcgaaag ggggatgtgc tgcaaggcga ttaagttggg taacgccagg gttttcccag 4980
   tcacgac 4987
<210> 30
   <211> 567
   <212> RNA
   <213> Homo sapiens
<400> 30
   gaattccgga gcctcaactt caggatgttg acaacattgc tgccgatact gctgctgtct 60
   ggctgggcct tttgtagcca agacgcctca gatggcctcc aaagacttca tatgctccag 120
   atctcctact tccgcgaccc ctatcacgtg tggtaccagg gcaacgcgtc gctgggggga 180
   cacctaacgc acgtgctgga aggcccagac accaacacca cgatcattca gctgcagccc 240
   ttgcaggagc ccgagagctg ggcgcgcacg cagagtggcc tgcagtccta cctgctccag 300
   ttccacggcc tcgtgcgcct ggtgcaccag gagcggacct tggcctttcc tctgaccatc 360
   cgctgcttcc tgggctgtga gctgcctccc gagggctcta gagcccatgt cttcttcgaa 420
   gtggctgtga atgggagctc ctttgtgagt ttccggccgg agagagcctt gtggcaggca 480
   gacacccagg tcacctccgg agtggtcacc ttcaccctgc agcagctcaa tgcctacaac 540
   cgcactcggt atgaactgcg ggaattc 567
<210> 31
   <211> 867
   <212> DNA
   <213> Unknown
<220>
   <223> /note="DNA fragment of pCXEGFP vector" /note="Recombinant DNA"
<400> 31
   gtcgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtacgcccc ctattgacgt caatgacggt aaatggcccg 300
   cctggcatta tgcccagtac atgaccttat gggactttcc tacttggcag tacatctacg 360
   tattagtcat cgctattacc atggtcgagg tgagccccac gttctgcttc actctcccca 420
   tctccccccc ctccccaccc ccaattttgt atttatttat tttttaatta ttttgtgcag 480
   cgatgggggc gggggggggg ggggggcgcg cgccaggcgg ggcggggcgg ggcgaggggc 540
   ggggcggggc gaggcggaga ggtgcggcgg cagccaatca gagcggcgcg ctccgaaagt 600
   ttccttttat ggcgaggcgg cggcggcggc ggccctataa aaagcgaagc gcgcggcggg 660
   cgggagtcgc tgcgcgctgc cttcgccccg tgccccgctc cgcgccgcct cgcgccgccc 720
   gccccggctc tgactgaccg cgttactccc acaggtgagc gggcgggacg gcccttctcc 780
   tccgggctgt aattagcgct tggtttaatg acggcttgtt tcttttctgt ggctgcgtga 840
   aagccttgag gggctccggg agggccc 867
<210> 32
   <211> 6332
   <212> DNA
   <213> Unknown
<220>
   <223> /note="pC+ZFN/Puro 3 vector" /note="Recombinant DNA vector carrying the Seq ID NO:30 Human sequence."
<400> 32
   gttgtaaaac gacggccagt gagcgcgcgt aatacgactc actatagggc gaattggagc 60
   tccaccgcgg tggcggccgc gtcgacattg attattgact agttattaat agtaatcaat 120
   tacggggtca ttagttcata gcccatatat ggagttccgc gttacataac ttacggtaaa 180
   tggcccgcct ggctgaccgc ccaacgaccc ccgcccattg acgtcaataa tgacgtatgt 240
   tcccatagta acgccaatag ggactttcca ttgacgtcaa tgggtggagt atttacggta 300
   aactgcccac ttggcagtac atcaagtgta tcatatgcca agtacgcccc ctattgacgt 360
   caatgacggt aaatggcccg cctggcatta tgcccagtac atgaccttat gggactttcc 420
   tacttggcag tacatctacg tattagtcat cgctattacc atggtcgagg tgagccccac 480
   gttctgcttc actctcccca tctccccccc ctccccaccc ccaattttgt atttatttat 540
   tttttaatta ttttgtgcag cgatgggggc gggggggggg ggggggcgcg cgccaggcgg 600
   ggcggggcgg ggcgaggggc ggggcggggc gaggcggaga ggtgcggcgg cagccaatca 660
   gagcggcgcg ctccgaaagt ttccttttat ggcgaggcgg cggcggcggc ggccctataa 720
   aaagcgaagc gcgcggcggg cgggagtcgc tgcgcgctgc cttcgccccg tgccccgctc 780
   cgcgccgcct cgcgccgccc gccccggctc tgactgaccg cgttactccc acaggtgagc 840
   gggcgggacg gcccttctcc tccgggctgt aattagcgct tggtttaatg acggcttgtt 900
   tcttttctgt ggctgcgtga aagccttgag gggctccggg agggcccttt gtgcgggggg 960
   gagcggctcg gggggtgcgt gcgtgtgtgt gtgcgtgggg agcgccgcgt gcggcccgcg 1020
   ctgcccggcg gctgtgagcg ctgcgggcgc ggcgcggggc tttgtgcgct ccgcgtgtgc 1080
   gcgaggggag cgcggccggg ggcggtgccc cgcggtgcgg gggggctgcg aggggaacaa 1140
   aggctgcgtg cggggtgtgt gcgtgggggg gtgagcaggg ggtgtgggcg cggcggtcgg 1200
   gctgtaaccc ccccctgcac ccccctcccc gagttgctga gcacggcccg gcttcgggtg 1260
   cggggctccg tgcggggcgt ggcgcggggc tcgccgtgcc gggcgggggg tggcggcagg 1320
   tgggggtgcc gggcggggcg gggccgcctc gggccgggga gggctcgggg gaggggcgcg 1380
   gcggccccgg agcgccggcg gctgtcgagg cgcggcgagc cgcagccatt gccttttatg 1440
   gtaatcgtgc gagagggcgc agggacttcc tttgtcccaa atctggcgga gccgaaatct 1500
   gggaggcgcc gccgcacccc ctctagcggg cgcgggcgaa gcggtgcggc gccggcagga 1560
   aggaaatggg cggggagggc cttcgtgcgt cgccgcgccg ccgtcccctt ctccatctcc 1620
   agcctcgggg ctgccgcagg gggacggctg ccttcggggg ggacggggca gggcggggtt 1680
   cggcttctgg cgtgtgaccg gcggctctag agcctctgct aaccatgttc atgccttctt 1740
   ctttttccta cagctcctgg gcaacgtgct ggttgttgtg ctgtctcatc attttggcaa 1800
   agaattcgat tccggagcct caacttcagg atgttgacaa cattgctgcc gatactgctg 1860
   ctgtctggct gggccttttg tagccaagac gcctcagatg gcctccaaag acttcatatg 1920
   ctccagatct cctacttccg cgacccctat cacgtgtggt accagggcaa cgcgtcgctg 1980
   gggggacacc taacgcacgt gctggaaggc ccagacacca acaccacgat cattcagctg 2040
   cagcccttgc aggagcccga gagctgggcg cgcacgcaga gtggcctgca gtcctacctg 2100
   ctccagttcc acggcctcgt gcgcctggtg caccaggagc ggaccttggc ctttcctctg 2160
   accatccgct gcttcctggg ctgtgagctg cctcccgagg gctctagagc ccatgtcttc 2220
   ttcgaagtgg ctgtgaatgg gagctccttt gtgagtttcc ggccggagag agccttgtgg 2280
   caggcagaca cccaggtcac ctccggagtg gtcaccttca ccctgcagca gctcaatgcc 2340
   tacaaccgca ctcggtatga actgcgggaa ttcataactt cgtataaagt atcctatacg 2400
   aagttatacc ggtatgaccg agtacaagcc cacggtgcgc ctcgccaccc gcgacgacgt 2460
   cccccgggcc gtacgcaccc tcgccgccgc gttcgccgac taccctgcaa cacgccatac 2520
   agtggaccct gaccgccaca tcgagcgggt caccgagctg caagaactct tcctcacgcg 2580
   cgtcgggctc gacatcggca aggtgtgggt cgcggacgac ggcgccgcgg tggcggtctg 2640
   gaccacgccg gagagcgtcg aagcgggggc ggtgttcgcc gagatcggcc cgcgcatggc 2700
   cgagttgagc ggttcccggc tggccgcgca gcaacagatg gaaggcctcc tggcgccgca 2760
   ccggcccaag gagcccgcgt ggttcctggc caccgtcggc gtctcgcccg accaccaggg 2820
   caagggtctg ggcagcgccg tcgtgctccc cggagtggag gcggccgagc gcgccggggt 2880
   gcccgccttc ctggagacct ccgcgccccg caacctcccc ttctacgagc ggctcggctt 2940
   caccgtcacc gccgacgtcg aggtgcccga aggaccgcgc acctggtgca tgacccgcaa 3000
   gcccggtgcc tgaactagtc tcgagataac ttcgtataat gtgtactata cgaagttata 3060
   gatctttttc cctctgccaa aaattatggg gacatcatga agccccttga gcatctgact 3120
   tctggctaat aaaggaaatt tattttcatt gcaatagtgt gttggaattt tttgtgtctc 3180
   tcactcggaa ggacatatgg gagggcaaat catttaaaac atcagaatga gtatttggtt 3240
   tagagtttgg caacatatgc catatgctgg ctgccatgaa caaaggtggc tataaagagg 3300
   tcatcagtat atgaaacagc cccctgctgt ccattcctta ttccatagaa aagccttgac 3360
   ttgaggttag atttttttta tattttgttt tgtgttattt ttttctttaa catccctaaa 3420
   attttcctta catgttttac tagccagatt tttcctcctc tcctgactac tcccagtcat 3480
   agctgtccct cttctcttat gaagatccct cgacctgcag cccaagcttg gtacccagct 3540
   tttgttccct ttagtgaggg ttaattgcgc gcttggcgta atcatggtca tagctgtttc 3600
   ctgtgtgaaa ttgttatccg ctcacaattc cacacaacat acgagccgga agcataaagt 3660
   gtaaagcctg gggtgcctaa tgagtgagct aactcacatt aattgcgttg cgctcactgc 3720
   ccgctttcca gtcgggaaac ctgtcgtgcc agctgcatta atgaatcggc caacgcgcgg 3780
   ggagaggcgg tttgcgtatt gggcgctctt ccgcttcctc gctcactgac tcgctgcgct 3840
   cggtcgttcg gctgcggcga gcggtatcag ctcactcaaa ggcggtaata cggttatcca 3900
   cagaatcagg ggataacgca ggaaagaaca tgtgagcaaa aggccagcaa aaggccagga 3960
   accgtaaaaa ggccgcgttg ctggcgtttt tccataggct ccgcccccct gacgagcatc 4020
   acaaaaatcg acgctcaagt cagaggtggc gaaacccgac aggactataa agataccagg 4080
   cgtttccccc tggaagctcc ctcgtgcgct ctcctgttcc gaccctgccg cttaccggat 4140
   acctgtccgc ctttctccct tcgggaagcg tggcgctttc tcatagctca cgctgtaggt 4200
   atctcagttc ggtgtaggtc gttcgctcca agctgggctg tgtgcacgaa ccccccgttc 4260
   agcccgaccg ctgcgcctta tccggtaact atcgtcttga gtccaacccg gtaagacacg 4320
   acttatcgcc actggcagca gccactggta acaggattag cagagcgagg tatgtaggcg 4380
   gtgctacaga gttcttgaag tggtggccta actacggcta cactagaagg acagtatttg 4440
   gtatctgcgc tctgctgaag ccagttacct tcggaaaaag agttggtagc tcttgatccg 4500
   gcaaacaaac caccgctggt agcggtggtt tttttgtttg caagcagcag attacgcgca 4560
   gaaaaaaagg atctcaagaa gatcctttga tcttttctac ggggtctgac gctcagtgga 4620
   acgaaaactc acgttaaggg attttggtca tgagattatc aaaaaggatc ttcacctaga 4680
   tccttttaaa ttaaaaatga agttttaaat caatctaaag tatatatgag taaacttggt 4740
   ctgacagtta ccaatgctta atcagtgagg cacctatctc agcgatctgt ctatttcgtt 4800
   catccatagt tgcctgactc cccgtcgtgt agataactac gatacgggag ggcttaccat 4860
   ctggccccag tgctgcaatg ataccgcgag acccacgctc accggctcca gatttatcag 4920
   caataaacca gccagccgga agggccgagc gcagaagtgg tcctgcaact ttatccgcct 4980
   ccatccagtc tattaattgt tgccgggaag ctagagtaag tagttcgcca gttaatagtt 5040
   tgcgcaacgt tgttgccatt gctacaggca tcgtggtgtc acgctcgtcg tttggtatgg 5100
   cttcattcag ctccggttcc caacgatcaa ggcgagttac atgatccccc atgttgtgca 5160
   aaaaagcggt tagctccttc ggtcctccga tcgttgtcag aagtaagttg gccgcagtgt 5220
   tatcactcat ggttatggca gcactgcata attctcttac tgtcatgcca tccgtaagat 5280
   gcttttctgt gactggtgag tactcaacca agtcattctg agaatagtgt atgcggcgac 5340
   cgagttgctc ttgcccggcg tcaatacggg ataataccgc gccacatagc agaactttaa 5400
   aagtgctcat cattggaaaa cgttcttcgg ggcgaaaact ctcaaggatc ttaccgctgt 5460
   tgagatccag ttcgatgtaa cccactcgtg cacccaactg atcttcagca tcttttactt 5520
   tcaccagcgt ttctgggtga gcaaaaacag gaaggcaaaa tgccgcaaaa aagggaataa 5580
   gggcgacacg gaaatgttga atactcatac tcttcctttt tcaatattat tgaagcattt 5640
   atcagggtta ttgtctcatg agcggataca tatttgaatg tatttagaaa aataaacaaa 5700
   taggggttcc gcgcacattt ccccgaaaag tgccacctaa attgtaagcg ttaatatttt      5760
   gttaaaattc gcgttaaatt tttgttaaat cagctcattt tttaaccaat aggccgaaat      5820
   cggcaaaatc ccttataaat caaaagaata gaccgagata gggttgagtg ttgttccagt      5880
   ttggaacaag agtccactat taaagaacgt ggactccaac gtcaaagggc gaaaaaccgt      5940
   ctatcagggc gatggcccac tacgtgaacc atcaccctaa tcaagttttt tggggtcgag      6000
   gtgccgtaaa gcactaaatc ggaaccctaa agggagcccc cgatttagag cttgacgggg      6060
   aaagccggcg aacgtggcga gaaaggaagg gaagaaagcg aaaggagcgg gcgctagggc      6120
   gctggcaagt gtagcggtca cgctgcgcgt aaccaccaca cccgccgcgc ttaatgcgcc      6180
   gctacagggc gcgtcccatt cgccattcag gctgcgcaac tgttgggaag ggcgatcggt      6240
   gcgggcctct tcgctattac gccagctggc gaaaggggga tgtgctgcaa ggcgattaag      6300
   ttgggtaacg ccagggtttt cccagtcacg ac                                    6332
<210> 33
   <211> 6767
   <212> DNA
   <213> Unknown
<220>
   <223> /note="pC+ZFN/Hygro vector" /note="Recombinant DNA vector carrying the Seq ID NO:30 Human sequence."
<400> 33
   gttgtaaaac gacggccagt gagcgcgcgt aatacgactc actatagggc gaattggagc 60
   tccaccgcgg tggcggccgc gtcgacattg attattgact agttattaat agtaatcaat 120
   tacggggtca ttagttcata gcccatatat ggagttccgc gttacataac ttacggtaaa 180
   tggcccgcct ggctgaccgc ccaacgaccc ccgcccattg acgtcaataa tgacgtatgt 240
   tcccatagta acgccaatag ggactttcca ttgacgtcaa tgggtggagt atttacggta 300
   aactgcccac ttggcagtac atcaagtgta tcatatgcca agtacgcccc ctattgacgt 360
   caatgacggt aaatggcccg cctggcatta tgcccagtac atgaccttat gggactttcc 420
   tacttggcag tacatctacg tattagtcat cgctattacc atggtcgagg tgagccccac 480
   gttctgcttc actctcccca tctccccccc ctccccaccc ccaattttgt atttatttat 540
   tttttaatta ttttgtgcag cgatgggggc gggggggggg ggggggcgcg cgccaggcgg 600
   ggcggggcgg ggcgaggggc ggggcggggc gaggcggaga ggtgcggcgg cagccaatca 660
   gagcggcgcg ctccgaaagt ttccttttat ggcgaggcgg cggcggcggc ggccctataa 720
   aaagcgaagc gcgcggcggg cgggagtcgc tgcgcgctgc cttcgccccg tgccccgctc 780
   cgcgccgcct cgcgccgccc gccccggctc tgactgaccg cgttactccc acaggtgagc 840
   gggcgggacg gcccttctcc tccgggctgt aattagcgct tggtttaatg acggcttgtt 900
   tcttttctgt ggctgcgtga aagccttgag gggctccggg agggcccttt gtgcgggggg 960
   gagcggctcg gggggtgcgt gcgtgtgtgt gtgcgtgggg agcgccgcgt gcggcccgcg 1020
   ctgcccggcg gctgtgagcg ctgcgggcgc ggcgcggggc tttgtgcgct ccgcgtgtgc 1080
   gcgaggggag cgcggccggg ggcggtgccc cgcggtgcgg gggggctgcg aggggaacaa 1140
   aggctgcgtg cggggtgtgt gcgtgggggg gtgagcaggg ggtgtgggcg cggcggtcgg 1200
   gctgtaaccc ccccctgcac ccccctcccc gagttgctga gcacggcccg gcttcgggtg 1260
   cggggctccg tgcggggcgt ggcgcggggc tcgccgtgcc gggcgggggg tggcggcagg 1320
   tgggggtgcc gggcggggcg gggccgcctc gggccgggga gggctcgggg gaggggcgcg 1380
   gcggccccgg agcgccggcg gctgtcgagg cgcggcgagc cgcagccatt gccttttatg 1440
   gtaatcgtgc gagagggcgc agggacttcc tttgtcccaa atctggcgga gccgaaatct 1500
   gggaggcgcc gccgcacccc ctctagcggg cgcgggcgaa gcggtgcggc gccggcagga 1560
   aggaaatggg cggggagggc cttcgtgcgt cgccgcgccg ccgtcccctt ctccatctcc 1620
   agcctcgggg ctgccgcagg gggacggctg ccttcggggg ggacggggca gggcggggtt 1680
   cggcttctgg cgtgtgaccg gcggctctag agcctctgct aaccatgttc atgccttctt 1740
   ctttttccta cagctcctgg gcaacgtgct ggttgttgtg ctgtctcatc attttggcaa 1800
   agaattcgat tccggagcct caacttcagg atgttgacaa cattgctgcc gatactgctg 1860
   ctgtctggct gggccttttg tagccaagac gcctcagatg gcctccaaag acttcatatg 1920
   ctccagatct cctacttccg cgacccctat cacgtgtggt accagggcaa cgcgtcgctg 1980
   gggggacacc taacgcacgt gctggaaggc ccagacacca acaccacgat cattcagctg 2040
   cagcccttgc aggagcccga gagctgggcg cgcacgcaga gtggcctgca gtcctacctg 2100
   ctccagttcc acggcctcgt gcgcctggtg caccaggagc ggaccttggc ctttcctctg 2160
   accatccgct gcttcctggg ctgtgagctg cctcccgagg gctctagagc ccatgtcttc 2220
   ttcgaagtgg ctgtgaatgg gagctccttt gtgagtttcc ggccggagag agccttgtgg 2280
   caggcagaca cccaggtcac ctccggagtg gtcaccttca ccctgcagca gctcaatgcc 2340
   tacaaccgca ctcggtatga actgcgggaa ttcataactt cgtataaagt atcctatacg 2400
   aagttatacc ggtatggata gatccggaaa gcctgaactc accgcgacgt ctgtcgagaa 2460
   gtttctgatc gaaaagttcg acagcgtctc cgacctgatg cagctctcgg agggcgaaga 2520
   atctcgtgct ttcagcttcg atgtaggagg gcgtggatat gtcctgcggg taaatagctg 2580
   cgccgatggt ttctacaaag atcgttatgt ttatcggcac tttgcatcgg ccgcgctccc 2640
   gattccggaa gtgcttgaca ttggggaatt cagcgagagc ctgacctatt gcatctcccg 2700
   ccgtgcacag ggtgtcacgt tgcaagacct gcctgaaacc gaactgcccg ctgttctgca 2760
   gccggtcgcg gaggccatgg atgcgatcgc tgcggccgat cttagccaga cgagcgggtt 2820
   cggcccattc ggaccgcaag gaatcggtca atacactaca tggcgtgatt tcatatgcgc 2880
   gattgctgat ccccatgtgt atcactggca aactgtgatg gacgacaccg tcagtgcgtc 2940
   cgtcgcgcag gctctcgatg agctgatgct ttgggccgag gactgccccg aagtccggca 3000
   cctcgtgcac gcggatttcg gctccaacaa tgtcctgacg gacaatggcc gcataacagc 3060
   ggtcattgac tggagcgagg cgatgttcgg ggattcccaa tacgaggtcg ccaacatctt 3120
   cttctggagg ccgtggttgg cttgtatgga gcagcagacg cgctacttcg agcggaggca 3180
   tccggagctt gcaggatcgc cgcggctccg ggcgtatatg ctccgcattg gtcttgacca 3240
   actctatcag agcttggttg acggcaattt cgatgatgca gcttgggcgc agggtcgatg 3300
   cgacgcaatc gtccgatccg gagccgggac tgtcgggcgt acacaaatcg cccgcagaag 3360
   cgcggccgtc tggaccgatg gctgtgtaga agtactcgcc gatagtggaa accgacgccc 3420
   cagcactcgt ccgagggcaa aggaatagac tagtctcgag ataacttcgt ataatgtgta 3480
   ctatacgaag ttatagatct ttttccctct gccaaaaatt atggggacat catgaagccc 3540
   cttgagcatc tgacttctgg ctaataaagg aaatttattt tcattgcaat agtgtgttgg 3600
   aattttttgt gtctctcact cggaaggaca tatgggaggg caaatcattt aaaacatcag 3660
   aatgagtatt tggtttagag tttggcaaca tatgccatat gctggctgcc atgaacaaag 3720
   gtggctataa agaggtcatc agtatatgaa acagccccct gctgtccatt ccttattcca 3780
   tagaaaagcc ttgacttgag gttagatttt ttttatattt tgttttgtgt tatttttttc 3840
   tttaacatcc ctaaaatttt ccttacatgt tttactagcc agatttttcc tcctctcctg 3900
   actactccca gtcatagctg tccctcttct cttatgaaga tccctcgacc tgcagcccaa 3960
   gcttggtacc cagcttttgt tccctttagt gagggttaat tgcgcgcttg gcgtaatcat 4020
   ggtcatagct gtttcctgtg tgaaattgtt atccgctcac aattccacac aacatacgag 4080
   ccggaagcat aaagtgtaaa gcctggggtg cctaatgagt gagctaactc acattaattg 4140
   cgttgcgctc actgcccgct ttccagtcgg gaaacctgtc gtgccagctg cattaatgaa 4200
   tcggccaacg cgcggggaga ggcggtttgc gtattgggcg ctcttccgct tcctcgctca 4260
   ctgactcgct gcgctcggtc gttcggctgc ggcgagcggt atcagctcac tcaaaggcgg 4320
   taatacggtt atccacagaa tcaggggata acgcaggaaa gaacatgtga gcaaaaggcc 4380
   agcaaaaggc caggaaccgt aaaaaggccg cgttgctggc gtttttccat aggctccgcc 4440
   cccctgacga gcatcacaaa aatcgacgct caagtcagag gtggcgaaac ccgacaggac 4500
   tataaagata ccaggcgttt ccccctggaa gctccctcgt gcgctctcct gttccgaccc 4560
   tgccgcttac cggatacctg tccgcctttc tcccttcggg aagcgtggcg ctttctcata 4620
   gctcacgctg taggtatctc agttcggtgt aggtcgttcg ctccaagctg ggctgtgtgc 4680
   acgaaccccc cgttcagccc gaccgctgcg ccttatccgg taactatcgt cttgagtcca 4740
   acccggtaag acacgactta tcgccactgg cagcagccac tggtaacagg attagcagag 4800
   cgaggtatgt aggcggtgct acagagttct tgaagtggtg gcctaactac ggctacacta 4860
   gaaggacagt atttggtatc tgcgctctgc tgaagccagt taccttcgga aaaagagttg 4920
   gtagctcttg atccggcaaa caaaccaccg ctggtagcgg tggttttttt gtttgcaagc 4980
   agcagattac gcgcagaaaa aaaggatctc aagaagatcc tttgatcttt tctacggggt 5040
   ctgacgctca gtggaacgaa aactcacgtt aagggatttt ggtcatgaga ttatcaaaaa 5100
   ggatcttcac ctagatcctt ttaaattaaa aatgaagttt taaatcaatc taaagtatat 5160
   atgagtaaac ttggtctgac agttaccaat gcttaatcag tgaggcacct atctcagcga 5220
   tctgtctatt tcgttcatcc atagttgcct gactccccgt cgtgtagata actacgatac 5280
   gggagggctt accatctggc cccagtgctg caatgatacc gcgagaccca cgctcaccgg 5340
   ctccagattt atcagcaata aaccagccag ccggaagggc cgagcgcaga agtggtcctg 5400
   caactttatc cgcctccatc cagtctatta attgttgccg ggaagctaga gtaagtagtt 5460
   cgccagttaa tagtttgcgc aacgttgttg ccattgctac aggcatcgtg gtgtcacgct 5520
   cgtcgtttgg tatggcttca ttcagctccg gttcccaacg atcaaggcga gttacatgat 5580
   cccccatgtt gtgcaaaaaa gcggttagct ccttcggtcc tccgatcgtt gtcagaagta 5640
   agttggccgc agtgttatca ctcatggtta tggcagcact gcataattct cttactgtca 5700
   tgccatccgt aagatgcttt tctgtgactg gtgagtactc aaccaagtca ttctgagaat 5760
   agtgtatgcg gcgaccgagt tgctcttgcc cggcgtcaat acgggataat accgcgccac 5820
   atagcagaac tttaaaagtg ctcatcattg gaaaacgttc ttcggggcga aaactctcaa 5880
   ggatcttacc gctgttgaga tccagttcga tgtaacccac tcgtgcaccc aactgatctt 5940
   cagcatcttt tactttcacc agcgtttctg ggtgagcaaa aacaggaagg caaaatgccg 6000
   caaaaaaggg aataagggcg acacggaaat gttgaatact catactcttc ctttttcaat 6060
   attattgaag catttatcag ggttattgtc tcatgagcgg atacatattt gaatgtattt 6120
   agaaaaataa acaaataggg gttccgcgca catttccccg aaaagtgcca cctaaattgt 6180
   aagcgttaat attttgttaa aattcgcgtt aaatttttgt taaatcagct cattttttaa 6240
   ccaataggcc gaaatcggca aaatccctta taaatcaaaa gaatagaccg agatagggtt 6300
   gagtgttgtt ccagtttgga acaagagtcc actattaaag aacgtggact ccaacgtcaa 6360
   agggcgaaaa accgtctatc agggcgatgg cccactacgt gaaccatcac cctaatcaag 6420
   ttttttgggg tcgaggtgcc gtaaagcact aaatcggaac cctaaaggga gcccccgatt 6480
   tagagcttga cggggaaagc cggcgaacgt ggcgagaaag gaagggaaga aagcgaaagg 6540
   agcgggcgct agggcgctgg caagtgtagc ggtcacgctg cgcgtaacca ccacacccgc 6600
   cgcgcttaat gcgccgctac agggcgcgtc ccattcgcca ttcaggctgc gcaactgttg 6660
   ggaagggcga tcggtgcggg cctcttcgct attacgccag ctggcgaaag ggggatgtgc 6720
   tgcaaggcga ttaagttggg taacgccagg gttttcccag tcacgac 6767
<210> 34
   <211> 6410
   <212> DNA
   <213> Unknown
<220>
   <223> /note="Recombinant DNA vector carrying the Seq ID NO:30 Human sequence." /note="pC+ZFN/RedT vector"
<400> 34
   gttgtaaaac gacggccagt gagcgcgcgt aatacgactc actatagggc gaattggagc      60
   tccaccgcgg tggcggccgc gtcgacattg attattgact agttattaat agtaatcaat     120
   tacggggtca ttagttcata gcccatatat ggagttccgc gttacataac ttacggtaaa     180
   tggcccgcct ggctgaccgc ccaacgaccc ccgcccattg acgtcaataa tgacgtatgt     240
   tcccatagta acgccaatag ggactttcca ttgacgtcaa tgggtggagt atttacggta     300
   aactgcccac ttggcagtac atcaagtgta tcatatgcca agtacgcccc ctattgacgt     360
   caatgacggt aaatggcccg cctggcatta tgcccagtac atgaccttat gggactttcc     420
   tacttggcag tacatctacg tattagtcat cgctattacc atggtcgagg tgagccccac     480
   gttctgcttc actctcccca tctccccccc ctccccaccc ccaattttgt atttatttat     540
   tttttaatta ttttgtgcag cgatgggggc gggggggggg ggggggcgcg cgccaggcgg     600
   ggcggggcgg ggcgaggggc ggggcggggc gaggcggaga ggtgcggcgg cagccaatca     660
   gagcggcgcg ctccgaaagt ttccttttat ggcgaggcgg cggcggcggc ggccctataa     720
   aaagcgaagc gcgcggcggg cgggagtcgc tgcgcgctgc cttcgccccg tgccccgctc     780
   cgcgccgcct cgcgccgccc gccccggctc tgactgaccg cgttactccc acaggtgagc     840
   gggcgggacg gcccttctcc tccgggctgt aattagcgct tggtttaatg acggcttgtt     900
   tcttttctgt ggctgcgtga aagccttgag gggctccggg agggcccttt gtgcgggggg     960
   gagcggctcg gggggtgcgt gcgtgtgtgt gtgcgtgggg agcgccgcgt gcggcccgcg    1020
   ctgcccggcg gctgtgagcg ctgcgggcgc ggcgcggggc tttgtgcgct ccgcgtgtgc    1080
   gcgaggggag cgcggccggg ggcggtgccc cgcggtgcgg gggggctgcg aggggaacaa    1140
   aggctgcgtg cggggtgtgt gcgtgggggg gtgagcaggg ggtgtgggcg cggcggtcgg    1200
   gctgtaaccc ccccctgcac ccccctcccc gagttgctga gcacggcccg gcttcgggtg    1260
   cggggctccg tgcggggcgt ggcgcggggc tcgccgtgcc gggcgggggg tggcggcagg    1320
   tgggggtgcc gggcggggcg gggccgcctc gggccgggga gggctcgggg gaggggcgcg 1380
   gcggccccgg agcgccggcg gctgtcgagg cgcggcgagc cgcagccatt gccttttatg 1440
   gtaatcgtgc gagagggcgc agggacttcc tttgtcccaa atctggcgga gccgaaatct 1500
   gggaggcgcc gccgcacccc ctctagcggg cgcgggcgaa gcggtgcggc gccggcagga 1560
   aggaaatggg cggggagggc cttcgtgcgt cgccgcgccg ccgtcccctt ctccatctcc 1620
   agcctcgggg ctgccgcagg gggacggctg ccttcggggg ggacggggca gggcggggtt 1680
   cggcttctgg cgtgtgaccg gcggctctag agcctctgct aaccatgttc atgccttctt 1740
   ctttttccta cagctcctgg gcaacgtgct ggttgttgtg ctgtctcatc attttggcaa 1800
   agaattcgat tccggagcct caacttcagg atgttgacaa cattgctgcc gatactgctg 1860
   ctgtctggct gggccttttg tagccaagac gcctcagatg gcctccaaag acttcatatg 1920
   ctccagatct cctacttccg cgacccctat cacgtgtggt accagggcaa cgcgtcgctg 1980
   gggggacacc taacgcacgt gctggaaggc ccagacacca acaccacgat cattcagctg 2040
   cagcccttgc aggagcccga gagctgggcg cgcacgcaga gtggcctgca gtcctacctg 2100
   ctccagttcc acggcctcgt gcgcctggtg caccaggagc ggaccttggc ctttcctctg 2160
   accatccgct gcttcctggg ctgtgagctg cctcccgagg gctctagagc ccatgtcttc 2220
   ttcgaagtgg ctgtgaatgg gagctccttt gtgagtttcc ggccggagag agccttgtgg 2280
   caggcagaca cccaggtcac ctccggagtg gtcaccttca ccctgcagca gctcaatgcc 2340
   tacaaccgca ctcggtatga actgcgggaa ttcataactt cgtataaagt atcctatacg 2400
   aagttatacc ggtatggcct cctccgagga cgtcatcaag gagttcatgc gcttcaaggt 2460
   gcgcatggag ggctccgtga acggccacga gttcgagatc gagggcgagg gcgagggccg 2520
   cccctacgag ggcacccaga ccgccaagct gaaggtgacc aagggcggcc ccctgccctt 2580
   cgcctgggac atcctgtccc cccagttcca gtacggctcc aaggtgtacg tgaagcaccc 2640
   cgccgacatc cccgactaca agaagctgtc cttccccgag ggcttcaagt gggagcgcgt 2700
   gatgaacttc gaggacggcg gcgtggtgac cgtgacccag gactcctccc tgcaggacgg 2760
   ctgcttcatc tacaaggtga agttcatcgg cgtgaacttc ccctccgacg gccccgtaat 2820
   gcagaagaag actatgggct gggaggcctc caccgagcgc ctgtaccccc gcgacggcgt 2880
   gctgaagggc gagatccaca aggccctgaa gctgaaggac ggcggccact acctggtgga 2940
   gttcaagtcc atctacatgg ccaagaagcc cgtgcagctg cccggctact actacgtgga 3000
   ctccaagctg gacatcacct cccacaacga ggactacacc atcgtggagc agtacgagcg 3060
   cgccgagggc cgccaccacc tgttcctgta gactagtctc gagataactt cgtataatgt 3120
   gtactatacg aagttataga tctttttccc tctgccaaaa attatgggga catcatgaag 3180
   ccccttgagc atctgacttc tggctaataa aggaaattta ttttcattgc aatagtgtgt 3240
   tggaattttt tgtgtctctc actcggaagg acatatggga gggcaaatca tttaaaacat 3300
   cagaatgagt atttggttta gagtttggca acatatgcca tatgctggct gccatgaaca 3360
   aaggtggcta taaagaggtc atcagtatat gaaacagccc cctgctgtcc attccttatt 3420
   ccatagaaaa gccttgactt gaggttagat tttttttata ttttgttttg tgttattttt 3480
   ttctttaaca tccctaaaat tttccttaca tgttttacta gccagatttt tcctcctctc 3540
   ctgactactc ccagtcatag ctgtccctct tctcttatga agatccctcg acctgcagcc 3600
   caagcttggt acccagcttt tgttcccttt agtgagggtt aattgcgcgc ttggcgtaat 3660
   catggtcata gctgtttcct gtgtgaaatt gttatccgct cacaattcca cacaacatac 3720
   gagccggaag cataaagtgt aaagcctggg gtgcctaatg agtgagctaa ctcacattaa 3780
   ttgcgttgcg ctcactgccc gctttccagt cgggaaacct gtcgtgccag ctgcattaat 3840
   gaatcggcca acgcgcgggg agaggcggtt tgcgtattgg gcgctcttcc gcttcctcgc 3900
   tcactgactc gctgcgctcg gtcgttcggc tgcggcgagc ggtatcagct cactcaaagg 3960
   cggtaatacg gttatccaca gaatcagggg ataacgcagg aaagaacatg tgagcaaaag 4020
   gccagcaaaa ggccaggaac cgtaaaaagg ccgcgttgct ggcgtttttc cataggctcc 4080
   gcccccctga cgagcatcac aaaaatcgac gctcaagtca gaggtggcga aacccgacag 4140
   gactataaag ataccaggcg tttccccctg gaagctccct cgtgcgctct cctgttccga 4200
   ccctgccgct taccggatac ctgtccgcct ttctcccttc gggaagcgtg gcgctttctc 4260
   atagctcacg ctgtaggtat ctcagttcgg tgtaggtcgt tcgctccaag ctgggctgtg 4320
   tgcacgaacc ccccgttcag cccgaccgct gcgccttatc cggtaactat cgtcttgagt 4380
   ccaacccggt aagacacgac ttatcgccac tggcagcagc cactggtaac aggattagca 4440
   gagcgaggta tgtaggcggt gctacagagt tcttgaagtg gtggcctaac tacggctaca 4500
   ctagaaggac agtatttggt atctgcgctc tgctgaagcc agttaccttc ggaaaaagag 4560
   ttggtagctc ttgatccggc aaacaaacca ccgctggtag cggtggtttt tttgtttgca 4620
   agcagcagat tacgcgcaga aaaaaaggat ctcaagaaga tcctttgatc ttttctacgg 4680
   ggtctgacgc tcagtggaac gaaaactcac gttaagggat tttggtcatg agattatcaa 4740
   aaaggatctt cacctagatc cttttaaatt aaaaatgaag ttttaaatca atctaaagta 4800
   tatatgagta aacttggtct gacagttacc aatgcttaat cagtgaggca cctatctcag 4860
   cgatctgtct atttcgttca tccatagttg cctgactccc cgtcgtgtag ataactacga 4920
   tacgggaggg cttaccatct ggccccagtg ctgcaatgat accgcgagac ccacgctcac 4980
   cggctccaga tttatcagca ataaaccagc cagccggaag ggccgagcgc agaagtggtc 5040
   ctgcaacttt atccgcctcc atccagtcta ttaattgttg ccgggaagct agagtaagta 5100
   gttcgccagt taatagtttg cgcaacgttg ttgccattgc tacaggcatc gtggtgtcac 5160
   gctcgtcgtt tggtatggct tcattcagct ccggttccca acgatcaagg cgagttacat 5220
   gatcccccat gttgtgcaaa aaagcggtta gctccttcgg tcctccgatc gttgtcagaa 5280
   gtaagttggc cgcagtgtta tcactcatgg ttatggcagc actgcataat tctcttactg 5340
   tcatgccatc cgtaagatgc ttttctgtga ctggtgagta ctcaaccaag tcattctgag 5400
   aatagtgtat gcggcgaccg agttgctctt gcccggcgtc aatacgggat aataccgcgc 5460
   cacatagcag aactttaaaa gtgctcatca ttggaaaacg ttcttcgggg cgaaaactct 5520
   caaggatctt accgctgttg agatccagtt cgatgtaacc cactcgtgca cccaactgat 5580
   cttcagcatc ttttactttc accagcgttt ctgggtgagc aaaaacagga aggcaaaatg 5640
   ccgcaaaaaa gggaataagg gcgacacgga aatgttgaat actcatactc ttcctttttc 5700
   aatattattg aagcatttat cagggttatt gtctcatgag cggatacata tttgaatgta 5760
   tttagaaaaa taaacaaata ggggttccgc gcacatttcc ccgaaaagtg ccacctaaat 5820
   tgtaagcgtt aatattttgt taaaattcgc gttaaatttt tgttaaatca gctcattttt 5880
   taaccaatag gccgaaatcg gcaaaatccc ttataaatca aaagaataga ccgagatagg 5940
   gttgagtgtt gttccagttt ggaacaagag tccactatta aagaacgtgg actccaacgt 6000
   caaagggcga aaaaccgtct atcagggcga tggcccacta cgtgaaccat caccctaatc 6060
   aagttttttg gggtcgaggt gccgtaaagc actaaatcgg aaccctaaag ggagcccccg 6120
   atttagagct tgacggggaa agccggcgaa cgtggcgaga aaggaaggga agaaagcgaa 6180
   aggagcgggc gctagggcgc tggcaagtgt agcggtcacg ctgcgcgtaa ccaccacacc 6240
   cgccgcgctt aatgcgccgc tacagggcgc gtcccattcg ccattcaggc tgcgcaactg 6300
   ttgggaaggg cgatcggtgc gggcctcttc gctattacgc cagctggcga aagggggatg 6360
   tgctgcaagg cgattaagtt gggtaacgcc agggttttcc cagtcacgac 6410
<210> 35
   <211> 21
   <212> DNA
   <213> Unknown
<220>
   <223> Primer
<400> 35
   tctgactgac cgcgttactc c 21
<210> 36
   <211> 18
   <212> DNA
   <213> Unknown
<220>
   <223> Primer
<400> 36
   tgcttcatgt ggtcgggg 18
<210> 37
   <211> 20
   <212> DNA
   <213> Unknown
<220>
   <223> Primer
<400> 37
   atcggcgcag ctatttaccc 20
<210> 38
   <211> 20
   <212> DNA
   <213> Unknown
<220>
   <223> Primer
<400> 38
   cttgcgggtc atgcaccagg 20
<210> 39
   <211> 22
   <212> DNA
   <213> Unknown
<220>
   <223> Primer
<400> 39
   ttcttgtagt cggggatgtc gg 22
<210> 40
   <211> 18
   <212> DNA
   <213> Unknown
<220>
   <223> Primer
<400> 40
   ggagcgcaca aagccccg 18
<210> 41
   <211> 8508
   <212> DNA
   <213> Unknown
<220>
   <223> pX330 vector carrying the site-specific endonuclease target site of EGFP gene (Enhanced green fluorescent protein)
<400> 41
   gttttagagc tagaaatagc aagttaaaat aaggctagtc cgttatcaac ttgaaaaagt 60
   ggcaccgagt cggtgctttt ttgttttaga gctagaaata gcaagttaaa ataaggctag 120
   tccgttttta gcgcgtgcgc caattctgca gacaaatggc tctagaggta cccgttacat 180
   aacttacggt aaatggcccg cctggctgac cgcccaacga cccccgccca ttgacgtcaa 240
   tagtaacgcc aatagggact ttccattgac gtcaatgggt ggagtattta cggtaaactg 300
   cccacttggc agtacatcaa gtgtatcata tgccaagtac gccccctatt gacgtcaatg 360
   acggtaaatg gcccgcctgg cattgtgccc agtacatgac cttatgggac tttcctactt 420
   ggcagtacat ctacgtatta gtcatcgcta ttaccatggt cgaggtgagc cccacgttct 480
   gcttcactct ccccatctcc cccccctccc cacccccaat tttgtattta tttatttttt 540
   aattattttg tgcagcgatg ggggcggggg gggggggggg gcgcgcgcca ggcggggcgg 600
   ggcggggcga ggggcggggc ggggcgaggc ggagaggtgc ggcggcagcc aatcagagcg 660
   gcgcgctccg aaagtttcct tttatggcga ggcggcggcg gcggcggccc tataaaaagc 720
   gaagcgcgcg gcgggcggga gtcgctgcga cgctgccttc gccccgtgcc ccgctccgcc 780
   gccgcctcgc gccgcccgcc ccggctctga ctgaccgcgt tactcccaca ggtgagcggg 840
   cgggacggcc cttctcctcc gggctgtaat tagctgagca agaggtaagg gtttaaggga 900
   tggttggttg gtggggtatt aatgtttaat tacctggagc acctgcctga aatcactttt 960
   tttcaggttg gaccggtgcc accatggact ataaggacca cgacggagac tacaaggatc 1020
   atgatattga ttacaaagac gatgacgata agatggcccc aaagaagaag cggaaggtcg 1080
   gtatccacgg agtcccagca gccgacaaga agtacagcat cggcctggac atcggcacca 1140
   actctgtggg ctgggccgtg atcaccgacg agtacaaggt gcccagcaag aaattcaagg 1200
   tgctgggcaa caccgaccgg cacagcatca agaagaacct gatcggagcc ctgctgttcg 1260
   acagcggcga aacagccgag gccacccggc tgaagagaac cgccagaaga agatacacca 1320
   gacggaagaa ccggatctgc tatctgcaag agatcttcag caacgagatg gccaaggtgg 1380
   acgacagctt cttccacaga ctggaagagt ccttcctggt ggaagaggat aagaagcacg 1440
   agcggcaccc catcttcggc aacatcgtgg acgaggtggc ctaccacgag aagtacccca 1500
   ccatctacca cctgagaaag aaactggtgg acagcaccga caaggccgac ctgcggctga 1560
   tctatctggc cctggcccac atgatcaagt tccggggcca cttcctgatc gagggcgacc 1620
   tgaaccccga caacagcgac gtggacaagc tgttcatcca gctggtgcag acctacaacc 1680
   agctgttcga ggaaaacccc atcaacgcca gcggcgtgga cgccaaggcc atcctgtctg 1740
   ccagactgag caagagcaga cggctggaaa atctgatcgc ccagctgccc ggcgagaaga 1800
   agaatggcct gttcggaaac ctgattgccc tgagcctggg cctgaccccc aacttcaaga 1860
   gcaacttcga cctggccgag gatgccaaac tgcagctgag caaggacacc tacgacgacg 1920
   acctggacaa cctgctggcc cagatcggcg accagtacgc cgacctgttt ctggccgcca 1980
   agaacctgtc cgacgccatc ctgctgagcg acatcctgag agtgaacacc gagatcacca 2040
   aggcccccct gagcgcctct atgatcaaga gatacgacga gcaccaccag gacctgaccc 2100
   tgctgaaagc tctcgtgcgg cagcagctgc ctgagaagta caaagagatt ttcttcgacc 2160
   agagcaagaa cggctacgcc ggctacattg acggcggagc cagccaggaa gagttctaca 2220
   agttcatcaa gcccatcctg gaaaagatgg acggcaccga ggaactgctc gtgaagctga 2280
   acagagagga cctgctgcgg aagcagcgga ccttcgacaa cggcagcatc ccccaccaga 2340
   tccacctggg agagctgcac gccattctgc ggcggcagga agatttttac ccattcctga 2400
   aggacaaccg ggaaaagatc gagaagatcc tgaccttccg catcccctac tacgtgggcc 2460
   ctctggccag gggaaacagc agattcgcct ggatgaccag aaagagcgag gaaaccatca 2520
   ccccctggaa cttcgaggaa gtggtggaca agggcgcttc cgcccagagc ttcatcgagc 2580
   ggatgaccaa cttcgataag aacctgccca acgagaaggt gctgcccaag cacagcctgc 2640
   tgtacgagta cttcaccgtg tataacgagc tgaccaaagt gaaatacgtg accgagggaa 2700
   tgagaaagcc cgccttcctg agcggcgagc agaaaaaggc catcgtggac ctgctgttca 2760
   agaccaaccg gaaagtgacc gtgaagcagc tgaaagagga ctacttcaag aaaatcgagt 2820
   gcttcgactc cgtggaaatc tccggcgtgg aagatcggtt caacgcctcc ctgggcacat 2880
   accacgatct gctgaaaatt atcaaggaca aggacttcct ggacaatgag gaaaacgagg 2940
   acattctgga agatatcgtg ctgaccctga cactgtttga ggacagagag atgatcgagg 3000
   aacggctgaa aacctatgcc cacctgttcg acgacaaagt gatgaagcag ctgaagcggc 3060
   ggagatacac cggctggggc aggctgagcc ggaagctgat caacggcatc cgggacaagc 3120
   agtccggcaa gacaatcctg gatttcctga agtccgacgg cttcgccaac agaaacttca 3180
   tgcagctgat ccacgacgac agcctgacct ttaaagagga catccagaaa gcccaggtgt 3240
   ccggccaggg cgatagcctg cacgagcaca ttgccaatct ggccggcagc cccgccatta 3300
   agaagggcat cctgcagaca gtgaaggtgg tggacgagct cgtgaaagtg atgggccggc 3360
   acaagcccga gaacatcgtg atcgaaatgg ccagagagaa ccagaccacc cagaagggac 3420
   agaagaacag ccgcgagaga atgaagcgga tcgaagaggg catcaaagag ctgggcagcc 3480
   agatcctgaa agaacacccc gtggaaaaca cccagctgca gaacgagaag ctgtacctgt 3540
   actacctgca gaatgggcgg gatatgtacg tggaccagga actggacatc aaccggctgt 3600
   ccgactacga tgtggaccat atcgtgcctc agagctttct gaaggacgac tccatcgaca 3660
   acaaggtgct gaccagaagc gacaagaacc ggggcaagag cgacaacgtg ccctccgaag 3720
   aggtcgtgaa gaagatgaag aactactggc ggcagctgct gaacgccaag ctgattaccc 3780
   agagaaagtt cgacaatctg accaaggccg agagaggcgg cctgagcgaa ctggataagg 3840
   ccggcttcat caagagacag ctggtggaaa cccggcagat cacaaagcac gtggcacaga 3900
   tcctggactc ccggatgaac actaagtacg acgagaatga caagctgatc cgggaagtga 3960
   aagtgatcac cctgaagtcc aagctggtgt ccgatttccg gaaggatttc cagttttaca 4020
   aagtgcgcga gatcaacaac taccaccacg cccacgacgc ctacctgaac gccgtcgtgg 4080
   gaaccgccct gatcaaaaag taccctaagc tggaaagcga gttcgtgtac ggcgactaca 4140
   aggtgtacga cgtgcggaag atgatcgcca agagcgagca ggaaatcggc aaggctaccg 4200
   ccaagtactt cttctacagc aacatcatga actttttcaa gaccgagatt accctggcca 4260
   acggcgagat ccggaagcgg cctctgatcg agacaaacgg cgaaaccggg gagatcgtgt 4320
   gggataaggg ccgggatttt gccaccgtgc ggaaagtgct gagcatgccc caagtgaata 4380
   tcgtgaaaaa gaccgaggtg cagacaggcg gcttcagcaa agagtctatc ctgcccaaga 4440
   ggaacagcga taagctgatc gccagaaaga aggactggga ccctaagaag tacggcggct 4500
   tcgacagccc caccgtggcc tattctgtgc tggtggtggc caaagtggaa aagggcaagt 4560
   ccaagaaact gaagagtgtg aaagagctgc tggggatcac catcatggaa agaagcagct 4620
   tcgagaagaa tcccatcgac tttctggaag ccaagggcta caaagaagtg aaaaaggacc 4680
   tgatcatcaa gctgcctaag tactccctgt tcgagctgga aaacggccgg aagagaatgc 4740
   tggcctctgc cggcgaactg cagaagggaa acgaactggc cctgccctcc aaatatgtga 4800
   acttcctgta cctggccagc cactatgaga agctgaaggg ctcccccgag gataatgagc 4860
   agaaacagct gtttgtggaa cagcacaagc actacctgga cgagatcatc gagcagatca 4920
   gcgagttctc caagagagtg atcctggccg acgctaatct ggacaaagtg ctgtccgcct 4980
   acaacaagca ccgggataag cccatcagag agcaggccga gaatatcatc cacctgttta 5040
   ccctgaccaa tctgggagcc cctgccgcct tcaagtactt tgacaccacc atcgaccgga 5100
   agaggtacac cagcaccaaa gaggtgctgg acgccaccct gatccaccag agcatcaccg 5160
   gcctgtacga gacacggatc gacctgtctc agctgggagg cgacaaaagg ccggcggcca 5220
   cgaaaaaggc cggccaggca aaaaagaaaa agtaagaatt cctagagctc gctgatcagc 5280
   ctcgactgtg ccttctagtt gccagccatc tgttgtttgc ccctcccccg tgccttcctt 5340
   gaccctggaa ggtgccactc ccactgtcct ttcctaataa aatgaggaaa ttgcatcgca 5400
   ttgtctgagt aggtgtcatt ctattctggg gggtggggtg gggcaggaca gcaaggggga 5460
   ggattgggaa gagaatagca ggcatgctgg ggagcggccg caggaacccc tagtgatgga 5520
   gttggccact ccctctctgc gcgctcgctc gctcactgag gccgggcgac caaaggtcgc 5580
   ccgacgcccg ggctttgccc gggcggcctc agtgagcgag cgagcgcgca gctgcctgca 5640
   ggggcgcctg atgcggtatt ttctccttac gcatctgtgc ggtatttcac accgcatacg 5700
   tcaaagcaac catagtacgc gccctgtagc ggcgcattaa gcgcggcggg tgtggtggtt 5760
   acgcgcagcg tgaccgctac acttgccagc gccctagcgc ccgctccttt cgctttcttc 5820
   ccttcctttc tcgccacgtt cgccggcttt ccccgtcaag ctctaaatcg ggggctccct 5880
   ttagggttcc gatttagtgc tttacggcac ctcgacccca aaaaacttga tttgggtgat 5940
   ggttcacgta gtgggccatc gccctgatag acggtttttc gccctttgac gttggagtcc 6000
   acgttcttta atagtggact cttgttccaa actggaacaa cactcaaccc tatctcgggc 6060
   tattcttttg atttataagg gattttgccg atttcggcct attggttaaa aaatgagctg 6120
   atttaacaaa aatttaacgc gaattttaac aaaatattaa cgtttacaat tttatggtgc 6180
   actctcagta caatctgctc tgatgccgca tagttaagcc agccccgaca cccgccaaca 6240
   cccgctgacg cgccctgacg ggcttgtctg ctcccggcat ccgcttacag acaagctgtg 6300
   accgtctccg ggagctgcat gtgtcagagg ttttcaccgt catcaccgaa acgcgcgaga 6360
   cgaaagggcc tcgtgatacg cctattttta taggttaatg tcatgataat aatggtttct 6420
   tagacgtcag gtggcacttt tcggggaaat gtgcgcggaa cccctatttg tttatttttc 6480
   taaatacatt caaatatgta tccgctcatg agacaataac cctgataaat gcttcaataa 6540
   tattgaaaaa ggaagagtat gagtattcaa catttccgtg tcgcccttat tccctttttt 6600
   gcggcatttt gccttcctgt ttttgctcac ccagaaacgc tggtgaaagt aaaagatgct 6660
   gaagatcagt tgggtgcacg agtgggttac atcgaactgg atctcaacag cggtaagatc 6720
   cttgagagtt ttcgccccga agaacgtttt ccaatgatga gcacttttaa agttctgcta 6780
   tgtggcgcgg tattatcccg tattgacgcc gggcaagagc aactcggtcg ccgcatacac 6840
   tattctcaga atgacttggt tgagtactca ccagtcacag aaaagcatct tacggatggc 6900
   atgacagtaa gagaattatg cagtgctgcc ataaccatga gtgataacac tgcggccaac 6960
   ttacttctga caacgatcgg aggaccgaag gagctaaccg cttttttgca caacatgggg 7020
   gatcatgtaa ctcgccttga tcgttgggaa ccggagctga atgaagccat accaaacgac 7080
   gagcgtgaca ccacgatgcc tgtagcaatg gcaacaacgt tgcgcaaact attaactggc 7140
   gaactactta ctctagcttc ccggcaacaa ttaatagact ggatggaggc ggataaagtt 7200
   gcaggaccac ttctgcgctc ggcccttccg gctggctggt ttattgctga taaatctgga 7260
   gccggtgagc gtggaagccg cggtatcatt gcagcactgg ggccagatgg taagccctcc 7320
   cgtatcgtag ttatctacac gacggggagt caggcaacta tggatgaacg aaatagacag 7380
   atcgctgaga taggtgcctc actgattaag cattggtaac tgtcagacca agtttactca 7440
   tatatacttt agattgattt aaaacttcat ttttaattta aaaggatcta ggtgaagatc 7500
   ctttttgata atctcatgac caaaatccct taacgtgagt tttcgttcca ctgagcgtca     7560
   gaccccgtag aaaagatcaa aggatcttct tgagatcctt tttttctgcg cgtaatctgc     7620
   tgcttgcaaa caaaaaaacc accgctacca gcggtggttt gtttgccgga tcaagagcta     7680
   ccaactcttt ttccgaaggt aactggcttc agcagagcgc agataccaaa tactgtcctt     7740
   ctagtgtagc cgtagttagg ccaccacttc aagaactctg tagcaccgcc tacatacctc     7800
   gctctgctaa tcctgttacc agtggctgct gccagtggcg ataagtcgtg tcttaccggg     7860
   ttggactcaa gacgatagtt accggataag gcgcagcggt cgggctgaac ggggggttcg     7920
   tgcacacagc ccagcttgga gcgaacgacc tacaccgaac tgagatacct acagcgtgag     7980
   ctatgagaaa gcgccacgct tcccgaaggg agaaaggcgg acaggtatcc ggtaagcggc     8040
   agggtcggaa caggagagcg cacgagggag cttccagggg gaaacgcctg gtatctttat     8100
   agtcctgtcg ggtttcgcca cctctgactt gagcgtcgat ttttgtgatg ctcgtcaggg     8160
   gggcggagcc tatggaaaaa cgccagcaac gcggcctttt tacggttcct ggccttttgc     8220
   tggccttttg ctcacatgtg agggcctatt tcccatgatt ccttcatatt tgcatatacg     8280
   atacaaggct gttagagaga taattggaat taatttgact gtaaacacaa agatattagt     8340
   acaaaatacg tgacgtagaa agtaataatt tcttgggtag tttgcagttt taaaattatg     8400
   ttttaaaatg gactatcata tgcttaccgt aacttgaaag tatttcgatt tcttggcttt     8460
   atatatcttg tggaaaggac gaaacaccgg agcgcaccat cttcttca                  8508
<210> 42
   <211> 20
   <212> DNA
   <213> Unknown
<220>
   <223> Primer
<400> 42
   tgctcctaat gcagtgttgc 20
<210> 43
   <211> 22
   <212> DNA
   <213> Unknown
<220>
   <223> Primer
<400> 43
   catgctgtag gttctttgta gc 22
<210> 44
   <211> 23
   <212> DNA
   <213> Sus scrofa
<220>
   <223> V2cr3 site-specific endonuclease target site of V2G locus
<400> 44
   ggaggcattc agtaaatatc agg 23
<210> 45
   <211> 8508
   <212> DNA
   <213> Unknown
<220>
   <223> pX330 vector in which the (SEQ ID NO:44) S.scrofaV2cr3 sequence was cloned
<400> 45
   gttttagagc tagaaatagc aagttaaaat aaggctagtc cgttatcaac ttgaaaaagt 60
   ggcaccgagt cggtgctttt ttgttttaga gctagaaata gcaagttaaa ataaggctag 120
   tccgttttta gcgcgtgcgc caattctgca gacaaatggc tctagaggta cccgttacat 180
   aacttacggt aaatggcccg cctggctgac cgcccaacga cccccgccca ttgacgtcaa 240
   tagtaacgcc aatagggact ttccattgac gtcaatgggt ggagtattta cggtaaactg 300
   cccacttggc agtacatcaa gtgtatcata tgccaagtac gccccctatt gacgtcaatg 360
   acggtaaatg gcccgcctgg cattgtgccc agtacatgac cttatgggac tttcctactt 420
   ggcagtacat ctacgtatta gtcatcgcta ttaccatggt cgaggtgagc cccacgttct 480
   gcttcactct ccccatctcc cccccctccc cacccccaat tttgtattta tttatttttt 540
   aattattttg tgcagcgatg ggggcggggg gggggggggg gcgcgcgcca ggcggggcgg 600
   ggcggggcga ggggcggggc ggggcgaggc ggagaggtgc ggcggcagcc aatcagagcg 660
   gcgcgctccg aaagtttcct tttatggcga ggcggcggcg gcggcggccc tataaaaagc 720
   gaagcgcgcg gcgggcggga gtcgctgcga cgctgccttc gccccgtgcc ccgctccgcc 780
   gccgcctcgc gccgcccgcc ccggctctga ctgaccgcgt tactcccaca ggtgagcggg 840
   cgggacggcc cttctcctcc gggctgtaat tagctgagca agaggtaagg gtttaaggga 900
   tggttggttg gtggggtatt aatgtttaat tacctggagc acctgcctga aatcactttt 960
   tttcaggttg gaccggtgcc accatggact ataaggacca cgacggagac tacaaggatc 1020
   atgatattga ttacaaagac gatgacgata agatggcccc aaagaagaag cggaaggtcg 1080
   gtatccacgg agtcccagca gccgacaaga agtacagcat cggcctggac atcggcacca 1140
   actctgtggg ctgggccgtg atcaccgacg agtacaaggt gcccagcaag aaattcaagg 1200
   tgctgggcaa caccgaccgg cacagcatca agaagaacct gatcggagcc ctgctgttcg 1260
   acagcggcga aacagccgag gccacccggc tgaagagaac cgccagaaga agatacacca 1320
   gacggaagaa ccggatctgc tatctgcaag agatcttcag caacgagatg gccaaggtgg 1380
   acgacagctt cttccacaga ctggaagagt ccttcctggt ggaagaggat aagaagcacg 1440
   agcggcaccc catcttcggc aacatcgtgg acgaggtggc ctaccacgag aagtacccca 1500
   ccatctacca cctgagaaag aaactggtgg acagcaccga caaggccgac ctgcggctga 1560
   tctatctggc cctggcccac atgatcaagt tccggggcca cttcctgatc gagggcgacc 1620
   tgaaccccga caacagcgac gtggacaagc tgttcatcca gctggtgcag acctacaacc 1680
   agctgttcga ggaaaacccc atcaacgcca gcggcgtgga cgccaaggcc atcctgtctg 1740
   ccagactgag caagagcaga cggctggaaa atctgatcgc ccagctgccc ggcgagaaga 1800
   agaatggcct gttcggaaac ctgattgccc tgagcctggg cctgaccccc aacttcaaga 1860
   gcaacttcga cctggccgag gatgccaaac tgcagctgag caaggacacc tacgacgacg 1920
   acctggacaa cctgctggcc cagatcggcg accagtacgc cgacctgttt ctggccgcca 1980
   agaacctgtc cgacgccatc ctgctgagcg acatcctgag agtgaacacc gagatcacca 2040
   aggcccccct gagcgcctct atgatcaaga gatacgacga gcaccaccag gacctgaccc 2100
   tgctgaaagc tctcgtgcgg cagcagctgc ctgagaagta caaagagatt ttcttcgacc 2160
   agagcaagaa cggctacgcc ggctacattg acggcggagc cagccaggaa gagttctaca 2220
   agttcatcaa gcccatcctg gaaaagatgg acggcaccga ggaactgctc gtgaagctga 2280
   acagagagga cctgctgcgg aagcagcgga ccttcgacaa cggcagcatc ccccaccaga 2340
   tccacctggg agagctgcac gccattctgc ggcggcagga agatttttac ccattcctga 2400
   aggacaaccg ggaaaagatc gagaagatcc tgaccttccg catcccctac tacgtgggcc 2460
   ctctggccag gggaaacagc agattcgcct ggatgaccag aaagagcgag gaaaccatca 2520
   ccccctggaa cttcgaggaa gtggtggaca agggcgcttc cgcccagagc ttcatcgagc 2580
   ggatgaccaa cttcgataag aacctgccca acgagaaggt gctgcccaag cacagcctgc 2640
   tgtacgagta cttcaccgtg tataacgagc tgaccaaagt gaaatacgtg accgagggaa 2700
   tgagaaagcc cgccttcctg agcggcgagc agaaaaaggc catcgtggac ctgctgttca 2760
   agaccaaccg gaaagtgacc gtgaagcagc tgaaagagga ctacttcaag aaaatcgagt 2820
   gcttcgactc cgtggaaatc tccggcgtgg aagatcggtt caacgcctcc ctgggcacat 2880
   accacgatct gctgaaaatt atcaaggaca aggacttcct ggacaatgag gaaaacgagg 2940
   acattctgga agatatcgtg ctgaccctga cactgtttga ggacagagag atgatcgagg 3000
   aacggctgaa aacctatgcc cacctgttcg acgacaaagt gatgaagcag ctgaagcggc 3060
   ggagatacac cggctggggc aggctgagcc ggaagctgat caacggcatc cgggacaagc 3120
   agtccggcaa gacaatcctg gatttcctga agtccgacgg cttcgccaac agaaacttca 3180
   tgcagctgat ccacgacgac agcctgacct ttaaagagga catccagaaa gcccaggtgt 3240
   ccggccaggg cgatagcctg cacgagcaca ttgccaatct ggccggcagc cccgccatta 3300
   agaagggcat cctgcagaca gtgaaggtgg tggacgagct cgtgaaagtg atgggccggc 3360
   acaagcccga gaacatcgtg atcgaaatgg ccagagagaa ccagaccacc cagaagggac 3420
   agaagaacag ccgcgagaga atgaagcgga tcgaagaggg catcaaagag ctgggcagcc 3480
   agatcctgaa agaacacccc gtggaaaaca cccagctgca gaacgagaag ctgtacctgt 3540
   actacctgca gaatgggcgg gatatgtacg tggaccagga actggacatc aaccggctgt 3600
   ccgactacga tgtggaccat atcgtgcctc agagctttct gaaggacgac tccatcgaca 3660
   acaaggtgct gaccagaagc gacaagaacc ggggcaagag cgacaacgtg ccctccgaag 3720
   aggtcgtgaa gaagatgaag aactactggc ggcagctgct gaacgccaag ctgattaccc 3780
   agagaaagtt cgacaatctg accaaggccg agagaggcgg cctgagcgaa ctggataagg 3840
   ccggcttcat caagagacag ctggtggaaa cccggcagat cacaaagcac gtggcacaga 3900
   tcctggactc ccggatgaac actaagtacg acgagaatga caagctgatc cgggaagtga 3960
   aagtgatcac cctgaagtcc aagctggtgt ccgatttccg gaaggatttc cagttttaca 4020
   aagtgcgcga gatcaacaac taccaccacg cccacgacgc ctacctgaac gccgtcgtgg 4080
   gaaccgccct gatcaaaaag taccctaagc tggaaagcga gttcgtgtac ggcgactaca 4140
   aggtgtacga cgtgcggaag atgatcgcca agagcgagca ggaaatcggc aaggctaccg 4200
   ccaagtactt cttctacagc aacatcatga actttttcaa gaccgagatt accctggcca 4260
   acggcgagat ccggaagcgg cctctgatcg agacaaacgg cgaaaccggg gagatcgtgt 4320
   gggataaggg ccgggatttt gccaccgtgc ggaaagtgct gagcatgccc caagtgaata 4380
   tcgtgaaaaa gaccgaggtg cagacaggcg gcttcagcaa agagtctatc ctgcccaaga 4440
   ggaacagcga taagctgatc gccagaaaga aggactggga ccctaagaag tacggcggct 4500
   tcgacagccc caccgtggcc tattctgtgc tggtggtggc caaagtggaa aagggcaagt 4560
   ccaagaaact gaagagtgtg aaagagctgc tggggatcac catcatggaa agaagcagct 4620
   tcgagaagaa tcccatcgac tttctggaag ccaagggcta caaagaagtg aaaaaggacc 4680
   tgatcatcaa gctgcctaag tactccctgt tcgagctgga aaacggccgg aagagaatgc 4740
   tggcctctgc cggcgaactg cagaagggaa acgaactggc cctgccctcc aaatatgtga 4800
   acttcctgta cctggccagc cactatgaga agctgaaggg ctcccccgag gataatgagc 4860
   agaaacagct gtttgtggaa cagcacaagc actacctgga cgagatcatc gagcagatca 4920
   gcgagttctc caagagagtg atcctggccg acgctaatct ggacaaagtg ctgtccgcct 4980
   acaacaagca ccgggataag cccatcagag agcaggccga gaatatcatc cacctgttta 5040
   ccctgaccaa tctgggagcc cctgccgcct tcaagtactt tgacaccacc atcgaccgga 5100
   agaggtacac cagcaccaaa gaggtgctgg acgccaccct gatccaccag agcatcaccg 5160
   gcctgtacga gacacggatc gacctgtctc agctgggagg cgacaaaagg ccggcggcca 5220
   cgaaaaaggc cggccaggca aaaaagaaaa agtaagaatt cctagagctc gctgatcagc 5280
   ctcgactgtg ccttctagtt gccagccatc tgttgtttgc ccctcccccg tgccttcctt 5340
   gaccctggaa ggtgccactc ccactgtcct ttcctaataa aatgaggaaa ttgcatcgca 5400
   ttgtctgagt aggtgtcatt ctattctggg gggtggggtg gggcaggaca gcaaggggga 5460
   ggattgggaa gagaatagca ggcatgctgg ggagcggccg caggaacccc tagtgatgga 5520
   gttggccact ccctctctgc gcgctcgctc gctcactgag gccgggcgac caaaggtcgc 5580
   ccgacgcccg ggctttgccc gggcggcctc agtgagcgag cgagcgcgca gctgcctgca 5640
   ggggcgcctg atgcggtatt ttctccttac gcatctgtgc ggtatttcac accgcatacg 5700
   tcaaagcaac catagtacgc gccctgtagc ggcgcattaa gcgcggcggg tgtggtggtt 5760
   acgcgcagcg tgaccgctac acttgccagc gccctagcgc ccgctccttt cgctttcttc 5820
   ccttcctttc tcgccacgtt cgccggcttt ccccgtcaag ctctaaatcg ggggctccct 5880
   ttagggttcc gatttagtgc tttacggcac ctcgacccca aaaaacttga tttgggtgat 5940
   ggttcacgta gtgggccatc gccctgatag acggtttttc gccctttgac gttggagtcc 6000
   acgttcttta atagtggact cttgttccaa actggaacaa cactcaaccc tatctcgggc 6060
   tattcttttg atttataagg gattttgccg atttcggcct attggttaaa aaatgagctg 6120
   atttaacaaa aatttaacgc gaattttaac aaaatattaa cgtttacaat tttatggtgc 6180
   actctcagta caatctgctc tgatgccgca tagttaagcc agccccgaca cccgccaaca 6240
   cccgctgacg cgccctgacg ggcttgtctg ctcccggcat ccgcttacag acaagctgtg 6300
   accgtctccg ggagctgcat gtgtcagagg ttttcaccgt catcaccgaa acgcgcgaga 6360
   cgaaagggcc tcgtgatacg cctattttta taggttaatg tcatgataat aatggtttct 6420
   tagacgtcag gtggcacttt tcggggaaat gtgcgcggaa cccctatttg tttatttttc 6480
   taaatacatt caaatatgta tccgctcatg agacaataac cctgataaat gcttcaataa 6540
   tattgaaaaa ggaagagtat gagtattcaa catttccgtg tcgcccttat tccctttttt 6600
   gcggcatttt gccttcctgt ttttgctcac ccagaaacgc tggtgaaagt aaaagatgct 6660
   gaagatcagt tgggtgcacg agtgggttac atcgaactgg atctcaacag cggtaagatc 6720
   cttgagagtt ttcgccccga agaacgtttt ccaatgatga gcacttttaa agttctgcta 6780
   tgtggcgcgg tattatcccg tattgacgcc gggcaagagc aactcggtcg ccgcatacac 6840
   tattctcaga atgacttggt tgagtactca ccagtcacag aaaagcatct tacggatggc 6900
   atgacagtaa gagaattatg cagtgctgcc ataaccatga gtgataacac tgcggccaac 6960
   ttacttctga caacgatcgg aggaccgaag gagctaaccg cttttttgca caacatgggg 7020
   gatcatgtaa ctcgccttga tcgttgggaa ccggagctga atgaagccat accaaacgac 7080
   gagcgtgaca ccacgatgcc tgtagcaatg gcaacaacgt tgcgcaaact attaactggc 7140
   gaactactta ctctagcttc ccggcaacaa ttaatagact ggatggaggc ggataaagtt 7200
   gcaggaccac ttctgcgctc ggcccttccg gctggctggt ttattgctga taaatctgga 7260
   gccggtgagc gtggaagccg cggtatcatt gcagcactgg ggccagatgg taagccctcc 7320
   cgtatcgtag ttatctacac gacggggagt caggcaacta tggatgaacg aaatagacag 7380
   atcgctgaga taggtgcctc actgattaag cattggtaac tgtcagacca agtttactca 7440
   tatatacttt agattgattt aaaacttcat ttttaattta aaaggatcta ggtgaagatc 7500
   ctttttgata atctcatgac caaaatccct taacgtgagt tttcgttcca ctgagcgtca 7560
   gaccccgtag aaaagatcaa aggatcttct tgagatcctt tttttctgcg cgtaatctgc 7620
   tgcttgcaaa caaaaaaacc accgctacca gcggtggttt gtttgccgga tcaagagcta 7680
   ccaactcttt ttccgaaggt aactggcttc agcagagcgc agataccaaa tactgtcctt 7740
   ctagtgtagc cgtagttagg ccaccacttc aagaactctg tagcaccgcc tacatacctc 7800
   gctctgctaa tcctgttacc agtggctgct gccagtggcg ataagtcgtg tcttaccggg 7860
   ttggactcaa gacgatagtt accggataag gcgcagcggt cgggctgaac ggggggttcg 7920
   tgcacacagc ccagcttgga gcgaacgacc tacaccgaac tgagatacct acagcgtgag 7980
   ctatgagaaa gcgccacgct tcccgaaggg agaaaggcgg acaggtatcc ggtaagcggc 8040
   agggtcggaa caggagagcg cacgagggag cttccagggg gaaacgcctg gtatctttat 8100
   agtcctgtcg ggtttcgcca cctctgactt gagcgtcgat ttttgtgatg ctcgtcaggg 8160
   gggcggagcc tatggaaaaa cgccagcaac gcggcctttt tacggttcct ggccttttgc 8220
   tggccttttg ctcacatgtg agggcctatt tcccatgatt ccttcatatt tgcatatacg 8280
   atacaaggct gttagagaga taattggaat taatttgact gtaaacacaa agatattagt 8340
   acaaaatacg tgacgtagaa agtaataatt tcttgggtag tttgcagttt taaaattatg 8400
   ttttaaaatg gactatcata tgcttaccgt aacttgaaag tatttcgatt tcttggcttt 8460
   atatatcttg tggaaaggac gaaacaccgg aggcattcag taaatatc 8508
<210> 46
   <211> 120
   <212> DNA
   <213> Sus scrofa
<220>
   <223> /note="lox2271 and lox5171 sites separated by Pstl site and flanked by 23bp Sus scrofa arms of V2G locus" /note="Primer"
<400> 46
   tcctcttcac ggtaatacct gatataactt cgtataaagt atcctatacg aagttatctg 60
   cagataactt cgtataatgt gtactatacg aagttatatt tactgaatgc ctccaacatt 120
<210> 47
   <211> 331
   <212> DNA
   <213> Sus scrofa
<220>
   <223> V2G genomic sequence of clone 1B2 upon CRISPR-targeting.
<400> 47
   gtatatagat gagctgtgga gcagggaagg gctttgctgg actccagctg ctgctctctg 60
   atgcagcagg aatctctagt cccatcacgg cgcctgcatc ctcagaacat tgacagaact 120
   ctgcctggaa tgttggaggc attcagtaaa tataacttcg tatagtacac attatacgaa 180
   gttatctgca gataccaggc agagttctgt caatgttctg atacacttgt tcatctactt 240
   ttctcctcac aatactcatt tgccgtggct gacattatat ctgttttaca gtccagctga 300
   atttcagagc aattaaataa atctgtgcaa a 331
<210> 48
   <211> 23
   <212> DNA
   <213> Sus scrofa
<220>
   <223> V2cr1 site-specific endonuclease target site of V2G locus
<400> 48
   gccactacat tggtactctc tgg 23
<210> 49
   <211> 2302
   <212> DNA
   <213> Sus scrofa
<220>
   <223> /note="v10.2 Reference genomeV2G locus" /note="Sus scrofa breed mixed chromosome 9, Sus scrofa 10.2 sequence ID: ref|NC_010451.3| gi:347618785 (bp 22916605 - 22918906)"
<400> 49
   ctcctttgac aaggtctagt ggatggaaaa gagggactct gggcctaggc agggtcattt       60
   atcttttggt cactgatttt tttttccttc actcagtgtt ggaggggaag gtccagaatc      120
   agaggacttg gattctgtct ctgtcctgct gccgtctagg acagagacag aagtcagttg      180
   accttaaaca agtcagttga cctctgtgag cactgttgat cttaaacaag tcagttgacc      240
   tctgtgagca ctgttccctc cctaattcag cattcagtat ctaccgaaga cgaactctgc      300
   tccaaacgct gagttaggct ctgggggagt agaaacatac aagggatatg gctcttgtct      360
   gcaaatggcc cttcgtccgc aggaggaaac agggctggtt tcacgtggtc agtcagggag      420
   gggccaagca aacctctggg aggctcctgg gccccagtcc gagatgactt cagttcccgg 480
   tgggtggtgg gccctgaggg tgtggccggg gtgaggcagt cctccactca gaggggctgc 540
   agaaattagc ccagctgtgt gatcttggac tagtcactga gcttctctga gcctctttcc 600
   tcctcccact tccccgaata gtcaagccct gtcttgtggt gcctttgtgg gcaagccgac 660
   tccttcatct ggcactcact ctggtgtgtt tcagtggttg tctgcctctt tccctgggct 720
   ctcagcagcg tgaaggctgg gctatgtcaa tcttattcac aggggtctcc cctgctccta 780
   atgcagtgtt gcatttagta ggtgctcagt taatgtctga tgaaggattg agtatataga 840
   tgagctgtgg agcagggaag ggctttgctg gactccagct gctgctctct gatgcagcag 900
   gaatctctag tcccatcacg gcgcctgcat cctcttcacg gtaatacctg atatttactg 960
   aatgcctcca acattccagg cagagttctg tcaatgttct gatacacttg ttcatctact 1020
   tttctcctca caatactcat ttgccgtggc tgacattata tctgttttac agtccagctg 1080
   aatttcagag caattaaata aatctgtgca aagtcataca gtaaagtgac agaacaagat 1140
   tccagttcag gtgtcctgac tccaaagcgt tttgcttccc tctgtgcaag ctgcatccag 1200
   ggctctcaga gctgcctgga cccagagagt accaatgtag tggctacaaa gaacctacag 1260
   catggcatgc aaagacctgt agccagaggg cgctcaagcg cgctcccttg gctgccctgg 1320
   agtcagtggg gatggagaag cagtcttggc agagtctggg gccaggatac taatgagctg 1380
   gtgcgcgtgg gtgctcactt ttccccctgg cgtcagtcag cctccttatg gggacgactg 1440
   tcatcccctc tctctttcaa ccacatcgac aagccccttc tctctccttg acccctgagg 1500
   agggattgtg cccggcagca tccccgcttc ctgtccgtgc atcccatagg gagtggaagc 1560
   cagaggatga aaagcgcagg gttttttccc ttctcttcca aggaatccac cagatgccac 1620
   actggctggg gaaccggcag tgcctcttct actctactca gcctgcaggg tggccgagct 1680
   ttgtctccca tgttcatgat tccacaactt tctggggcac ctctcttttc acatcctctc 1740
   ccacagcctc ctgtggtctc agcctcccac tcccctctcc tcaccccgcc agtcctccct 1800
   tctttttctc tttggcggag ctgtagaagg gcccctgggg tcaaggaagt gcagagacta 1860
   atagcctctt agcataactc ctccggagtc atgcccaact ggcctctgct tttgcatacc 1920
   tcacgttaca ttgaaaaagc ctccctctgt ttccacaact tggtgtgaag ggcctcagaa 1980
   ggaggatggt tgtgttctgt gcccccaaga aagagctggg ggaaaaatga agcctcctgc 2040
   catctattga tcaaccccct tctctgtaca tacactctag aaagatgata aaggcacgac 2100
   cctgtcctta tgacacgctc cgctcctaag acactaggga cctgacctag gagacaattg 2160
   taaaccccag ttcccatgga agtcagcctc gagagaacct gaatttcata tgcaaggcat 2220
   gatgaaagca cagaggaagg agagaataat tctgagagca aatttggaga ggctttaagg 2280
   agaagaaggc tttgtgccag gc 2302
<210> 50
   <211> 23
   <212> DNA
   <213> Sus scrofa
<220>
   <223> /note="V2cr5" /note="site-specific endonuclease target site of V2G locus"
<400> 50
   tatcaggtat taccgtgaag agg 23
<210> 51
   <211> 23
   <212> DNA
   <213> Sus scrofa
<220>
   <223> /note="V2cr6" /note="site-specific endonuclease target site of V2G locus"
<400> 51
   cacggcaaat gagtattgtg agg 23
<210> 52
   <211> 23
   <212> DNA
   <213> Sus scrofa
<220>
   <223> /note="V2cr7 " /note="site-specific endonuclease target site of V2G locus"
<400> 52
   ctcacaatac tcatttgccg tgg 23
<210> 53
   <211> 23
   <212> DNA
   <213> Sus scrofa
<220>
   <223> /note="V2cr8 " /note="site-specific endonuclease target site of V2G locus"
<400> 53
   gaggatgcag gcgccgtgat ggg 23
<210> 54
   <211> 20
   <212> DNA
   <213> Sus scrofa
<220>
   <223> Primer
<220>
   <223> /note="pCAGGS5â\200\231RV" /note="primer targeting CMV enhancer of pV2G-TEP vector. Assay Left arm PCR screening to verify Homologous Recombination into V2G"
<400> 54
   cctattggcg ttactatggg 20
<210> 55
   <211> 20
   <212> DNA
   <213> Sus scrofa
<220>
   <223> Primer
<220>
   <223> /note="Puro2-fw " /note="primer targeting PAC (PuroR) gene of pV2G-TEP vector. Assay: Right arm PCR screening to verify Homologous Recombination into V2G"
<400> 55
   atcggcaagg tgtgggtcgc 20
<210> 56
   <211> 578
   <212> DNA
   <213> Sus scrofa
<220>
   <223> Left arm homology sequence of V2G locus cloned into pV2G-TEP vector
<400> 56
   ctaccgaaga cgaactctgc tccaaacgct gagttaggct ctgggggagt agaaacatac 60
   aagggatatg gctcttgtct gcaaatggcc cttcgtccgc aggaggaaac agggctggtt 120
   tcacgtggtc agtcagggag gggccaagca aacctctggg aggctcctgg gccccagtcc 180
   gagatgactt cagttcccgg tgggtggtgg gccctgaggg tgtggccggg gtgaggcagt 240
   cctccactca gaggggctgc agaaattagc ccagctgtgt gatcttggac tagtcactga 300
   gcttctctga gcctctttcc tcctcccact tccccgaata gtcaagccct gtcttgtggt 360
   gcctttgtgg gcaagccgac tccttcatct ggcactcact ctggtgtgtt tcagtggttg 420
   tctgcctctt tccctgggct ctcagcagcg tgaaggctgg gctatgtcaa tcttattcac 480
   aggggtctcc cctgctccta atgcagtgtt gcatttagta ggtgctcagt taatgtctga 540
   tgaaggattg agtatataga tgagctgtgg agcaggga 578
<210> 57
   <211> 1181
   <212> DNA
   <213> Sus scrofa
<220>
   <223> Right arm homology sequence of V2G locus cloned into pV2G-TEP vector
<400> 57
   tatctgtttt acagtccagc tgaatttcag agcaattaaa taaatctgtg caaagtcata      60
   cagtaaagtg acagaacaag attccagttc aggtgtcctg actccaaagc gttttgcttc     120
   cctctgtgca agctgcatcc agggctctca gagctgcctg gacccagaga gtaccaatgt     180
   agtggctaca aagaacctac agcatggcat gcaaagacct gtagccagag ggcgctcaag     240
   cgcgctccct tggctgccct ggagtcagtg gggatggaga agcagtcttg gcagagtctg     300
   gggccaggat actaatgagc tggtgcgcgt gggtgctcac ttttccccct ggcgtcagtc     360
   agcctcctta tggggacgac tgtcatcccc tctctctttc aaccacatcg acaagcccct     420
   tctctctcct tgacccctga ggagggattg tgcccggcag catccccgct tcctgtccgt     480
   gcatcccata gggagtggaa gccagaggat gaaaagcgca gggttttttc ccttctcttc     540
   caaggaatcc accagatgcc acactggctg gggaaccggc agtgcctctt ctactctact     600
   cagcctgcag ggtggccgag ctttgtctcc catgttcatg attccacaac tttctggggc     660
   acctctcttt tcacatcctc tcccacagcc tcctgtggtc tcagcctccc actcccctct     720
   cctcaccccg ccagtcctcc cttctttttc tctttggcgg agctgtagaa gggcccctgg     780
   ggtcaaggaa gtgcagagac taatagcctc ttagcataac tcctccggag tcatgcccaa     840
   ctggcctctg cttttgcata cctcacgtta cattgaaaaa gcctccctct gtttccacaa     900
   cttggtgtga agggcctcag aaggaggatg gttgtgttct gtgcccccaa gaaagagctg     960
   ggggaaaaat gaagcctcct gccatctatt gatcaacccc cttctctgta catacactct    1020
   agaaagatga taaaggcacg accctgtcct tatgacacgc tccgctccta agacactagg    1080
   gacctgacct aggagacaat tgtaaacccc agttcccatg gaagtcagcc tcgagagaac    1140
   ctgaatttca tatgcaaggc atgatgaaag cacagaggaa g                        1181
<210> 58
   <211> 10682
   <212> DNA
   <213> Sus scrofa
<220>
   <223> pV2G-TEP targeting vector
<400> 58
   ctagcgcggc cgcgaagttc ctatactttc tagagaatag gaacttctat ctgttttaca      60
   gtccagctga atttcagagc aattaaataa atctgtgcaa agtcatacag taaagtgaca     120
   gaacaagatt ccagttcagg tgtcctgact ccaaagcgtt ttgcttccct ctgtgcaagc     180
   tgcatccagg gctctcagag ctgcctggac ccagagagta ccaatgtagt ggctacaaag     240
   aacctacagc atggcatgca aagacctgta gccagagggc gctcaagcgc gctcccttgg 300
   ctgccctgga gtcagtgggg atggagaagc agtcttggca gagtctgggg ccaggatact 360
   aatgagctgg tgcgcgtggg tgctcacttt tccccctggc gtcagtcagc ctccttatgg 420
   ggacgactgt catcccctct ctctttcaac cacatcgaca agccccttct ctctccttga 480
   cccctgagga gggattgtgc ccggcagcat ccccgcttcc tgtccgtgca tcccataggg 540
   agtggaagcc agaggatgaa aagcgcaggg ttttttccct tctcttccaa ggaatccacc 600
   agatgccaca ctggctgggg aaccggcagt gcctcttcta ctctactcag cctgcagggt 660
   ggccgagctt tgtctcccat gttcatgatt ccacaacttt ctggggcacc tctcttttca 720
   catcctctcc cacagcctcc tgtggtctca gcctcccact cccctctcct caccccgcca 780
   gtcctccctt ctttttctct ttggcggagc tgtagaaggg cccctggggt caaggaagtg 840
   cagagactaa tagcctctta gcataactcc tccggagtca tgcccaactg gcctctgctt 900
   ttgcatacct cacgttacat tgaaaaagcc tccctctgtt tccacaactt ggtgtgaagg 960
   gcctcagaag gaggatggtt gtgttctgtg cccccaagaa agagctgggg gaaaaatgaa 1020
   gcctcctgcc atctattgat caaccccctt ctctgtacat acactctaga aagatgataa 1080
   aggcacgacc ctgtccttat gacacgctcc gctcctaaga cactagggac ctgacctagg 1140
   agacaattgt aaaccccagt tcccatggaa gtcagcctcg agagaacctg aatttcatat 1200
   gcaaggcatg atgaaagcac agaggaagaa gggcgaattc tgcagatatc catcacactg 1260
   gcggccgctc gagcatgcat ctagagggcc caattcgccc tatagtgagt cgtattacaa 1320
   ttcactggcc gtcgttttac aacgtcgtga ctgggaaaac cctggcgtta cccaacttaa 1380
   tcgccttgca gcacatcccc ctttcgccag ctggcgtaat agcgaagagg cccgcaccga 1440
   tcgcccttcc caacagttgc gcagcctgaa tggcgaatgg acgcgccctg tagcggcgca 1500
   ttaagcgcgg cgggtgtggt ggttacgcgc agcgtgaccg ctacacttgc cagcgcccta 1560
   gcgcccgctc ctttcgcttt cttcccttcc tttctcgcca cgttcgccgg ctttccccgt 1620
   caagctctaa atcgggggct ccctttaggg ttccgattta gtgctttacg gcacctcgac 1680
   cccaaaaaac ttgattaggg tgatggttca cgtagtgggc catcgccctg atagacggtt 1740
   tttcgccctt tgacgttgga gtccacgttc tttaatagtg gactcttgtt ccaaactgga 1800
   acaacactca accctatctc ggtctattct tttgatttat aagggatttt gccgatttcg 1860
   gcctattggt taaaaaatga gctgatttaa caaaaattta acgcgaattt taacaaaatt 1920
   cagggcgcaa gggctgctaa aggaagcgga acacgtagaa agccagtccg cagaaacggt 1980
   gctgaccccg gatgaatgtc agctactggg ctatctggac aagggaaaac gcaagcgcaa 2040
   agagaaagca ggtagcttgc agtgggctta catggcgata gctagactgg gcggttttat 2100
   ggacagcaag cgaaccggaa ttgccagctg gggcgccctc tggtaaggtt gggaagccct 2160
   gcaaagtaaa ctggatggct ttcttgccgc caaggatctg atggcgcagg ggatcaagat 2220
   ctgatcaaga gacaggatga ggatcgtttc gcatgattga acaagatgga ttgcacgcag 2280
   gttctccggc cgcttgggtg gagaggctat tcggctatga ctgggcacaa cagacaatcg 2340
   gctgctctga tgccgccgtg ttccggctgt cagcgcaggg gcgcccggtt ctttttgtca 2400
   agaccgacct gtccggtgcc ctgaatgaac tgcaggacga ggcagcgcgg ctatcgtggc 2460
   tggccacgac gggcgttcct tgcgcagctg tgctcgacgt tgtcactgaa gcgggaaggg 2520
   actggctgct attgggcgaa gtgccggggc aggatctcct gtcatcccac cttgctcctg 2580
   ccgagaaagt atccatcatg gctgatgcaa tgcggcggct gcatacgctt gatccggcta 2640
   cctgcccatt cgaccaccaa gcgaaacatc gcatcgagcg agcacgtact cggatggaag 2700
   ccggtcttgt cgatcaggat gatctggacg aagagcatca ggggctcgcg ccagccgaac 2760
   tgttcgccag gctcaaggcg cgcatgcccg acggcgagga tctcgtcgtg acccatggcg 2820
   atgcctgctt gccgaatatc atggtggaaa atggccgctt ttctggattc atcgactgtg 2880
   gccggctggg tgtggcggac cgctatcagg acatagcgtt ggctacccgt gatattgctg 2940
   aagagcttgg cggcgaatgg gctgaccgct tcctcgtgct ttacggtatc gccgctcccg 3000
   attcgcagcg catcgccttc tatcgccttc ttgacgagtt cttctgaatt gaaaaaggaa 3060
   gagtatgagt attcaacatt tccgtgtcgc ccttattccc ttttttgcgg cattttgcct 3120
   tcctgttttt gctcacccag aaacgctggt gaaagtaaaa gatgctgaag atcagttggg 3180
   tgcacgagtg ggttacatcg aactggatct caacagcggt aagatccttg agagttttcg 3240
   ccccgaagaa cgttttccaa tgatgagcac ttttaaagtt ctgctatgtg gcgcggtatt 3300
   atcccgtatt gacgccgggc aagagcaact cggtcgccgc atacactatt ctcagaatga 3360
   cttggttgag tactcaccag tcacagaaaa gcatcttacg gatggcatga cagtaagaga 3420
   attatgcagt gctgccataa ccatgagtga taacactgcg gccaacttac ttctgacaac 3480
   gatcggagga ccgaaggagc taaccgcttt tttgcacaac atgggggatc atgtaactcg 3540
   ccttgatcgt tgggaaccgg agctgaatga agccatacca aacgacgagc gtgacaccac 3600
   gatgcctgta gcaatggcaa caacgttgcg caaactatta actggcgaac tacttactct 3660
   agcttcccgg caacaattaa tagactggat ggaggcggat aaagttgcag gaccacttct 3720
   gcgctcggcc cttccggctg gctggtttat tgctgataaa tctggagccg gtgagcgtgg 3780
   gtctcgcggt atcattgcag cactggggcc agatggtaag ccctcccgta tcgtagttat 3840
   ctacacgacg gggagtcagg caactatgga tgaacgaaat agacagatcg ctgagatagg 3900
   tgcctcactg attaagcatt ggtaactgtc agaccaagtt tactcatata tactttagat 3960
   tgatttaaaa cttcattttt aatttaaaag gatctaggtg aagatccttt ttgataatct 4020
   catgaccaaa atcccttaac gtgagttttc gttccactga gcgtcagacc ccgtagaaaa 4080
   gatcaaagga tcttcttgag atcctttttt tctgcgcgta atctgctgct tgcaaacaaa 4140
   aaaaccaccg ctaccagcgg tggtttgttt gccggatcaa gagctaccaa ctctttttcc 4200
   gaaggtaact ggcttcagca gagcgcagat accaaatact gttcttctag tgtagccgta 4260
   gttaggccac cacttcaaga actctgtagc accgcctaca tacctcgctc tgctaatcct 4320
   gttaccagtg gctgctgcca gtggcgataa gtcgtgtctt accgggttgg actcaagacg 4380
   atagttaccg gataaggcgc agcggtcggg ctgaacgggg ggttcgtgca cacagcccag 4440
   cttggagcga acgacctaca ccgaactgag atacctacag cgtgagctat gagaaagcgc 4500
   cacgcttccc gaagggagaa aggcggacag gtatccggta agcggcaggg tcggaacagg 4560
   agagcgcacg agggagcttc cagggggaaa cgcctggtat ctttatagtc ctgtcgggtt 4620
   tcgccacctc tgacttgagc gtcgattttt gtgatgctcg tcaggggggc ggagcctatg 4680
   gaaaaacgcc agcaacgcgg cctttttacg gttcctggcc ttttgctggc cttttgctca 4740
   catgttcttt cctgcgttat cccctgattc tgtggataac cgtattaccg cctttgagtg 4800
   agctgatacc gctcgccgca gccgaacgac cgagcgcagc gagtcagtga gcgaggaagc 4860
   ggaagagcgc ccaatacgca aaccgcctct ccccgcgcgt tggccgattc attaatgcag 4920
   ctggcacgac aggtttcccg actggaaagc gggcagtgag cgcaacgcaa ttaatgtgag 4980
   ttagctcact cattaggcac cccaggcttt acactttatg cttccggctc gtatgttgtg 5040
   tggaattgtg agcggataac aatttcacac aggaaacagc tatgaccatg attacgccaa 5100
   gcttggtacc gagctcggat ccactagtaa cggccgccag tgtgctggaa ttcgcccttc 5160
   taccgaagac gaactctgct ccaaacgctg agttaggctc tgggggagta gaaacataca 5220
   agggatatgg ctcttgtctg caaatggccc ttcgtccgca ggaggaaaca gggctggttt 5280
   cacgtggtca gtcagggagg ggccaagcaa acctctggga ggctcctggg ccccagtccg 5340
   agatgacttc agttcccggt gggtggtggg ccctgagggt gtggccgggg tgaggcagtc 5400
   ctccactcag aggggctgca gaaattagcc cagctgtgtg atcttggact agtcactgag 5460
   cttctctgag cctctttcct cctcccactt ccccgaatag tcaagccctg tcttgtggtg 5520
   cctttgtggg caagccgact ccttcatctg gcactcactc tggtgtgttt cagtggttgt 5580
   ctgcctcttt ccctgggctc tcagcagcgt gaaggctggg ctatgtcaat cttattcaca 5640
   ggggtctccc ctgctcctaa tgcagtgttg catttagtag gtgctcagtt aatgtctgat 5700
   gaaggattga gtatatagat gagctgtgga gcagggaggg tgccagggcg tgcccttggg 5760
   ctccccgggc gcgtaataac ttcgtataaa gtatcctata cgaagttata tgcgcatgaa 5820
   ttcggtaccg cggccgcgtc gacattgatt attgactagt tattaatagt aatcaattac 5880
   ggggtcatta gttcatagcc catatatgga gttccgcgtt acataactta cggtaaatgg 5940
   cccgcctggc tgaccgccca acgacccccg cccattgacg tcaataatga cgtatgttcc 6000
   catagtaacg ccaataggga ctttccattg acgtcaatgg gtggagtatt tacggtaaac 6060
   tgcccacttg gcagtacatc aagtgtatca tatgccaagt acgcccccta ttgacgtcaa 6120
   tgacggtaaa tggcccgcct ggcattatgc ccagtacatg accttatggg actttcctac 6180
   ttggcagtac atctacgtat tagtcatcgc tattaccatg gtcgaggtga gccccacgtt 6240
   ctgcttcact ctccccatct cccccccctc cccaccccca attttgtatt tatttatttt 6300
   ttaattattt tgtgcagcga tgggggcggg gggggggggg gggcgcgcgc caggcggggc 6360
   ggggcggggc gaggggcggg gcggggcgag gcggagaggt gcggcggcag ccaatcagag 6420
   cggcgcgctc cgaaagtttc cttttatggc gaggcggcgg cggcggcggc cctataaaaa 6480
   gcgaagcgcg cggcgggcgg gagtcgctgc gcgctgcctt cgccccgtgc cccgctccgc 6540
   gccgcctcgc gccgcccgcc ccggctctga ctgaccgcgt tactcccaca ggtgagcggg 6600
   cgggacggcc cttctcctcc gggctgtaat tagcgcttgg tttaatgacg gcttgtttct 6660
   tttctgtggc tgcgtgaaag ccttgagggg ctccgggagg gccctttgtg cggggggagc 6720
   ggctcggggg gtgcgtgcgt gtgtgtgtgc gtggggagcg ccgcgtgcgg ctccgcgctg 6780
   cccggcggct gtgagcgctg cgggcgcggc gcggggcttt gtgcgctccg cagtgtgcgc 6840
   gaggggagcg cggccggggg cggtgccccg cggtgcgggg ggggctgcga ggggaacaaa 6900
   ggctgcgtgc ggggtgtgtg cgtggggggg tgagcagggg gtgtgggcgc gtcggtcggg 6960
   ctgcaacccc ccctgcaccc ccctccccga gttgctgagc acggcccggc ttcgggtgcg 7020
   gggctccgta cggggcgtgg cgcggggctc gccgtgccgg gcggggggtg gcggcaggtg 7080
   ggggtgccgg gcggggcggg gccgcctcgg gccggggagg gctcggggga ggggcgcggc 7140
   ggcccccgga gcgccggcgg ctgtcgaggc gcggcgagcc gcagccattg ccttttatgg 7200
   taatcgtgcg agagggcgca gggacttcct ttgtcccaaa tctgtgcgga gccgaaatct 7260
   gggaggcgcc gccgcacccc ctctagcggg cgcggggcga agcggtgcgg cgccggcagg 7320
   aaggaaatgg gcggggaggg ccttcgtgcg tcgccgcgcc gccgtcccct tctccctctc 7380
   cagcctcggg gctgtccgcg gggggacggc tgccttcggg ggggacgggg cagggcgggg 7440
   ttcggcttct ggcgtgtgac cggcggctct agagcctctg ctaaccatgt tcatgccttc 7500
   ttctttttcc tacagctcct gggcaacgtg ctggttattg tgctgtctca tcattttggc 7560
   aaagaattcg ccaccatggt gagcaagggc gaggagctgt tcaccggggt ggtgcccatc 7620
   ctggtcgagc tggacggcga cgtaaacggc cacaagttca gcgtgtccgg cgagggcgag 7680
   ggcgatgcca cctacggcaa gctgaccctg aagttcatct gcaccaccgg caagctgccc 7740
   gtgccctggc ccaccctcgt gaccaccctg acctacggcg tgcagtgctt cagccgctac 7800
   cccgaccaca tgaagcagca cgacttcttc aagtccgcca tgcccgaagg ctacgtccag 7860
   gagcgcacca tcttcttcaa ggacgacggc aactacaaga cccgcgccga ggtgaagttc 7920
   gagggcgaca ccctggtgaa ccgcatcgag ctgaagggca tcgacttcaa ggaggacggc 7980
   aacatcctgg ggcacaagct ggagtacaac tacaacagcc acaacgtcta tatcatggcc 8040
   gacaagcaga agaacggcat caaggtgaac ttcaagatcc gccacaacat cgaggacggc 8100
   agcgtgcagc tcgccgacca ctaccagcag aacaccccca tcggcgacgg ccccgtgctg 8160
   ctgcccgaca accactacct gagcacccag tccgccctga gcaaagaccc caacgagaag 8220
   cgcgatcaca tggtcctgct ggagttcgtg accgccgccg ggatcactct cggcatggac 8280
   gagctgtaca agtaagaatt cactcctcag gtgcaggctg cctatcagaa ggtggtggct 8340
   ggtgtggcca atgccctggc tcacaaatac cactgagatc tttttccctc tgccaaaaat 8400
   tatggggaca tcatgaagcc ccttgagcat ctgacttctg gctaataaag gaaatttatt 8460
   ttcattgcaa tagtgtgttg gaattttttg tgtctctcac tcggaaggac atatgggagg 8520
   gcaaatcatt taaaacatca gaatgagtat ttggtttaga gtttggcaac atatgccata 8580
   tgctggctgc catgaacaaa ggtggctata aagaggtcat cagtatatga aacagccccc 8640
   tgctgtccat tccttattcc atagaaaagc cttgacttga ggttagattt tttttatatt 8700
   ttgttttgtg ttattttttt ctttaacatc cctaaaattt tccttacatg ttttactagc 8760
   cagatttttc ctcctctcct gactactccc agtcatagct gtccctcttc tcttatgaag 8820
   atccctcgac ctgcagccca agcttggcgt aatcatggtc atagctgttt cctgtgtgaa 8880
   attgttatcc gctcacaatt ccacacaaca tacgagccgg aagcataaag tgtaaagcct 8940
   ggggtgccta atgagtgagc taactcacat taattgcgtt gcgctcactg cccgctttcc 9000
   agtcgggaaa cctgtcgtgc cagcggatcc ctcgagcgta cgataacttc gtataatgtg 9060
   tactatacga agttatgaag ttcctatact ttctagagaa taggaacttc gttatcgaat 9120
   tgttgttgtt aacttgttta ttgcagctta taatggttac aaataaagca atagcatcac 9180
   aaatttcaca aataaagcat ttttttcact gcattctagt tgtggtttgt ccaaactcat 9240
   caatgtatct tatcatgtct ggatctcgcg cgccaggctg tggaatgtgt gtcagttagg 9300
   gtgtggaaag tccccaggct ccccagcagg cagaagtatg caaagcatgc atctcaatta 9360
   gtcagcaacc aggtgtggaa agtccccagg ctccccagca ggcagaagta tgcaaagcat 9420
   gcatctcaat tagtcagcaa ccatagtccc gcccctaact ccgcccatcc cgcccctaac 9480
   tccgcccagt tccgcccatt ctccgcccca tggctgacta atttttttta tttatgcaga 9540
   ggccgaggcc gcctcggcct ctgagctatt ccagaagtag tgaggaggct tttttggagg 9600
   ccgtcggccg ccacgaccgg tgccgccacc atcccctgac ccacgcccct gacccctcac 9660
   aaggagacga ccttccatga ccgagtacaa gcccacggtg cgcctcgcca cccgcgacga     9720
   cgtcccccgg gccgtacgca ccctcgccgc cgcgttcgcc gactaccctg caacacgcca     9780
   tacagtggac cctgaccgcc acatcgagcg ggtcaccgag ctgcaagaac tcttcctcac     9840
   gcgcgtcggg ctcgacatcg gcaaggtgtg ggtcgcggac gacggcgccg cggtggcggt     9900
   ctggaccacg ccggagagcg tcgaagcggg ggcggtgttc gccgagatcg gcccgcgcat     9960
   ggccgagttg agcggttccc ggctggccgc gcagcaacag atggaaggcc tcctggcgcc    10020
   gcaccggccc aaggagcccg cgtggttcct ggccaccgtc ggcgtctcgc ccgaccacca    10080
   gggcaagggt ctgggcagcg ccgtcgtgct ccccggagtg gaggcggccg agcgcgccgg    10140
   ggtgcccgcc ttcctggaga cctccgcgcc ccgcaacctc cccttctacg agcggctcgg    10200
   cttcaccgtc accgccgacg tcgagtgccc gaaggaccgc gcgacctggt gcatgacccg    10260
   caagcccggt gcctgacgcc cgccccacga cccgcagcgc ccgaccgaaa ggagcgcacg    10320
   accccatggc tccgaccgaa gccacccggg gcggccccgc cgaccccgca cccgcccccg    10380
   aggcccaccg actctagagc tcgctgatca gcctcgactg tgcctttcta gttgccagcc    10440
   atctgttgtt tgcccctccc ccgtgccttc cttgaccctg gaaggtgcca ctcccactgt    10500
   cctttcctaa taaaatgagg aaattgcatc gcattgtctg agtaggtgtc attctattct    10560
   ggggggtggg gtggggcagg acagcaaggg ggaggattgg gaagacaata gcaggcatgc    10620
   tggggatgcg gtgggctcta tggcttctga ggcggaaaga accagctggg gctcgagatc    10680
   ca                                                                   10682